# NEW EUROPEAN PATENT SPECIFICATION

(11) **EP 1 978 034 B2**
(45) Date of publication and mention of the opposition decision: **16.05.2018**
(45) Mention of the grant of the patent: 17.09.2014
(21) Application number: 06843658.3
(22) Date of filing: 28.12.2006
(51) Int. Cl.: C07K 16/18, A61K 39/395, A61P 1/16, A61P 1/18, A61P 9/00, A61P 9/04, A61P 11/00, A61P 13/12, A61P 19/04, A61P 19/10, A61P 29/00, A61P 35/00, C07K 16/46, G01N 33/53, C12P 21/08

(54) **ANTI-PERIOSTIN ANTIBODY AND PHARMACEUTICAL COMPOSITION CONTAINING THE SAME FOR PREVENTING OR TREATING PERIOSTIN-RELATED DISEASE**
ANTIPERIOSTIN-ANTIKÖRPER UND PHARMAZEUTISCHE ZUSAMMENSETZUNG ZUR PRÄVENTION ODER BEHANDLUNG VON MIT PERIOSTIN IN ZUSAMMENHANG STEHENDEN KRANKHEITEN, DIE DIESE ENTHÄLT
ANTICORPS ANTI-PERIOSTINE ET COMPOSITION PHARMACEUTIQUE POUR PREVENIR OU TRAITER UNE MALADIE LIEE A LA PERIOSTINE CONTENANT CET ANTICORPS

(30) Priority: 28.12.2005 JP 2005380009
(43) Date of publication of application: 08.10.2008
(73) Proprietor: Osaka University, Suita-shi Osaka 565-0871 (JP)
(72) Inventor: TANIYAMA, Yoshiaki, Suita-shi, Osaka 565-0871 (JP); MORISHITA, Ryuichi, Suita-shi, Osaka 565-0871 (JP); KATSURAGI, Naruto, Ibaraki-shi, Osaka 567-0021 (JP)
(74) Representative: Hoffmann Eitle
(86) International application number: PCT/JP2006/326280
(87) International publication number: WO 2007/077934

(56) References cited:
- WO-A1-02/20055
- WO-A2-2004/106495
- WO-A2-2005/019471
- LITVIN JUDITH ET AL: "Expression and function of periostin-isoforms in bone" JOURNAL OF CELLULAR BIOCHEMISTRY, WILEY-LISS INC, US LNKD- DOI:10.1002/JCB.20115, vol. 92, no. 5, 1 August 2004 (2004-08-01) , pages 1044-1061, XP002569260 ISSN: 0730-2312 [retrieved on 2004-06-14]
- LITVIN J ET AL: "Periostin and periostin-like factor in the human heart: possible therapeutic targets" CARDIOVASCULAR PATHOLOGY, ELSEVIER SCIENCE, NEW YORK, NY, US LNKD- DOI:10.1016/J.CARPATH.2005.09.001, vol. 15, no. 1, 1 January 2006 (2006-01-01), pages 24-32, XP025016479 ISSN: 1054-8807 [retrieved on 2006-01-01]
- HORIUCHI K. ET AL.: 'Identification and characterization of a novel protein, periostin, with restricted expression to periosteum and periodontal ligament and increased expression by transforming growth factor beta' JOURNAL OF BONE AND MINERAL RESEARCH vol. 14, no. 7, 1999, pages 1239 - 1249, XP003015313
- TANIYAMA Y. ET AL.: 'Periostin as a Novel Factor Responsible for Ventricular Dilation' FOLIA ENDOCRINOLOGICA JAPONICA vol. 80, no. 2, 20 September 2004, page 496, XP003015314
- KATSURAGI N. ET AL.: 'Periostin as a novel factor responsible for ventricular dilation' CIRCULATION vol. 110, no. 13, 2004, pages 1806 - 1813, XP003015315

## Description

### DETAILED DESCRIPTION OF THE INVENTION

### TECHNICAL FIELD

The present invention relates to antibodies against periostin isoforms having anti-cell adhesive properties, especially anti-periostin antibodies having the ability to neutralize anti-cell adhesive properties. More specifically, it relates to anti-periostin antibodies recognizing a splice variant of periostin having anti-cell adhesive properties specifically expressed in interstitial tissue during tissue restructuring such as cardiac hypertrophy, which are useful for prevention or treatment of periostin-related diseases, such as heart failure, or are useful for diagnosis of these diseases.

### BACKGROUND ART

Chronic heart failure is a condition in which the heart cannot pump enough blood to various organs due to decreased myocardial contractility. Conventionally, it has been treated with cardiotonic drugs that increase myocardial contractility such as digitalis drugs. However, these drugs have been shown to impair vital prognosis during long-term administration, due to excessive consumption of myocardial energy. Thus, recently prevailing therapies are those using diuretics, β-blockers or angiotensin inhibitors that reduce excessive workload on the heart by the sympathetic nervous system or renin-angiotensin-aldosterone system activated in heart failure condition. However, patients with heart failure have limited activities in their daily life and cannot maintain their quality of life because they are prohibited from hard exercise or the like. Moreover, the vital prognosis of patients with heart failure cannot be fully ensured. It is therefore desirable to develop a new drug effective for treating heart failure, which enables an improvement in the quality of life and an improvement in long-term vital prognosis.

On the other hand, periostin is an extracellular matrix protein and consists of a polypeptide having a molecular weight of about 90000. Each polypeptide chain has a signal sequence, a cysteine-rich domain, a fourfold repeated domain, and a C-terminal domain.

Periostin was first called osteoblast-specific factor-2 (OSF-2) and was isolated and identified as a gene specifically expressed in the mouse osteoblast cell line MC3T3-E1 (patent document 1, non-patent document 1), and later came to be known as periostin and was reported to have adhesion-promoting activity in osteoblast cells (non-patent document 2).

In early studies, periostin was thought to be an extracellular matrix specifically expressed in bone tissue. However, it is currently known to be expressed not only in bone tissue but also very highly at the onset of heart failure (non-patent document 3, non-patent document 4), aneurysms

(non-patent document 5), highly metastatic cancers (non-patent document 6, non-patent document 7, non-patent document 8), preeclampsia (non-patent document 9) as well as very slightly in normal tissue. Moreover, some periostin splice variants were shown to be expressed in osteoblasts (non-patent document 1, non-patent document 2, non-patent document 10, non-patent document 3).

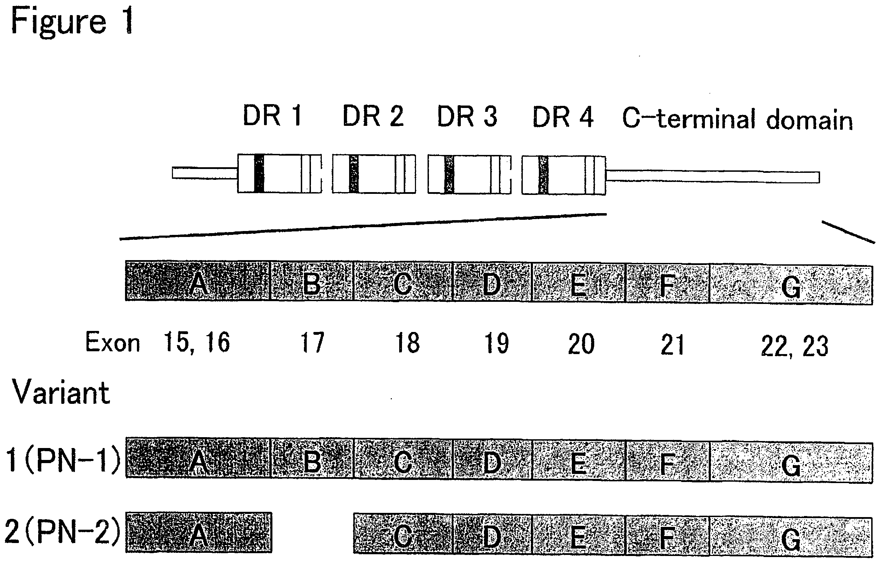

As to functions of periostin, a periostin splice variant consisting of 811 amino acids (corresponding to PN-2 in Figure 1) (non-patent document 2) and a periostin splice variant consisting of 782 amino acids (non-patent document 11) were reported to have cell adhesive properties. In contrast, it has been reported that a periostin splice variant consisting of 838 amino acids (corresponding to PN-1 in Figure 1) prevents heart fibroblasts from adhering to a plate coated with the periostin splicing variant, i.e., has no cell adhesive activity; the gene expression of the periostin splice variant consisting of 838 amino acids (corresponding to PN-1 in Figure 1) is significantly increased in heart failure model rats as compared with normal rats; this variant is an aggravating factor inducing heart dilation; and that the survival rate was significantly increased by inhibition of the expression of this protein (non-patent document 3). Further, there is a report of a prophylactic or therapeutic agent for heart failure, in which an antisense nucleotide against the periostin splice variant consisting of 838 amino acids is used to suppress expression of the periostin splicing variant (patent document 2).

As shown above, it has been suggested that expression of the periostin gene is related to the pathology of heart failure, but the relationship between the structure of periostin splicing variants and heart failure has been unknown.

Thus, we made an attempt to clarify the structure of periostin related to the pathology of heart failure by using antibodies.

As to periostin antibodies, there are reports of an antibody related to the inhibition of chemotaxis of periostin (non-patent document 12) and an antibody having inhibitory activity against periostin-induced cell growth (non-patent document 13). However, there has been neither a report of antibodies showing the structure of a region responsible for cell adhesive activity of periostin nor a report showing the relation between the cell adhesive activity of periostin and diseases such as heart failure.
Patent document 1: JPA No. HEI-5-268982
Patent document 2: Republication WO02/020055
Patent document 3: International Publication WO2005/019471
Non-patent document 1: Takeshita S. et al., Biochem J (1993) 294, 271-8
Non-patent document 2: Horiuchi K. et al., J. Bone Miner. Res. (1999) 14, 1239-49
Non-patent document 3: Katsuragi N. et al., Circulation (2004) 110, 1806-13
Non-patent document 4: Wang D. et al., Hypertension (2003) 42, 88-95
Non-patent document 5: Peters DG. et al., Stroke (2001) 32, 1036-42
Non-patent document 6: Shao R. et al., Mol Cell Biol. (2004) 24, 3992-4003
Non-patent document 7: Gonzalez HE. et al., Arch Otolaryngol Head Neck Surg. (2003) 129, 754-9
Non-patent document 8: Sasaki H. et al., Breast Cancer Res Treat.(2003) 77, 245-52
Non-patent document 9: Sasaki H. et al., Am J Obstet Gynecol. (2002) 186, 103-8
Non-patent document 10: Litvin J. et al., J Cell Biochem. (2004) 92, 1044-61
Non-patent document 11: Gillan L. et al., Cancer Res. (2002) 62, 5358-64
Non-patent document 12: Lindner V. et al., Arterioscler Thromb Vasc Biol. (2005) 25, 77-83
Non-patent document 13: Tai IT. et al., Carcinogenesis (2005) 26, 908-15

### DISCLOSURE OF THE INVENTION

The present invention aims to provide, e.g., a novel and effective prophylactic or therapeutic agent for heart failure, which enables an improvement in the quality of life and an improvement in long-term vital prognosis. More specifically, the present invention aims to provide an antibody against a periostin isoform having anti-cell adhesive activity. The present invention also aims to provide a hybridoma producing the antibody, DNA encoding the antibody, a recombinant vector containing the DNA, a transformant obtainable by introducing the recombinant vector into a host cell, as well as a method for producing the antibody, comprising culturing the transformant and collecting the antibody from the culture. The present invention further aims to provide a pharmaceutical composition comprising the antibody for preventing or treating a disease in which a periostin isoform having anti-cell adhesive activity is involved. The present invention further aims to provide a method for preventing or treating a disease in which a periostin isoform having anti-cell adhesive activity is involved, comprising administering the pharmaceutical composition to a patient, as well as a method for diagnosing the disease.

We clarified that a periostin splicing variant having no cell adhesive activity (PN-1) has anti-cell adhesive activity, i.e., the activity of detachment of adhered cells. On the other hand, we also confirmed that periostin having cell adhesive activity (PN-2) has no anti-cell adhesive activity, i.e., does not detach adhered cells. Moreover, we noted a difference in structure and cell adhesive activity between periostin splice variants having anti-cell adhesive activity (PN-1) and periostin showing no anti-cell adhesive activity (PN-2), and considered that diseases related to periostin isoforms having anti-cell adhesive activity could be prevented or treated by inhibiting a region specifically present in the periostin splice variants having anti-cell adhesive activity. In other words, we considered that inhibitors against said region might be useful as prophylactic or therapeutic agents for diseases related to periostin isoforms having anti-cell adhesive activity.

Analysis of periostin splice variants highly expressed during heart failure revealed that the C-terminal domains in which the splice variants are formed consist of exons 15 to 23; specifically rats have the following variants (1) to (4):
(1) a variant retaining all the exons (called PN-1; consisting of 838 amino acids shown as SEQ ID NO: 1; the cDNA sequence shown as SEQ ID NO: 6),
(2) a variant lacking Exon-17 (called PN-2; consisting of 811 amino acids shown as SEQ ID NO: 5; 27 amino acids (Exon-17) shown in SEQ ID NO: 3 are deleted from PN-1; the cDNA sequence shown as SEQ ID NO: 7),
(3) a variant lacking Exon-21 (called PN-3; consisting of 810 amino acids),
(4) a variant lacking Exon-17 and Exon-21 (called PN-4; consisting of 783 amino acids).

In addition to rats, mouse and human PN-1 and PN-2 were also found (mouse PN-1:SEQ ID NO: 8 (amino acid sequence), SEQ ID NO: 9 (cDNA sequence); mouse PN-2: SEQ ID NO: 10 (amino acid sequence), SEQ ID NO: 11 (cDNA sequence); human PN-1: SEQ ID NO: 12 (cDNA sequence); human PN-2: SEQ ID NO: 13 (amino acid sequence), SEQ ID NO: 14 (cDNA sequence)). Among them, PN-1 was highly expressed while PN-2 and PN-3 were expressed to a lesser extent in rat cardiac hypertrophy tissue. Thus, we contemplated preparing an antibody specifically recognizing the amino acid residue part encoded by Exon-17 as an inhibitor against that site, which site is structurally different in PN-1 and PN-2 and exclusively found in PN-1.

In order to prepare an antibody, the material used as an immunogen must be hydrophilic, and if an antibody is to be prepared using a part of a large polypeptide such as protein, the part used as an immunogen must be exposed on the surface of the protein to form an epitope part. Thus, in order to examine the possibility of using the Exon-17 peptide chain as an antigen, an epitope search was initially performed using Accelrys software that is widely used in the field of bioinformatics Mac Vector 7.2. It was judged from "Hydrophilicity", "Surface Probability" and "Antigenicity" that TTKIITKLVEPKIKVIQGSLQPIIKTE (SEQ ID NO: 3) of the Exon-17 region is mostly hydrophobic, suggesting that this region is very unlikely to be exposed on the surface of the protein molecule and has no immunogenicity, so it cannot be used to prepare an antibody, and therefore, it was presumed that it would be difficult to use to practically prepare an antibody.

However, we ventured to prepare an antibody against the amino acid sequence encoded by Exon-17 on the assumption that the use of an antibody against a polypeptide region encoded by Exon-17 specifically found in PN-1 would be most suitable for specifically inhibiting functions of PN-1. A peptide consisting of 27 amino acids constituting a peptide encoded by the Exon-17 region was synthesized and used to immunize rabbits, and the resulting serum was purified to give an IgG fraction, whereby an anti-Exon-17 peptide polyclonal antibody was prepared. When periostin protein PN-1 was then added to an 80% confluent culture of heart fibroblasts, nearly 100% cell detachment (i.e., anti-cell adhesive activity) was observed. Administration of the anti-Exon-17 peptide antibody to this experimental system inhibited the cell detachment mediated by periostin protein PN-1, showing that the anti-Exon-17 peptide antibody is an antibody having a neutralizing activity against periostin protein PN-1. Then, acute myocardial infarction model rats were prepared and weekly administration of the anti-Exon-17 peptide antibody was continued to show that heart dilation was significantly inhibited 4 weeks after the preparation of the models, and that heart function was improved. It was also shown that these properties were sustained and cardiac fibrosis was inhibited even 8 weeks after the preparation of the models. This indicates that the anti-Exon-17 peptide antibody is an antibody having the activity of inhibiting heart dilation and cardiac fibrosis associated with the progress of heart failure condition, and the activity of improving heart function, leading to accomplishment of the present invention.

As a solution to the problems described above, the present invention provides an antibody as claimed against a periostin isoform having anti-cell adhesive activity specifically expressed in the heart during heart failure or the like, particularly an antibody against the periostin splice site.

Namely, the present invention includes the following aspects.
[1.] An antibody against a periostin isoform having anti-cell adhesive activity, wherein the antibody specifically recognises a site responsible for the anti-cell adhesion of periostin, is against one of the amino acid sequences selected from the group of SEQ ID NOs: 3, 4, 21, 22, 23, 24, 26 and 34, and has ability to inhibit anti-cell adhesive activity of periostin.
[2.] The antibody of [1], wherein the amino acid sequence is the amino acid sequence of SEQ ID NOs: 3, 4, or 21.
[3.] The antibody of [1], wherein the antibody specifically recognizes an amino acid sequence shown in SEQ ID NO: 34.
[4.] The antibody of any one of [1] to [3], which is a monoclonal antibody.
[5.] An antibody of [4], produced by a hybridoma cell line FERM BP-10718.
[6.] A hybridoma producing the antibody of [1], obtainable by a process comprising the steps of:
   immunising a non-human mammal with a peptide having one of the amino acid sequences selected from the group of SEQ ID No. 3, 4 and 21, or a peptide selected from the group of SEQ ID : No 3, 4 and 21 coupled to a carrier protein via cysteine residues introduced into the N-terminus of said peptide, and
   fusing an antibody-producing cell of the mammal with a myeloma cell.
[7.] A hybridoma cell line FERM BP-10718.
[8.] A method for producing an antibody of [4], comprising the steps of:
   immunising a non-human mammal with peptide having one of the amino acid sequences selected from the group of SEQ ID No. 3, 4 and 21, or a peptide selected from the group of SEQ ID : NO 3, 4 and 21 coupled to a carrier protein via cysteine residues introduced into the N-terminus of said peptide,
   fusing an antibody-producing cell of the mammal with a myeloma cell; and
   culturing the obtained hybridoma.
[9.] A method for producing an antibody, comprising a step of culturing the hybridoma cell line FERM BP-10718.
[10.] A pharmaceutical composition comprising the antibody of any one of [1] to [5].
[11.] A pharmaceutical composition for use in preventing or treating a disease, comprising the antibody of any one of [1] to [5] wherein the disease is heart failure, myocardial infarction, heart dilation, cardiac hypertrophy, cardiac fibrosis, cardiomyopathy, myocarditis, valvular disease or cancer.
[12.] The antibody as defined in any one of [1] to [5] for use in a method for preventing or treating a disease , wherein the disease is a heart failure, myocardial infarction, heart dilation, cardiac hypertrophy, cardiac fibrosis, cardiomyopathy, myocarditis, valvular disease or cancer.
[13.] The antibody as defined in any one of [1] to [5] for use in a method for diagnosing a disease in which a periostin isoform having anti-cell adhesive activity is involved, comprising measuring the amount of the periostin isoform in a biological sample, wherein the disease is heart failure, myocardial infarction, heart dilation, cardiac hypertrophy, cardiac fibrosis, cardiomyopathy, myocarditis, valvular disease or cancer.
[14.] The use of [13], wherein the antibody is a labeled antibody.
[15.] The antibody as defined in any one of [1] to [5] for use in a method for detecting or quantifying a periostin isoform having anti-cell adhesive activity in a sample.
[16.] A diagnostic reagent for use in diagnosing a disease, comprising the antibody of any one of [1] to [5] wherein the disease is heart failure, myocardial infarction, heart dilation, cardiac hypertrophy, cardiac fibrosis, cardiomyopathy, myocarditis, valvular disease or cancer.

Also disclosed herein are:
(1) An antibody against a periostin isoform having anti-cell adhesive activity.
(2) An antibody against a periostin isoform having an amino acid sequence shown in SEQ ID NO: 1 or SEQ ID NO: 2.
(3) An antibody against a periostin isoform having an amino acid sequence shown in SEQ ID NO: 3 or SEQ ID NO: 4.
(4) An antibody against a periostin isoform having an amino acid sequence shown in SEQ ID NO: 34.
(5) An antibody against a region responsible for the anti-cell adhesive activity of a periostin isoform having anti-cell adhesive activity.
(6) An antibody against a peptide consisting of an amino acid sequence shown in SEQ ID NO: 3 or SEQ ID NO: 4 or a part thereof.
(7) An antibody against a peptide having an amino acid sequence shown in SEQ ID NO: 34 or a partial amino acid sequence thereof.
(8) The antibody as defined in any one of (1) to (7), which specifically recognizes an amino acid sequence shown in SEQ ID NO: 3 or SEQ ID NO: 4, or a partial site thereof.
(9) The antibody as defined in any one of (1) to (7), which specifically recognizes an amino acid sequence shown in SEQ ID NO: 34, or a partial site thereof.
(10) An antibody binding to a peptide consisting of an amino acid sequence shown in SEQ ID NO: 26.
(11) The antibody as defined in any one of (1) to (10), which has an ability to neutralize anti-cell adhesive activity.
(12) The antibody as defined in any one of (1) to (11), which is a polyclonal antibody, monoclonal antibody, chimeric antibody, humanized antibody or human antibody.
(13) The antibody as defined in (12), which is a monoclonal antibody.
(14) An antibody produced by a hybridoma cell line FERM BP-10718.
(15) An antibody fragment consisting of a partial fragment of the monoclonal antibody as defined in (13).
(16) An antibody derivative comprising a protein or low-molecular drug bound to the antibody as defined in any one of (1) to (14) or the antibody fragment as defined in (15).
(17) A hybridoma producing the antibody as defined in any one of (1) to (14).
(18) The hybridoma as defined in (17), which is a hybridoma cell line FERM BP-10718.
(19) DNA encoding the antibody as defined in any one of (1) to (14), the antibody fragment as defined in (15) or the derivative as defined in (16), wherein the derivative comprises a protein bound to the antibody or antibody fragment.
(20) A recombinant vector containing the DNA as defined in (19).
(21) A transformant obtainable by introducing the recombinant vector as defined in (20) into a host cell.
(22) A method for producing the antibody as defined in any one of (1) to (14), the antibody fragment as defined in (15) or the derivative as defined in (16), wherein the derivative comprises a protein bound to the antibody or antibody fragment, said method comprising culturing the transformant as defined in (21) to produce the antibody, antibody fragment or derivative in the culture, and collecting the antibody, antibody fragment or derivative from the culture.
(23) A pharmaceutical composition comprising the antibody as defined in any one of (1) to (14), the antibody fragment as defined in (15) or the derivative as defined in (16).
(24) A pharmaceutical composition for preventing or treating a disease in which a periostin isoform having anti-cell adhesive activity is involved, comprising the antibody as defined in any one of (1) to (14), the antibody fragment as defined in (15) or the derivative as defined in (16).
(25) The pharmaceutical composition as defined in (24), wherein the disease is heart failure, myocardial infarction, heart dilation, cardiac hypertrophy, cardiac fibrosis, cardiomyopathy, myocarditis, valvular disease, cancer, aneurysm, arteriosclerosis, central neurodegenerative disease, renal disease, rheumatoid arthritis, osteoporosis, pulmonary emphysema, pulmonary hypertension, chronic obstructive pulmonary disease (COPD), nephritis, pancreatitis, hepatitis, hepatic fibrosis or pulmonary fibrosis.
(26) A method for preventing or treating a disease in which a periostin isoform having anti-cell adhesive activity is involved, comprising administering the pharmaceutical composition as defined in (23) to a patient.
(27) The method as defined in (26), wherein the disease is heart failure, myocardial infarction, heart dilation, cardiac hypertrophy, cardiac fibrosis, cardiomyopathy, myocarditis, valvular disease, cancer, aneurysm, arteriosclerosis, central neurodegenerative disease, renal disease, rheumatoid arthritis, osteoporosis, pulmonary emphysema, pulmonary hypertension, chronic obstructive pulmonary disease (COPD), nephritis, pancreatitis, hepatitis, hepatic fibrosis or pulmonary fibrosis.
(28) A method for diagnosing a disease in which a periostin isoform having anti-cell adhesive activity is involved, by using the antibody as defined in any one of (1) to (14), the antibody fragment as defined in (15) or the derivative as defined in (16).
(29) The method as defined in (28) wherein the antibody is a labeled antibody.
(30) The method as defined in (28) or 29, wherein the disease is heart failure, myocardial infarction, heart dilation, cardiac hypertrophy, cardiac fibrosis, cardiomyopathy, myocarditis, valvular disease, cancer, aneurysm, arteriosclerosis, central neurodegenerative disease, renal disease, rheumatoid arthritis, osteoporosis, pulmonary emphysema, pulmonary hypertension, chronic obstructive pulmonary disease (COPD), nephritis, pancreatitis, hepatitis, hepatic fibrosis or pulmonary fibrosis.
(31) The antibody as defined in any one of (1) to (14), the antibody fragment as defined in (15) or the derivative as defined in (16), which has been labeled.
(32) A method for quantifying a periostin isoform having anti-cell adhesive activity in a sample by using the antibody as defined in any one of (1) to (14), the antibody fragment as defined in (15) or the derivative as defined in (16).
(33) A method for detecting a periostin isoform having anti-cell adhesive activity in a sample by using the antibody as defined in any one of (1) to (14), the antibody fragment as defined in (15) or the derivative as defined in (16).
(34) A diagnostic reagent for a disease in which a periostin isoform having anti-cell adhesive activity is involved, comprising the antibody as defined in any one of (1) to (14), the antibody fragment as defined in (15) or the derivative as defined in (16).

The expression "amino acid sequence shown in SEQ ID NO:" as used herein includes variants of the amino acid sequence obtained by deleting, substituting or adding one or more amino acids.

### BRIEF EXPLANATION OF THE DRAWINGS

Figure 1 is a schematic diagram showing a rat periostin splicing variant.
Figure 2 is a diagram showing assay results of the anti-cell adhesive properties of rat PN-1 (Example 2).
Figure 3 is a diagram showing assay results of the inhibition of rat PN-1 activity by anti-rat Exon-17 peptide antibody (Example 3).
Figure 4-1 is a diagram showing assay results of the inhibition of heart dilation by anti-rat Exon-17 peptide antibody 4 weeks after the preparation of acute myocardial infarction models (Example 4: anterior wall thickness, posterior wall thickness).
Figure 4-2 is a diagram showing assay results of the inhibition of heart dilation by anti-rat Exon-17 peptide antibody 4 weeks after the preparation of acute myocardial infarction models (Example 4: end-diastolic inner diameter, end-systolic inner diameter, heart function).
Figure 4-3 is a diagram showing assay results of the inhibition of heart dilation by anti-rat Exon-17 peptide antibody 4 weeks after the preparation of acute myocardial infarction models (Example 4: heart rate, infarction area).
Figure 5-1 is a diagram showing assay results of the inhibition of heart dilation by anti-rat Exon-17 peptide antibody 8 weeks after the preparation of acute myocardial infarction models (Example 4: anterior wall thickness, posterior wall thickness).
Figure 5-2 is a diagram showing assay results of the inhibition of heart dilation by anti-rat Exon-17 peptide antibody 8 weeks after the preparation of acute myocardial infarction models (Example 4: end-diastolic inner diameter, end-systolic inner diameter, heart function).
Figure 5-3 is a diagram showing assay results of the inhibition of heart dilation by anti-rat Exon-17 peptide antibody 8 weeks after the preparation of acute myocardial infarction models (Example 4: heart rate, infarction area).
Figure 6-1 is a diagram showing hemodynamics of model rats treated with anti-rat Exon-17 peptide antibody (Example 4: LVP, heart rate).
Figure 6-2 is a diagram showing hemodynamics of model rats treated with anti-rat Exon-17 peptide antibody (Example 4: (+) dP/dt, (-) dP/dt).
Figure 6-3 is a diagram showing hemodynamics of model rats treated with anti-rat Exon-17 peptide antibody (Example 4: SBP, DBP, LVEDP).
Figure 7 is a diagram showing the results of histological analysis of model rats treated with anti-rat Exon-17 peptide antibody (Example 4).
Figure 8 is a diagram showing the minor axis diameters of myocardial cells of model rats treated with anti-rat Exon-17 peptide antibody (Example 4).
Figure 9-1 is a diagram showing the results of gene expression analysis of model rats treated with anti-rat Exon-17 peptide antibody (Example 4: G3PDH).
Figure 9-2 is a diagram showing the results of gene expression analysis of model rats treated with anti-rat Exon-17 peptide antibody (Example 4: ET-1/G3, Angiotensinogen/G3).
Figure 9-3 is a diagram showing the results of gene expression analysis of model rats treated with anti-rat Exon-17 peptide antibody (Example 4: α-MHC/G3, β-MHC/G3).
Figure 9-4 is a diagram showing the results of gene expression analysis of model rats treated with anti-rat Exon-17 peptide antibody (Example 4: Col-I/G3, Col-III/G3).
Figure 9-5 is a diagram showing the results of gene expression analysis of model rats treated with anti-rat Exon-17 peptide antibody (Example 4: TGF-β/G3, TNF-α/G3).
Figure 10 is a diagram showing assay results of the anti-cell adhesive properties of human PN-1 (Example 15).
Figure 11 is a diagram showing assay results of the inhibition of human PN-1 activity by anti-human Exon-17 monoclonal antibody (Example 16).
Figure 12-1 is a diagram showing assay results of the inhibition of heart dilation by anti-human Exon-17 monoclonal antibody 4 weeks after the preparation of acute myocardial infarction models (Example 17: anterior wall thickness, posterior wall thickness).
Figure 12-2 is a diagram showing assay results of the inhibition of heart dilation by anti-human Exon-17 monoclonal antibody 4 weeks after the preparation of acute myocardial infarction models (Example 17: end-diastolic inner diameter, end-systolic inner diameter, heart function).
Figure 12-3 is a diagram showing assay results of the inhibition of heart dilation by anti-human Exon-17 monoclonal antibody 4 weeks after the preparation of acute myocardial infarction models (Example 17: heart rate, infarction area).

### THE MOST PREFERRED EMBODIMENTS OF THE INVENTION

In an embodiment, the present invention provides an antibody against a periostin isoform having anti-cell adhesive activity. Periostin here is one of extracellular matrix proteins and is known to include some splice variants, some of which are specifically expressed in the heart during heart failure or the like. In the present invention, antibodies can be used as substances (i.e., as inhibitors) for inhibiting functions of a periostin splicing variant specifically expressed in the heart during heart failure or the like because of their high specificity, safety for humans and for other reasons. In the present invention, an antibody can be prepared against an antigen composed of a chemically synthesized peptide consisting of an amino acid sequence encoded by the Exon-17 region of the C-terminal domain at which a splice variant specific to the heart during heart failure or the like is formed, though such a peptide can also be obtained from any source by enzymatic digestion of periostin protein or by genetic engineering techniques.

In the present invention, the expression "having anti-cell adhesive activity" means having the action of separating or eliminating adhered cells. Likewise, the expression "having no anti-cell adhesive activity" means that adhered cells are neither detached nor peeled off. In this case, the cells maintain their adhered state. The presence or absence of anti-cell adhesive activity may be determined by culturing cells (e.g., heart fibroblasts) in a culture plate to allow the cells to adhere to the culture plate, adding an assay sample and further culturing the cells, washing the plate to remove separated cells, staining the remaining cells, and confirming the state of the adhered cells.

In the present invention, periostin isoforms having anti-cell adhesive activity preferably include periostin isoforms consisting of an amino acid sequence of SEQ ID NO: 1 (rat periostin PN-1, 838 amino acids), SEQ ID NO: 2 (human periostin PN-1, 836 amino acids having an N-terminal signal sequence shorter by 2 amino acids than that of rat periostin), or SEQ ID NO: 8 (mouse periostin PN-1). Other periostin isoforms having anti-cell adhesive activity include periostin isoforms having an amino acid sequence shown in SEQ ID NO: 3 (27 amino acids encoded by Exon-17 of rat periostin), SEQ ID NO: 4 (27 amino acids encoded by Exon-17 of human periostin) or SEQ ID NO: 21 (27 amino acids encoded by Exon-17 of mouse periostin). Further examples include periostin isoforms having an amino acid sequence shown in SEQ ID NO: 22 (9 amino acids covering from the 1st to the 9th amino acids from the N-terminus of the amino acid sequence encoded by Exon-17 of human periostin), SEQ ID NO: 23 (9 amino acids covering from the 1st to the 9th amino acids from the N-terminus of the amino acid sequence encoded by Exon-17 of rat periostin) or SEQ ID NO: 24 (9 amino acids covering from the 1st to the 9th amino acids from the N-terminus of the amino acid sequence encoded by Exon-17 of mouse periostin). Further examples include periostin isoforms having an amino acid sequence shown in SEQ ID NO: 34 (6 amino acids covering from the 1st to the 6th amino acids from the N-terminus of the amino acid sequence encoded by Exon-17 of human periostin).

Regions responsible for the anti-cell adhesive activity of periostin include, e.g., the splice site of Exon-17. Specific examples include an amino acid residue part shown in SEQ ID NO: 3 of a periostin isoform having an amino acid sequence shown in SEQ ID NO: 1 (672-698 amino acids of SEQ ID NO: 1), an amino acid residue part shown in SEQ ID NO: 4 of a periostin isoform having an amino acid sequence shown in SEQ ID NO: 2 (670-696 amino acids of SEQ ID NO: 2), and an amino acid residue part shown in SEQ ID NO: 21 of a periostin isoform having an amino acid sequence shown in SEQ ID NO: 8 (672-698 amino acids of SEQ ID NO: 8). Further examples include an amino acid sequence shown in SEQ ID NO: 22 or SEQ ID NO: 23, as well as SEQ ID NO: 34 (an amino acid sequence shown in SEQ ID NO: 22 or SEQ ID NO: 23, as well as an amino acid sequence consisting of the N-terminal 1st to 6th amino acid residues of SEQ ID NO: 22 or SEQ ID NO: 23).

In a preferred embodiment of the present invention, the expression "capable of specifically recognizing a site responsible for anti-cell adhesion" means that a region responsible for anti-cell adhesion of periostin preferably including the splice site of Exon-17 can be specifically recognized. For example, antibodies capable of specifically recognizing a site responsible for anti-cell adhesion of periostin include antibodies against an amino acid residue part shown in SEQ ID NO: 3 of a periostin isoform having an amino acid sequence shown in SEQ ID NO: 1 (672-698 amino acids of SEQ ID NO: 1), an amino acid residue part shown in SEQ ID NO: 4 of a periostin isoform having an amino acid sequence shown in SEQ ID NO: 2 (670-696 amino acids of SEQ ID NO: 2), an amino acid residue part shown in SEQ ID NO: 21 of a periostin isoform having an amino acid sequence shown in SEQ ID NO: 8 (672-698 amino acids of SEQ ID NO: 8), or a part thereof. Further examples include antibodies against a polypeptide having an amino acid sequence shown in SEQ ID NO: 22 or SEQ ID NO: 23, or a part thereof. Further examples include antibodies against a periostin isoform having an amino acid sequence consisting of the N-terminal 1st to 6th amino acid residues of SEQ ID NO: 22 or SEQ ID NO: 23 (SEQ ID NO: 34).

The expression "inhibiting a region responsible for the anti-cell adhesive activity of periostin" means that the action or activity of the above "region responsible for the anti-cell adhesive activity of periostin" is inhibited, and more specifically means that the action or activity of periostin is inhibited using, for example, an antibody capable of specifically recognizing the above site responsible for anti-cell adhesive activity.

In an embodiment, antibodies of the present invention are monoclonal antibodies and polyclonal antibodies obtained by using the antigens as described above. The "monoclonal antibodies" here refer to any monoclonal antibody showing reactivity against the antigens described above, and the "monoclonal antibodies" include natural antibodies obtained by immunizing mammals such as mice, rats, hamsters, guinea pigs or rabbits with the antigens, chimeric monoclonal antibodies (chimeric antibodies) and humanized monoclonal antibodies (humanized antibodies; CDR-grafted antibodies) that can be prepared by using genetic recombination techniques, as well as human monoclonal antibodies (human antibodies) that can be prepared by using human antibody-producing transgenic animals or the like. Antibodies of the present invention include monoclonal antibodies having any isotype such as IgG (IgG1, IgG2, IgG3, IgG4), IgM, IgA, IgD or IgE, preferably IgG (IgG1, IgG2, IgG3, IgG4) or IgM.

When the peptides described above are to be used as antigens, they can be used alone as antigens but also can be used for immunization by adsorption to a macromolecular material such as polyvinyl pyrrolidone, latex or polymethyl methacrylate, or coupling to a carrier protein such as KLH (Keyhole Limpet Hemocyanin) or BSA (bovine serum albumin), and any method can be used. Generally, the peptides may preferably be coupled to a carrier protein by known methods (e.g., "New series of Development of Drugs, Vol. 14, Hirokawa Publishing Co., 1991"). The peptides are coupled to a carrier protein via cysteine residues introduced into the C- or N-terminus of the peptides so that the peptides have directionality. Crosslinkers commonly used in the field of the art can be used so long as they are suitable for this purpose. Suitable crosslinkers include succinimidyl-4-(N-maleimidomethyl)cyclohexane-1- carboxylate (hereinafter abbreviated as "SMCC") or 3-maleimidobenzoic acid-N-hydroxysuccinimide ester (MBS). Monoclonal antibodies are prepared by culturing hybridomas prepared by the cell fusion method of Kohler and Milstein (G. Kohler et al Nature (1975) 256,495-7) to secrete the antibodies and isolating them from the cultures. That is, a mammal is immunized with a peptide having an amino acid sequence encoded by Exon-17 or the like and then antibody-producing cells of this animal are fused to myeloma cells to give hybridomas. Search for hybridomas producing antibodies binding to Exon-17 is performed by e.g., an enzyme immunoassay (hereinafter abbreviated as "ELISA") for hybridoma supernatants using a microplate on which the antigen has been immobilized. Animals to be immunized are not specifically limited, but include various mammals such as mice, rats, guinea pigs, rabbits, sheep, goats, cats, dogs, etc. Among the animals listed above, Balb/c mice are generally used for preparation of monoclonal antibodies because of ease of handling or for other reasons, but other strains of mice can also be used. The concentration of the antigen used for immunization here is selected to form sufficient amounts of antigenically stimulated lymphocytes. Preferably, 1-100 µg of an antigen is diluted to an appropriate concentration with physiological saline or the like and suspended in Freund's complete adjuvant or Freund's incomplete adjuvant or the like, and the suspension is administered to an animal by intraperitoneal or subcutaneous injection or other means. Administration is performed once to several times every 2-4 weeks. The final immunization is normally performed by administering a solution of 1-100 µg of the antigen in physiological saline by intravenous or subcutaneous injection or other means. Several days after the final immunization, antibody-producing cells such as lymphocytes, preferably spleen cells or lymph node cells, are removed from the immunized animal for cell fusion. Cell fusion using spleen cells as antibody-producing cells is explained below, though antibody-producing cells other than spleen cells can also be used for cell fusion. Spleen cells prepared from the spleen aseptically removed 3-4 days after the final immunization are fused to appropriate myeloma cells in the presence of a fusion promoter. The myeloma cells used for fusion may be derived from mammals, but generally those derived from the same species as the animal used for immunization. Various cell lines are already known, for example, SP2/0-Ag14(SP2) [Nature, 276, 269(1978)], NS-1-Ag4/1(NS-1), P3-X63Ag8U.1(P3U1) [Curr. Top. Microbiol. Immunol. 81, 1-7(1978): available from ATCC under ATCC No. CRL-1597], P3-NS1-1-Ag4-1, P3-X63Ag8(P3), FO, X63Ag8.653(X63.653), 210.RCY3.Ag1.2.3, S194/5XXO.BUI, SKO-007, GM15006TG-A12 and the like are preferably used for mice, and Y3.Ag1.2.3 and the like are preferably used for rats. Preferred fusion promoters include polyethylene glycol (PEG) having a molecular weight of 1000-6000 and Sendai virus (HVJ). Generally, the ratio of spleen cells and myeloma cells during fusion is preferably 10:1-2:1.

Hybridomas can be separated from fused cells by culturing of a mixture of unfused spleen cells, unfused myeloma cells and fused cells in a selective medium inhibiting the survival of unfused myeloma cells for an appropriate period until unfused cells die (about 1 week). The selective medium may be e.g., HAT medium (medium containing hypoxanthine, aminopterin and thymidine). In this selective medium, unfused myeloma cells die, and unfused spleen cells die after a certain period of time (after about 1 week) because they are non-tumorous cells, so that hybridomas can be obtained by selecting viable cells. Hybridomas producing desired antibodies can be obtained by searching of strains producing the desired antibodies and cloning of the strains to prepare monoclonal antibodies by standard limiting dilution. Thus obtained hybridomas producing monoclonal antibodies of the present invention can grow in media suitable for their growth and can be readily stored in deep freezers or liquid nitrogen. Thus obtained hybridomas can produce antibodies by growing them in nutrient media or in the abdominal cavity of a mammal, and the produced antibodies can be purified from culture supernatants or the ascites fluid or serum of the mammal. As an example of the hybridomas of the present invention, a hybridoma can be used that was deposited under FERM BP-10718 on November 1, 2006 with the International Patent Organism Depositary, the National Institute of Advanced Industrial Science and Technology. Purification of the antibodies can be performed by standard isolation/purification methods such as centrifugation, dialysis, salting out with ammonium sulfate or the like, ion exchange chromatography using a DEAE column or the like, gel filtration, affinity chromatography, etc. The isotypes and subclasses of thus obtained monoclonal antibodies can be determined using an identification method such as Ouchterlony assay, ELISA, or RIA. Ouchterlony assay is convenient but requires a concentration operation if the monoclonal antibody concentration is low. When ELISA or RIA is used, however, the isotypes and subclasses of the monoclonal antibodies can be identified by direct reaction of the culture supernatant with an antigen-adsorbed solid phase and by use of antibodies corresponding to various immunoglobulin isotypes and subclasses as secondary antibodies. More convenient methods employ commercially available identification kits (e.g., Mouse Typer kit; Bio-Rad) or the like. The quantification of protein can be performed by the Folin-Lowry method and calculation from the absorbance at 280 nm [1.4 (OD280) = 1 mg/ml immunoglobulin]. Thus obtained monoclonal antibodies of the present disclosure specifically recognize an amino acid sequence shown in SEQ ID NO: 1 or SEQ ID NO: 2, an amino acid sequence shown in SEQ ID NO: 3 or SEQ ID NO: 4, a periostin isoform (PN-1) having an amino acid sequence shown in SEQ ID NO: 34, a peptide consisting of an amino acid sequence shown in SEQ ID NO: 3 or SEQ ID NO: 4, or a peptide having an amino acid sequence shown in SEQ ID NO: 34. Preferably, the monoclonal antibodies of the present invention can specifically recognize and bind to a peptide consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4), and a peptide consisting of an amino acid sequence covering from the 669th tyrosine to the 679th valine from the N-terminus of the amino acid sequence of human periostin PN-1 (SEQ ID NO: 2). Namely, the monoclonal antibodies of the present invention can specifically recognize an amino acid sequence site (TTKIITKVV; SEQ ID NO: 22), or a part thereof, which covers from the N-terminal threonine to the 9th valine of the amino acid sequence of the human periostin Exon-17 peptide chain (SEQ ID NO: 4). More preferably, the monoclonal antibodies of the present invention can recognize and bind to a peptide comprising alanine substitutions at the 1st and the 8-10th amino acids from the N-terminus of a peptide consisting of an amino acid sequence (YTTKIITKVV, SEQ ID NO: 26) covering from the -1st tyrosine to the 9th valine from the N-terminus of the human periostin Exon-17 peptide chain (SEQ ID NO: 4). Namely, the monoclonal antibodies of the present invention can specifically recognize at least an amino acid sequence site (SEQ ID NO: 34), or a part thereof, which covers from the 1st threonine to the 6th threonine from the N-terminus of the amino acid sequence of the human periostin Exon-17 peptide chain (SEQ ID NO: 4) or the amino acid sequence of the rat periostin Exon-17 peptide chain (SEQ ID NO: 3). Moreover, the monoclonal antibodies of the present invention have the activity of suppressing or inhibiting the anti-cell adhesive properties of human periostin-1 protein, i.e., neutralizing the anti-cell adhesive properties of human periostin-1 protein. Furthermore, the monoclonal antibodies of the present invention can suppress the heart dilation, cardiac hypertrophy and cardiac fibrosis induced during heart failure or the like to improve heart function.

When polyclonal antibodies are used as antibodies of the present invention, the polyclonal antibodies can be obtained by standard methods such as the method described in "New Lecture on Biochemical Experiments, 12, edited by the Japanese Biochemical Society, Tokyo Kagaku Dozin, 1992". Animals to be immunized are not specifically limited, but include horses, goats, sheep, rabbits, guinea pigs, mice, chickens, etc. When a rabbit is to be immunized, an antigen is diluted to an appropriate concentration with physiological saline or the like and suspended in Freund's complete adjuvant, Freund's incomplete adjuvant or aluminum hydroxide adjuvant or the like, and the suspension is injected at a dose of 10-1000 µg per animal followed by 1-3 booster injections after 2-4 weeks to give antisera. Multi-site subcutaneous injection is preferred. Preparation of polyclonal antibodies from antisera can be performed by the method as described for the purification of monoclonal antibodies. Thus obtained polyclonal antibodies of the present disclosure specifically recognize an amino acid sequence shown in SEQ ID NO: 1 or SEQ ID NO: 2, an amino acid sequence shown in SEQ ID NO: 3 or SEQ ID NO: 4, a periostin isoform (PN-1) having an amino acid sequence shown in SEQ ID NO: 34, a peptide consisting of an amino acid sequence shown in SEQ ID NO: 3 or SEQ ID NO: 4, or a peptide having an amino acid sequence shown in SEQ ID NO: 34. Preferably, the polyclonal antibodies of the present invention can specifically recognize and bind to a peptide consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4), and a peptide consisting of an amino acid sequence covering from the 669th tyrosine to the 679th valine from the N-terminus of the amino acid sequence of human periostin PN-1 (SEQ ID NO: 2). Namely, the polyclonal antibodies of the present invention can specifically recognize an amino acid sequence site (TTKIITKVV; SEQ ID NO: 22), or a part thereof, which covers from the N-terminal threonine to the 9th valine of the amino acid sequence of the human periostin Exon-17 peptide chain (SEQ ID NO: 4). More preferably, the polyclonal antibodies of the present invention can recognize and bind to a peptide comprising alanine substitutions at the 1st and the 8-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4). Namely, the polyclonal antibodies of the present invention can specifically recognize at least an amino acid sequence site (SEQ ID NO: 34), or a part thereof, which covers from the 1st threonine to the 6th threonine from the N-terminus of the amino acid sequence of the human periostin Exon-17 peptide chain (SEQ ID NO: 4) or the amino acid sequence of the rat periostin Exon-17 peptide chain (SEQ ID NO: 3). Moreover, the polyclonal antibodies of the present invention have the activity of suppressing or inhibiting the anti-cell adhesive properties of human periostin-1 protein, i.e., neutralizing the anti-cell adhesive properties of human periostin-1 protein. Furthermore, the polyclonal antibodies of the present invention can suppress the heart dilation, cardiac hypertrophy and cardiac fibrosis induced during heart failure or the like to improve heart function.

### Preparation of humanized antibodies

Immunoglobulin G (hereinafter simply referred to as "IgG") consists of two light polypeptide chains having a molecular weight of about 23000 (hereinafter referred to as "light chain") and two heavy polypeptide chains having a molecular weight of about 50000 (hereinafter referred to as "heavy chain"). The heavy and light chains both have a repeating structure of conserved amino acid sequence regions consisting of about 110 residues, which constitute a basic unit of three-dimensional structure of IgG (hereinafter referred to as "domain"). The heavy and light chains consist of 4 and 2 successive domains, respectively. In both of the heavy and light chains, the amino acid sequence of the amino terminal domain is more variable between antibody molecules than that of the other domains, and this domain is called variable domain (hereinafter referred to as "V domain"). At the amino terminus of IgG, the V domains of the heavy and light chains are complementarily associated to form a variable region. In contrast, the remaining domains collectively form a constant region. The constant region has a sequence characteristic of each animal species, e.g., the constant region of mouse IgG differs from the constant region of human IgG so that mouse IgG is recognized as foreign matter by the human immune system, resulting in a Human Anti Mouse Antibody (hereinafter referred to as "HAMA") response (Schroff RW. et al. Cancer Res. (1985) 45,879-85). Thus, mouse antibodies cannot be repeatedly administered to humans. In order to administer such antibodies to humans, the antibody molecules must be modified to prevent HAMA response while maintaining the specificity of the antibodies. According to the results of X-ray crystallography, such a domain is generally in the form of an elliptic cylindrical structure formed of two antiparallel beta sheets consisting of 3 to 5 beta-chains. In the variable region, three loops for each of the V domains of the heavy and light chains are assembled to form an antigen-binding site. These loops are called complementarity determining regions (hereinafter referred to as "CDRs), which are most variable in amino acid sequence. The remaining parts of the variable region other than the CDRs serve to maintain the structures of the CDRs and are called "framework". Kabatt et al. collected a number of primary sequences of heavy and light chain variable regions and prepared a table classifying the primary sequences into CDRs and frameworks on the basis of sequence conservation (Kabatt et al. SEQUENCES OF IMMUNOLOGICAL INTEREST, 5th edition, NIH publication, No. 91-3242, E.A.). The frameworks were further classified into a plurality of subgroups having common amino acid sequence patterns. The presence of a consensus framework between human and mouse sequences was also found. Such studies on structural features of IgG led to the development of the processes for preparing humanized antibodies described below. At an early stage of the studies, chimeric antibodies having a variable region from a mouse antibody fused to a constant region from a human antibody were proposed (Morrison SL. et al Proc Natl Acad Sci U S A. (1984)81, 6851-5). However, such chimeric antibodies may induce a HAMA response, especially when they are administered for a long term, because they still contain many non-human amino acid residues (Begent et al., Br. J. Cancer, (1990)62, 487).

A method for further reducing amino acid residues derived from a non-human mammal that may induce a HAMA response to humans by transferring only the CDRs into a human antibody was proposed (Peter T et al. Nature, (1986) 321, 522-5), but grafting of only the CDRs was normally insufficient to maintain immunoglobulin activity against the antigen. On the other hand, Chothia et al. used X-ray crystallographic data in 1987 to find that (a) the amino acid sequences of the CDRs contain a site directly binding to the antigen and a site maintaining the structures of the CDRs, and possible three-dimensional structures of the CDRs are classified into multiple typical patterns (canonical structures), and that (b) the classes of the canonical structures are determined by not only the CDRs but also the types of amino acids at specific locations on the framework (Chothia C. et al. J. Mol. Biol. (1987)196, 901-17). Based on this finding, a document suggested that when CDR grafting is used, amino acid residues on a part of the framework should also be grafted into a human antibody in addition to the CDR sequences (JPA No. HEI-4-502408). Generally, an antibody derived from a non-human mammal having CDRs to be grafted is defined as "donor", and a human antibody into which the CDRs are grafted is defined as "acceptor", and considerations in CDR grafting are to conserve the structures of the CDRs to the extent possible to maintain the activity of the immunoglobulin molecule. To achieve this object, two points should be kept in mind, i.e. (a) which subgroup of acceptor should be selected, and (b) which amino acid residue should be selected from the framework of the donor.

Queen et al. proposed methods for designing immunoglobulins wherein an amino acid residue in the framework of a donor is grafted into an acceptor in addition to the CDR sequences when at least one of the following criteria is satisfied (JPA No. HEI-4-502408):
(a) the amino acid in the framework region of the acceptor is rare for that position and the corresponding amino acid in the donor is common for that position;
(b) the amino acid is immediately adjacent to one of the CDRs; or
(c) the amino acid is predicted to have a side chain atom within about 3 angstroms of the CDRs in a three-dimensional immunoglobulin model and to be capable of interacting with the antigen or with the CDRs of the humanized antibody.

The DNA encoding the heavy or light chain of an anti-Exon-17 monoclonal antibody of the present invention can be obtained by preparation of mRNA from hybridoma cells producing the anti-Exon-17 monoclonal antibody, conversion of the mRNA into cDNA by reverse transcriptase and then isolation of the DNA encoding the heavy or light chain of the antibody.

### Preparation of human antibodies

As used herein, the "human antibody" or "human immunoglobulin" means an immunoglobulin in which all the regions constituting the immunoglobulin including heavy chain variable regions (VH) and heavy chain constant regions (CH) as well as light chain variable regions (VL) and light chain constant regions (CL) are derived from genes encoding a human immunoglobulin. In other words, it means an antibody in which the heavy chain is derived from a human immunoglobulin heavy chain gene and the light chain is derived from a human immunoglobulin light chain gene. Human antibodies can be prepared by standard methods, e.g., by immunization of a transgenic animal prepared by integration of at least a human immunoglobulin gene into the locus of a non-human mammal such as a mouse with an antigen, in the same manner as described above for the preparation of monoclonal antibodies. For example, transgenic mice producing human antibodies can be prepared by the methods described in prior documents (Mendez MJ et al. Nature Genetics (1997)15, 146-56, Green LL et al. Nature Genetics (1994)7, 13-21, JPA HEI-4-504365; International Publication No. WO94/25585; Nikkei Science, June, pp. 40-50, 1995; Nils Lonberg et al. Nature (1994) 368, 856-9, and JPA No. HEI-6-500233).

Antibodies used in the present invention are not limited to whole antibody molecules and may be antibody fragments or derivatives as long as they can inhibit (suppress) the activity of a periostin isoform having anti-cell adhesive activity.

Antibody fragments include, for example, Fab, F(ab')₂, Fv, single chain antibody (scFv), disulfide-stabilized antibody (dsFv), a CDR-containing peptide, etc.

Among the antibody fragments of the present invention, Fab, F(ab')₂ and the like can be obtained by treating an antibody inhibiting the anti-cell adhesive activity of periostin with a proteolytic enzyme such as papain or pepsin, or alternatively, can be prepared by constructing a gene encoding the resulting antibody fragment and introducing this construct into an expression vector, followed by expression in an appropriate host cell.

Among the antibody fragments of the present invention, scFv can be prepared by linking together an H chain V region and an L chain V region from an antibody inhibiting the anti-cell adhesive activity of periostin by using an appropriate peptide linker or the like. Alternatively, scFv can be prepared by constructing a DNA segment encoding the entire sequences or desired amino acid sequences of a gene encoding an H chain or H chain V region from the above antibody and a gene encoding an L chain or L chain V region from the antibody, and introducing this construct into an expression vector, followed by expression in an appropriate host cell.

Among the antibody fragments of the present invention, dsFv is an antibody fragment in which polypeptides modified to replace one amino acid residue by a cysteine residue in both H and L chain V regions from an antibody inhibiting the anti-cell adhesive activity of periostin are linked together between these cysteine residues via a disulfide linkage. An amino acid residue to be replaced by a cysteine residue can be selected by stereostructural estimation of the antibody. dsFv can be prepared by constructing a DNA segment encoding the entire sequence or a desired amino acid sequence of a gene encoding the antibody fragment, and introducing this construct into an expression vector, followed by expression in an appropriate host cell.

Among the antibody fragments of the present invention, a CDR-containing peptide comprises at least one or more CDR regions selected from CDR regions in H or L chains of an antibody inhibiting the anti-cell adhesive activity of periostin. Also, multiple CDR regions may be linked together by techniques using an appropriate peptide linker or the like. The CDR-containing peptide may also be prepared by constructing a DNA segment encoding the entire sequence or a desired amino acid sequence of a gene encoding the peptide, and introducing this construct into an expression vector, followed by expression in an appropriate host cell. Alternatively, the CDR-containing peptide can also be prepared by chemical synthesis such as Fmoc or tBoc method.

In the present invention, it is also possible to use derivatives of the above antibodies or antibody fragments, which are modified to have a protein or low-molecular compound bound thereto. These modifications may be accomplished by known techniques.

DNAs encoding the antibodies, antibody fragments and their protein-bound derivatives of the present invention can be determined in a routine manner. Also, recombinant vectors containing the DNAs can be prepared in a routine manner and introduced in a routine manner into host cells to create transformants. Further, these antibodies, antibody fragments and their protein-bound derivatives can be prepared in a routine manner by culturing the transformants to produce the antibodies, antibody fragments and their protein-bound derivatives in the cultures, and collecting the antibodies, antibody fragments and their protein-bound derivatives from the cultures.

In an embodiment, the antibodies, antibody fragments or derivatives of the present invention can be used to prevent or treat diseases in which a periostin isoform having anti-cell adhesive activity is involved. "Diseases in which a periostin isoform having anti-cell adhesive activity is involved" refer to diseases during which a gene of a periostin isoform having anti-cell adhesive activity is highly expressed and the production of a protein encoded by the gene is increased. They also refer to diseases whose pathology is exacerbated by an increase in the gene or protein. Such diseases in which a periostin isoform having anti-cell adhesive activity is involved are not specifically limited, but include heart failure, myocardial infarction, heart enlargement(dilation), cardiac hypertrophy, cardiac fibrosis, cardiomyopathy, myocarditis, valvular disease, cancers, aneurysms, arteriosclerosis, central neurodegenerative disease, renal diseases, rheumatoid arthritis, osteoporosis, pulmonary emphysema, pulmonary hypertension, chronic obstructive pulmonary disease (COPD), (acute and chronic) nephritis, (acute and chronic) pancreatitis, (acute and chronic) hepatitis, hepatic fibrosis or pulmonary fibrosis. Cancers to which the antibodies of the present invention can be applied include, but not limited to, cancers of breast, large bowel, lung, malignant melanoma, bone, pancreas, stomach, skin, uterus, ovary, rectum, colon, uterus, fallopian tube, esophagus, small intestine, thyroid, parathyroid, adrenal gland, prostate, bladder and kidney, especially cancers of breast, large bowel, lung and malignant melanoma.

The present invention also provides a diagnostic reagent for a disease (e.g., heart failure) in which a periostin isoform having anti-cell adhesive activity is involved, prepared by labeling of an antibody as described above with a marker. Markers that can be used here include enzymes, radioisotopes, fluorescent dyes, etc. The enzymes used here are not specifically limited so long as they satisfy criteria such as high turnover number, stability even after conjugation, and the ability to specifically react with their substrates to develop color, etc., and enzymes used in standard enzyme immunoassays (EIA) can be used. Examples of preferred enzymes include peroxidases, β-galactosidases, alkaline phosphatases, glucose oxidase, acetylcholine esterase, glucose-6-phosphate dehydrogenase, malate dehydrogenase, etc. Enzyme inhibitors and coenzymes and the like can also be used.

Conjugation of these enzymes and antibodies can be performed by known methods using crosslinkers such as maleimide compounds. Substrates that can be used are known materials, selected depending on the enzymes used. For example, when the enzyme used is a peroxidase, 3,3',5,5'-tetramethylbenzidine can be used, or when the enzyme used is an alkaline phosphatase, paranitrophenol or the like can be used. Radioisotopes that can be used as markers include those used in the standard radioimmunoassay (RIA) such as 125I and 3H. Fluorescent dyes that can be used are those used in the standard fluoroimmunoassay such as fluorescence isothiocyanate (FITC) and tetramethyl rhodamine isothiocyanate (TRITC). The present diagnostic reagent can also be used as immunohistological staining capable of specifically staining affected interstitial tissue of the heart. When it is labeled with a radioisotope, it can also be used to image the lesion during heart failure by internal administration.

The present disclosure also provides a method for detecting or quantifying a periostin isoform having anti-cell adhesive activity in a biological sample obtained by preparing serum from human or animal blood, i.e., in serum, comprising the use of the antibody, antibody fragment or derivative of the present invention. The present disclosure further provides a method for diagnosing a disease (e.g., heart failure) in which a periostin isoform having anti-cell adhesive activity is involved, comprising detection or quantification of the periostin isoform. In the present method, a periostin isoform having anti-cell adhesive activity can be detected by a so-called sandwich ELISA (Enzyme-linked immunosorbent assay). When the diagnostic kit of the present disclosure is used, a sample is initially contacted with a plate on which a primary anti-periostin antibody has been immobilized to form a complex, and a secondary anti-periostin antibody labeled with a marker is bound to this complex, and then the signal intensity of the marker in this ternary complex is measured, whereby a periostin isoform having anti-cell adhesive activity can be detected or quantified. In particular, since a periostin isoform having anti-cell adhesive activity is a splicing variant which is specifically expressed during the pathology of heart failure or the like, the pathology in heart failure or the like can be diagnosed by monitoring its production.

In this way, an antibody of the present invention can be used here as the secondary antibody by labeling of the antibody.

Pharmaceutical compositions comprising the antibodies, antibody fragments or derivatives of the present invention as active ingredients may be prepared using carriers and/or excipients or other additives, which are used in standard formulation techniques.

The active ingredients of the pharmaceutical compositions according to the present invention are preferably administered in admixture with known pharmacologically acceptable carriers, excipients, diluents or the like by any administration mode commonly used for pharmaceutical preparations, for example, by the oral or parenteral (e.g., intravenous, intramuscular or subcutaneous) route. For example, the pharmaceutical compositions of the present invention can be prepared by appropriately mixing the active ingredients with physiologically acceptable carriers, flavors, excipients, stabilizers, diluents, emulsifiers, solutions, suspensions, syrups or the like, and can be used in the form of tablets, powders, granules, solutions or the like. Additives which can be incorporated into tablets or the like include, for example, binders such as gelatin and lubricants such as corn starch. Tablets may also be coated with a sugar coating or a gastric or enteric film. In the dosage form of capsules, the above compositions can further comprise liquid carriers. Injectable sterile compositions can also be prepared by applying standard formulae. Injectable aqueous vehicles include isotonic solutions containing glucose and the like, which may be used in combination with appropriate solubilizers such as polyethylene glycol, etc. The compositions may also be incorporated with buffers, stabilizers, preservatives, antioxidants, soothing agents and the like. For oral administration, when the active ingredients are likely to be decomposed in the digestive tract, the compositions may be administered orally as formulations that are resistant to decomposition in the digestive tract, for example, as microcapsules encapsulating the active ingredients within liposomes. It is also possible to use other administration modes intended for absorption through mucous membranes other than the digestive tract, including rectal, intranasal, sublingual and transpulmonary routes. In this case, the compositions can be administered in the form of suppositories, nose drops, sublingual tablets, transpulmonary agents or the like.

When the pharmaceutical compositions of the present invention are used for therapeutic purposes, their dosage is set at a therapeutically effective dosage, which varies depending on, e.g., an age and a body weight of a subject to which the composition is to be administered, a severity of symptoms and a route of administration, and thus administration is determined on an individual basis. In general, the daily adult dosage for oral administration is about 0.1 to 1000 mg, given as a single dose or in divided doses. For continuous intravenous administration, the compositions can be administered in the range of 0.01µg/kg/min to 1.0µg/kg/min, desirably 0.025µg/kg/min to 0.1µg/kg/min.

The following examples further illustrate the present invention in detail and specifically, without, however, thus limiting the invention.

### EXAMPLES

### [Preparation example 1] Search for periostin by subtraction

### 1-1 Preparation of pathologic model rats of heart failure and collection of left ventricular samples

Male Dahl salt-sensitive rats (Dahl-S) (Shimizu Laboratory Supplies) were raised on an 8% high salt diet from 6 weeks of age, and the left ventricle was collected from three animals each at cardiac hypertrophy stage (11 weeks of age) and heart failure stage (14 weeks of age).

### 1-2 Preparation of mRNA

Total RNA was prepared from about 500 mg of the left ventricle using ISOGEN (Nippon Gene) as instructed by the manufacturer. Then, mRNA was purified from about 40 µg of the combined total RNA from three animals each at cardiac hypertrophy stage and heart failure stage using Fast Track 2.0 Kit (Invitrogen) as instructed by the manufacturer to recover about 3µg of mRNA at each stage.

### 1-3 cDNA subtraction

cDNA subtraction was performed using PCR-Select cDNA subtraction kit (Clontech) as instructed by the manufacturer. That is, cDNA was synthesized from 2µg of each mRNA obtained in 1-2 above and digested with restriction enzyme RsaI. Then, subtraction hybridization was performed using the cDNA synthesized from the animals at 14 weeks of age as tester cDNA and the cDNA synthesized from the animals at 11 weeks of age as driver cDNA after 2 adapters included in the kit had been separately linked to the tester cDNA. Then, a cDNA fragment with altered expression level was specifically amplified by PCR using primers complementary to the adapters to give amplification product 1.

A similar subtraction operation was performed using the cDNA synthesized from the animals at 11 weeks of age as tester cDNA and the cDNA synthesized from the animals at 14 weeks of age as driver cDNA to give amplification product 2.

### 1-4 Dot blot screening

### A. Preparation of dot blots

Amplification product 1 was TA cloned into a PCR II vector (Invitrogen) and clones having the insert fragment were selected. The insert fragment of each clone was amplified by PCR reaction, and then 1 µl each of the reaction solution was heat-treated and then dot-blotted on 2 nylon membrane filters (Boehringer) and fixed with a UV crosslinker (Stratagene).

### B. Preparation of cDNA probes

Amplification product 1 was digested with restriction enzymes RsaI and EaeI, SmaI to remove the adapters and subjected to random prime labeling with DIG-dUTP using DIG High Prime DNA labeling/detection kit II (Boehringer) as instructed by the manufacturer to prepare cDNA probe 1. Similarly, cDNA probe 2 was prepared from amplification product 2.

### C. Screening

One of the dot blot membranes prepared in A above was hybridized with cDNA probe 1 and the other with cDNA probe 2. Specifically, hybridization was performed in DIG Easy Hyb solution at 42°C overnight using DIG High Prime DNA labeling/detection kit II (Boehringer) as instructed by the manufacturer. The membranes were washed twice with 2 x SSC, 0.1% SDS at room temperature for 5minutes and twice with 0.1 x SSC, 0.1% SDS at 68°C for 15minutes, and then reacted with alkaline phosphatase-labeled DIG antibodies in the blocking buffer included in the kit, and then CSPD ready-to use was added to advance chemiluminescence and X-ray film was exposed. Clones showing a stronger signal in cDNA probe 1 than cDNA probe 2 were selected as positive clones and sequenced.

### 1-5 Sequencing

The nucleotide sequences were determined by analysis on an automatic DNA sequencer model 373A (PE Applied Biosystems) using THERMO Sequenase™ II dye terminator cycle sequencing kit (Amersham Pharmacia). Thus obtained gene sequences were compared with sequences in the GenBank databank to reveal that one of the clones (SF014) was a gene having an 86% homology to mouse periostin (GenBank Accession No. D13664).

### [Preparation example 2] Cloning of rat periostin cDNA

Rat periostin cDNA was isolated by screening 10 phage subpools of about 4000 clones (a total of about 40,000 clones) prepared from a rat aorta cDNA library (Clontech) inserted into □gt11 vector by PCR using primers (1) 5'-GTTCATTGAAGGTGGCGATGGTC-3' (SEQ ID NO: 15), and (2) 5'-GAGATAAAATCCCTGCATGGTCCT-3' (SEQ ID NO: 16) designed on the basis of the nucleotide sequence of SF014 to give 3 positive subpools. One of the subpools was screened by hybridization using the fragment amplified by PCR as a probe labeled with alkaline phosphatase using AlkPhos Direct™ (Amersham Pharmacia) to give one positive clone rat periostin #1. Its insert fragment was subcloned into the EcoRI site of pBluescript II (Stratagene) and the total nucleotide sequence was determined according to the method of Preparation example 1-5.

The resulting clone had a length of about 3kb corresponding to nucleotide 292 to the 3' end of mouse periostin (GenBank Accession No. D13664), suggesting that it was a 5'-truncated clone.

Thus, SMART™ RACE cDNA Amplification Kit (Clontech) was used as instructed by the manufacturer to perform 5'-RACE reaction using rat aorta cDNA as a template and the primer (2) 5'-GAGATAAAATCCCTGCATGGTCCT-3' (SEQ ID NO: 16) and a primer (3) 5'-CACGGTCGATGACATGGACAACACC-3' (SEQ ID NO: 17) designed on the basis of the nucleotide sequence of rat periostin #1. The resulting PCR product was TA cloned into PCR II vector of Invitrogen to give a clone designated as rat periostin 5'RACE #1. The nucleotide sequence was determined according to the method of Preparation example 1-5.

The results showed that rat periostin 5'RACE #1 was a clone longer by about 300bp than the initially obtained rat periostin #1 in the 5' direction with the 5' end being longer by 15bp than the 5' end of mouse periostin (GenBank Accession No. D13664). Ten phage subpools of about 40,000 clones (a total of about 400,000 clones) prepared from the rat aorta cDNA library were screened by PCR using a primer (4) 5'-ACGGAGCTCAGGGCTGAAGATG-3' (SEQ ID NO: 18) designed on the basis of the nucleotide sequence of rat periostin 5'RACE #1 and the primer (3) 5'-CACGGTCGATGACATGGACAACACC-3' (SEQ ID NO: 17) to give 2 positive subpools. One of the subpools was screened by hybridization using the fragment amplified by PCR as a probe to give one positive clone designated as rat periostin #2. The insert fragment was subcloned into the EcoRI site of pBluescript II (Stratagene) and the nucleotide sequence was determined according to the method of Preparation example 1-5.

The resulting clone had a length of about 2.6 kb with the 5' end being the same as that of the clone obtained with 5'-RACE and the 3' end corresponding to up to nucleotide 2410 of mouse periostin (GenBank Accession No. D13664). The nucleotide sequence of rat periostin 5'RACE #1 previously obtained was exactly the same as the nucleotide sequence of the relevant region of rat periostin #2. The full length of rat periostin cDNA was completed by rat periostin #1 and rat periostin #2. The nucleotide sequence of this full-length cDNA and the amino acid sequence translated from this nucleotide sequence are shown as SEQ ID NOs: 6 and 1.

### [Preparation example 3] Construction of a Myc-His-rat periostin fusion protein expression vector

An expression vector having a Myc epitope and 6 histidine tags at the carboxyl terminus of the protein translated from the coding region of the rat periostin gene obtained in Preparation example 2, and having a CMV promoter, was prepared.

Initially, a fragment of about 500bp obtained by digestion of rat periostin 5'RACE #1 obtained in Preparation example 2 with restriction enzymes EcoRI and HindIII and a fragment of about 2780bp obtained by digestion of rat periostin #1 obtained in Preparation example 2 with restriction enzymes HindIII and HpaI were ligated to a vector fragment obtained by digesting pTracer-CMV2 vector (Invitrogen) with restriction enzymes EcoRI and EcoRV using a ligation kit (Takara Bio Inc.) to give a plasmid designated as pTracer-CMV2/rat periostin. Thus prepared pTracer-CMV2/rat periostin was digested with restriction enzymes EcoRI and SmaI to give a fragment of about 2330 bp containing the coding region of the rat periostin gene, and PCR was performed using rat periostin #1 obtained in Preparation example 2 as a template and primer (5) 5'-GACCCGGGAAGAACGCATCATC-3' (SEQ ID NO: 19) designed on the basis of the sequence of the template and primer (6) 5'-TGGGTGACCCTGAGAACGGCCTTCTCTTGATC-3' (SEQ ID NO: 20) designed to insert a BstEII site immediately before the stop codon of rat periostin and the amplification product was purified and then digested with restriction enzymes SmaI and BstEII to give a fragment of about 270bp. These two fragments were ligated to a vector fragment obtained by digestion of an expression vector constructing plasmid pcDNA4/Myc-His/type C (Invitrogen) with restriction enzymes EcoRI and BstEII using a ligation kit (Takara Bio Inc.) to give a plasmid designated as pcDNA4/Myc-His/rat periostin. The total nucleotide sequence of the insert was confirmed by the method described in Preparation example 1-5.

### [Preparation example 4] Construction of a baculovirus expression vector

The plasmid pcDNA4/Myc-His/rat periostin obtained in Preparation example 3 was digested with restriction enzymes SacI and PmeI to excise a peptide fragment rat PN-1/Myc-His. This fragment was ligated to a vector fragment obtained by digesting pFastBacHTc (Invitrogen) with restriction enzymes SacI and KpnI (blunting) using a ligation kit (Takara Bio Inc.) to give an expression vector designated as pFastBac/rat periostin-1/Myc-His. The nucleotide sequence of the insert was confirmed by the method described in Preparation example 1-5.

### [Preparation example 5] Preparation and cultivation of a recombinant baculovirus

DH10BAC cells of Escherichia coli were transformed with pFastBac/rat periostin-1/Myc-His obtained in Preparation example 5 to prepare a recombinant baculovirus. Electrophoresis and PCR confirmed that the resulting baculovirus contains the desired insert.

Insect Sf9 cells (2 x 10⁶cells/mL) infected with this recombinant baculovirus at MOI=0.1 were cultured in a serum-free medium (containing 50µg/mL gentamicin in 2000mL of Sf-900IISFM (Invitrogen)) at 28°C for 4-5 days and then the culture supernatant was harvested.

### [Preparation example 6] Purification of rat periostin protein

To an SP Sepharose Fast Flow column (10mL bed volume) equilibrated with an equilibration buffer (50mM sodium acetate buffer, pH 6.0, 0.1M sodium chloride) was applied 2000mL of the culture supernatant obtained in Preparation example 5, and the resulting flow-through fraction was pooled as an SP Sepharose flow-through fraction.

The column was washed with the equilibration buffer until the absorbance at 280nm approached 0 (about 100mL) to give an SP Sepharose wash fraction.

The column was eluted with 100mL of an elution buffer (50mM sodium dihydrogen phosphate (pH8.0), 0.5M sodium chloride, 5mM imidazole) to give an SP Sepharose eluate fraction.

Then, 100mL of the SP Sepharose eluate fraction was applied to an Ni-NTA agarose column (5mL bed volume) equilibrated with 50mM sodium phosphate buffer, pH8.0, 0.5M sodium chloride and 5mM imidazole, and the resulting flow-through fraction was pooled as an Ni-NTA agarose flow-through fraction.

The column was washed with about 50mL of a washing buffer (50mL sodium dihydrogen phosphate, pH8.0, 0.5M sodium chloride, 5mM imidazole) to give an Ni-NTA agarose wash fraction.

The column was eluted with about 25mL each of elution buffers (1) 50mM sodium dihydrogen phosphate, 0.5M sodium chloride, 20mM imidazole, followed by similar compositions except that the imidazole concentrations were (2) 30mM, (3) 40mM, (4) 50mM and (5) 60mM to give Ni-NTA agarose eluate fractions (1)-(6).

Fractions shown to contain the desired protein by Western blotting were concentrated to 1mL or less.

Then, the concentrated samples were applied to a gel filtration column (Sephacryl S-200HR φ11mm x 95cm; 90 bed volume) equilibrated with degassed PBS (-) (137mM NaCl, 8.1 mM Na₂HPO4, 2.68mM KCl, 1.47mM KH₂PO₄) and eluted with PBS (-) and the eluate was lyophilized to give a purified rat periostin protein.

### [Example 1]

### Synthesis of rat Exon-17 peptide chain and preparation of polyclonal antibody against it

A structure specific to rat PN-1 was identified as Exon-17 sequence by sequence comparison since heart dilation was induced by increased expression of the PN-1 gene in the heart of normal SD rats and the survival rate was improved by administration of an antisense oligonucleotide against rat periostin to the heart of Dahl heart failure model rats and rat PN-1 was shown to have no cell adhesive properties in contrast to previously reported PN-2. A peptide having a Cys residue added to the N-terminus of the amino acid sequence constituting this Exon-17 was chemically synthesized in 10mg yield at a purity of 80% or more. Rabbits (Kbl:JW) were immunized with the polypeptide coupled to 6 mg of a carrier protein KLH. FCA (Freund's complete adjuvant) was used in the primary immunization, and FIA (Freund's incomplete adjuvant) was used in the secondary and subsequent immunizations. Administration was performed at 20 dorsal subcutaneous sites at weeks 0, 2, 4 and 6 using a peptide dose of 800µg/animal in the primary immunization, and 400 µg/animal in the secondary and subsequent immunizations. The antibody titer was determined by ELISA and total sera were collected at week 7. Then, an affinity column was prepared by use of a synthetic peptide, and only the antibody specifically reacting to the Exon-17 peptide was collected. The polyclonal antibody against the peptide encoded by Exon-17 of rat periostin is hereinafter referred to as anti-rat Exon-17 peptide antibody.

### [Example 2]

### In vitro study of the presence or absence of anti-cell adhesive activity of rat PN-1

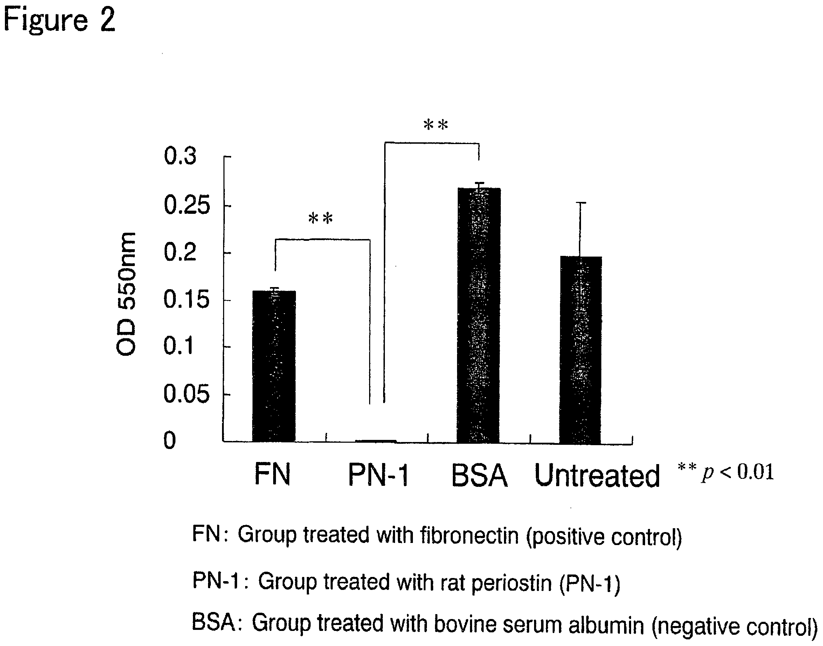

Rat heart fibroblasts were obtained by a method similar to those described in literature (Ruwhof C, van Wamel AE, Egas JM, van der Laarse A. Mol Cell Biochem. 2000 May; 208 (1-2):89-98, Ashizawa N, Graf K, Do YS, Nunohiro T, Giachelli CM, Meehan WP, Tuan TL, Hsueh WA. J Clin Invest. 1996 Nov. 15; 98 (10):2218-27). Specifically, 20 SD rats at 1-2 days of age were anesthetized with ether and the chest was disinfected with ethanol. The heart was isolated and placed in a dish containing PBS (-), where the heart was transversely incised to bleed it and further washed with PBS (-) three times, and then PBS (-) was discarded to the minimum possible level and the heart was minced with scissors. Then, the minced tissue was agitated in a 1:1 mixture of PBS (-): collagenase/trypsin at 37°C for 15 minutes, and then cells were lysed by pipetting as thoroughly as possible. Then, the cell lysate was filtered through a platinum mesh in a centrifugal tube, and the platinum mesh was washed with 10ml of M199 medium containing 10% serum and 10ml of PBS (-). Then, the centrifugal tube was spun at 1500rpm for 10 minutes, and the cells obtained as a pellet fraction were stirred in 20ml of M199 medium containing 10% serum and plated on a dish. The dish was allowed to stand at 37°C for 1 hour, and then cells adhered to the dish were collected as rat heart fibroblasts. The rat heart fibroblasts were plated on a 96-well plate at a density of 6.4 x 10⁴ cells/100 µl and cultured overnight, and then the culture was incubated in fresh DMEM medium with 10% FBS containing 10µg/ml cycloheximide at 37°C for 1 hour. Then, the cells were washed twice with DMEM medium (serum free) prewarmed at 37°C, and rat periostin protein prepared according to the Preparation examples was added to DMEM medium (serum free) at a final concentration of 10µg/ml. Fibronectin having cell adhesion-promoting properties was used as a positive control and BSA (bovine serum albumin) was used as a negative control. After incubation at 37°C for 1 hour, microscopy showed that all the cells were separated in the group treated with rat periostin protein, and the cells were washed twice with PBS (-) and then fixed in 10% neutral buffered formalin for 30 minutes. Then, the cells were washed with PBS (-) three times and then stained with crystal violet for 30 minutes. Then, the degree of staining was measured using a plate reader at 550nm (BIO-RAD, Model 680 MICRO PLATE READER) (Figure 2). As a result, the control groups treated with fibronectin and BSA and the untreated group did not show anti-cell adhesive properties because the adhered cells were not separated, in contrast to the group treated with rat PN-1 in which the cells were separated, showing that rat PN-1 has a separating effect on adhered cells, i.e., anti-cell adhesive properties.

### [Example 3]

### In vitro study of the neutralizing activity of anti-rat Exon-17 peptide antibody

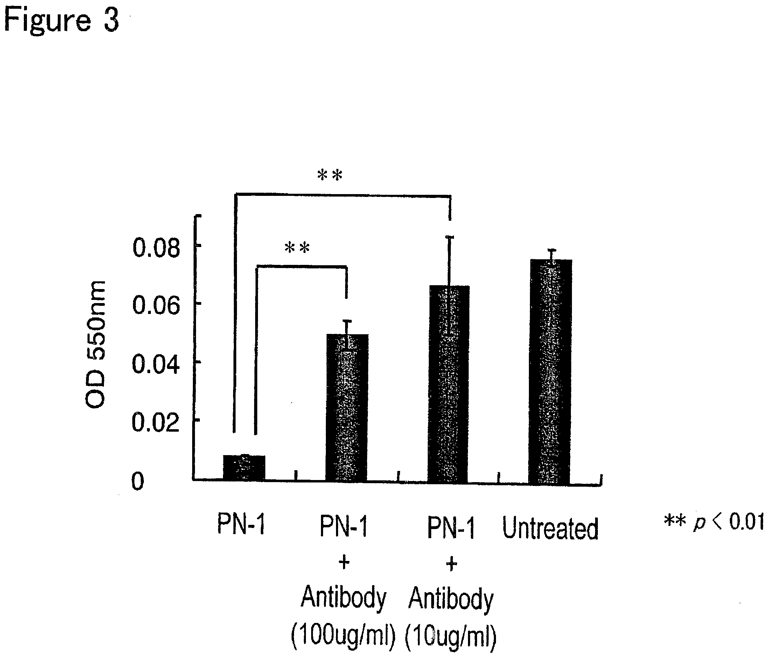

SD rat heart fibroblasts obtained by a method similar to that of Example 2 were plated on a 96-well plate at a density of 6.4 x 10⁴cells/100µl and cultured overnight, and then the culture was incubated in fresh DMEM medium with 10% FBS containing 10µg/ml cycloheximide at 37°C for 1 hour. Then, the cells were washed twice with DMEM medium (serum free) prewarmed at 37°C, and rat periostin protein and anti-rat Exon-17 peptide antibody were added to DMEM medium (serum free) at final concentrations of 10µg/ml and 100µg/ml, respectively. Rat periostin protein alone was used as a positive control and BSA was used as a negative control. After incubation at 37°C for 1 hour, microscopy showed that all the cells were separated in the group treated with rat periostin protein alone, and the cells were washed twice with PBS (-) and then fixed in 10% neutral buffered formalin for 30 minutes. Then, the cells were washed with PBS (-) three times and then stained with crystal violet for 30 minutes. Then, the degree of staining was measured using a plate reader at 550nm (BIO-RAD, Model 680 MICRO PLATE READER) (Figure 3). The results showed that anti-rat Exon-17 peptide antibody is an antibody having the activity of inhibiting PN-1-induced separation of adhered cells, i.e., inhibiting the anti-cell adhesive properties of PN-1, i.e., neutralizing the anti-cell adhesive properties of rat PN-1.

### [Example 4]

### Effect of anti-rat Exon-17 peptide antibody on acute myocardial infarction model rats

A male Lewis rat weighing 250-300g was fixed on a rat surgical table after the animal was thoroughly anesthetized by peritoneal administration of pentobarbital (0.1ml/100g). A tube was orally inserted into the trachea and connected to a rat ventilator (tidal volume 3ml, 80 breaths/min), and the skin was laterally incised from the left third intercostal space of the sternum and the underlying greater pectoral muscle was also laterally incised, and the intercostal space was opened using a rat rib spreader to expose the heart.

Then, the left coronary artery nearly beneath the left atrium was ligated with 1.0 silk using a curved needle having a diameter of 5mm. After visual confirmation that the anterior and lateral walls along which the left coronary artery runs had been changed from red to white to show sufficient blockage of the coronary bloodstream and the disappearance of wall motion at these sites (in the sham operation group, the needle was passed through the coronary artery and then removed without ligation), the third and fourth ribs were fixed by ligation with 3.0 silk (after the lung was expanded to remove the air existing outside the lung in the rib cage so that the lung can be easily expanded). The incision site in the skin was sutured with 3.0 silk in the same manner and then observed for a while, and the tube was removed after confirmation of recovery of consciousness and resumption of spontaneous breathing.

Acute myocardial infarction models were sequentially prepared by the foregoing procedure.

On the following day, percutaneous echocardiography was performed under intranasal anesthesia with isoflurane, and small infarction models having an infarction size less than 20% of the entire periphery of the left ventricle were excluded. The remaining infarction models were ranked in order of increasing heart function, and alternately classified into a group treated with anti-rat Exon-17 peptide antibody and a group treated with a control antibody (rabbit IgG) each 200µg via tail vein.

The antibodies were administered to each group on the day following the preparation of the models and at intervals of 6 days after the initial administration, a total of 4 times.

The heart was evaluated by echocardiography through the chest wall at intervals of one week until the end of 8 weeks. At the end of 8 weeks, a tube was inserted into the trachea and connected to a ventilator under anesthesia with pentobarbital, and the skin was incised from the left neck and the neck muscles were retracted with forceps to expose the left common carotid artery, and after bleeding was stopped by ligation at the origin of the left carotid artery, the artery was pierced at a distal site by small scissors and a rat mirror catheter was inserted from that site in such a manner that the catheter tip with a pressure sensor reached the inside of the left ventricle while the catheter was connected to a computer to measure heart function and blood pressure and the like.

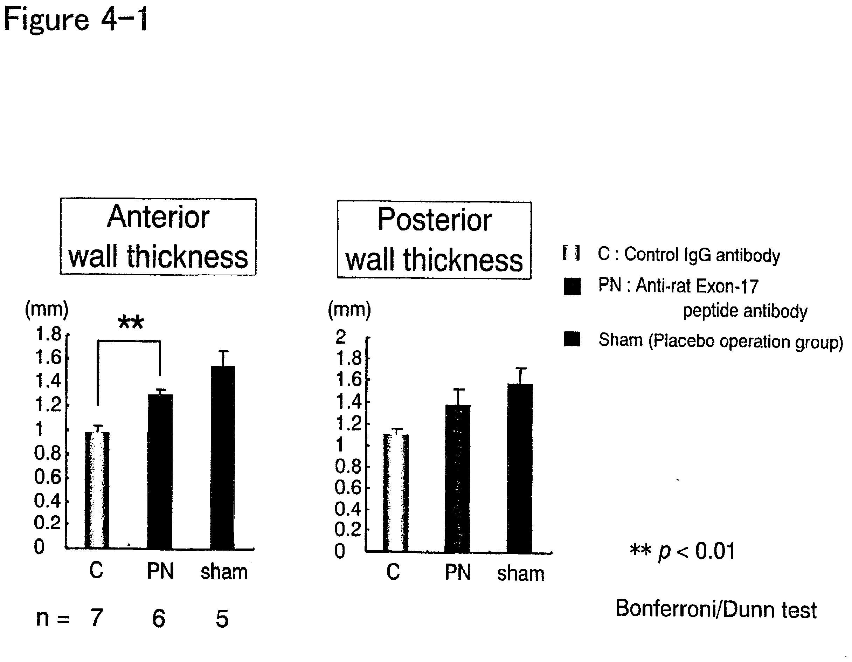

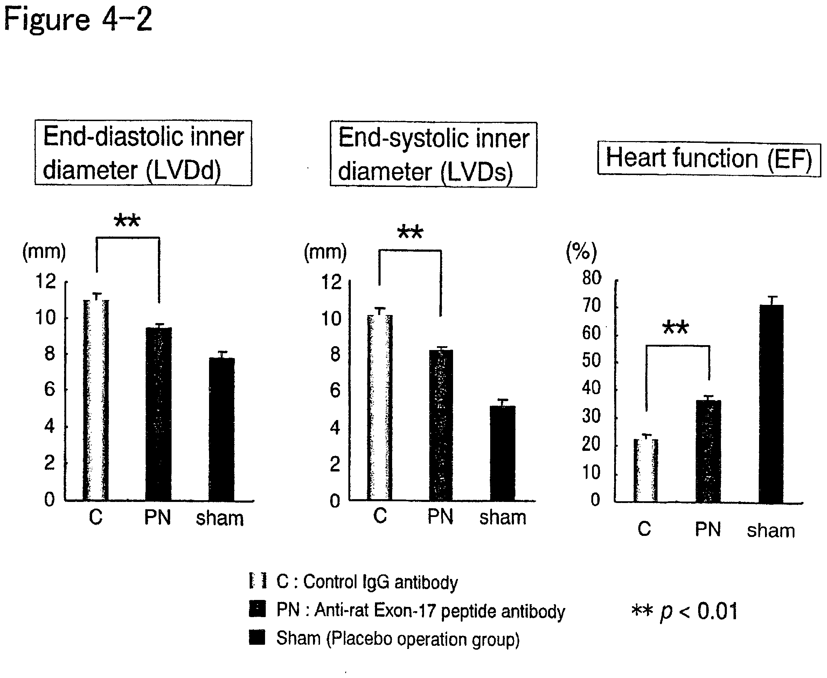

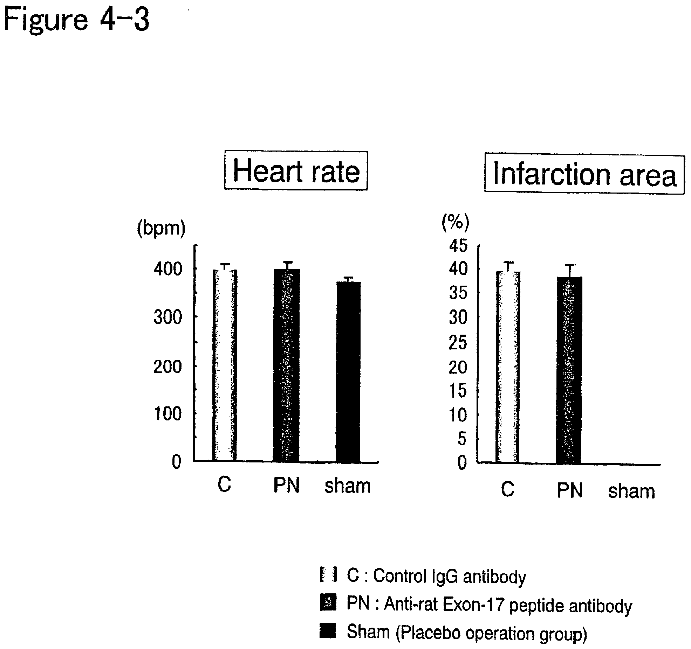

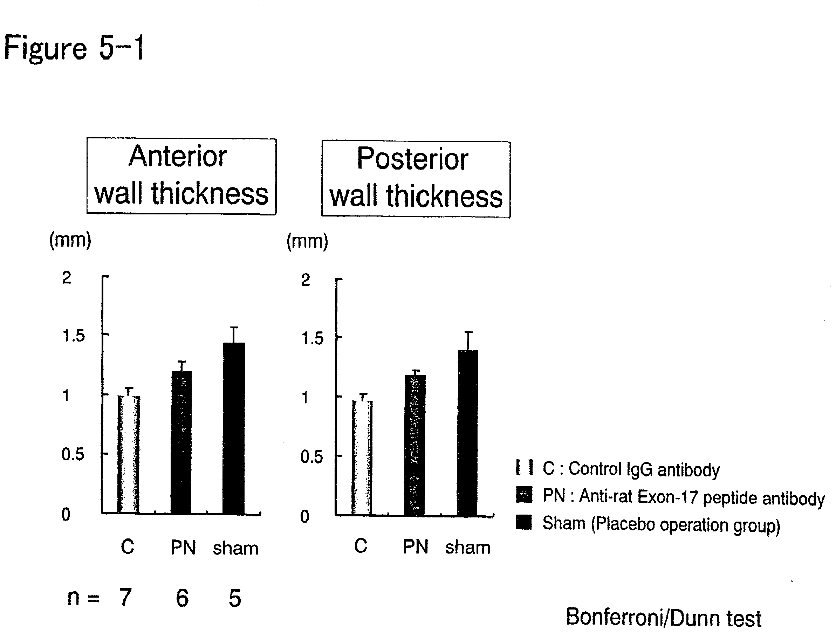

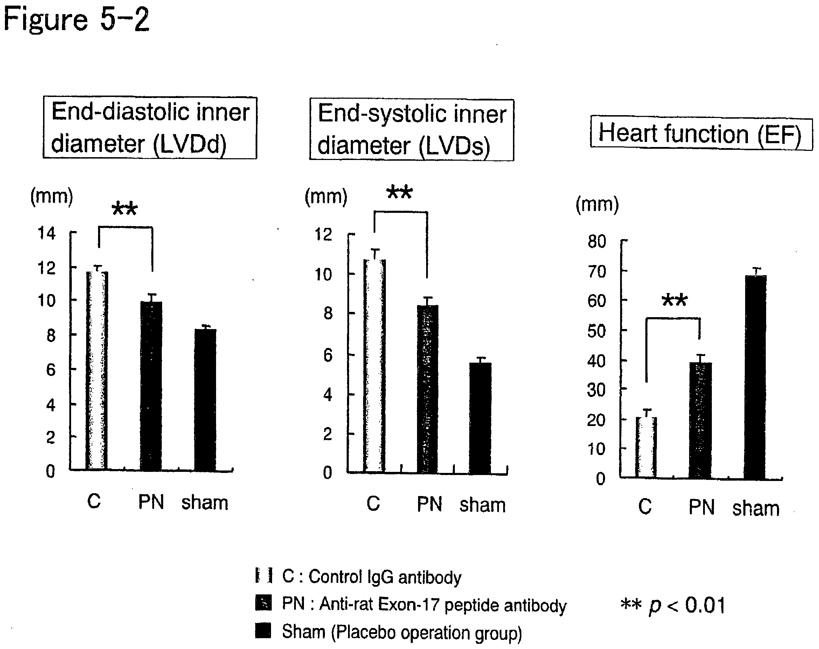

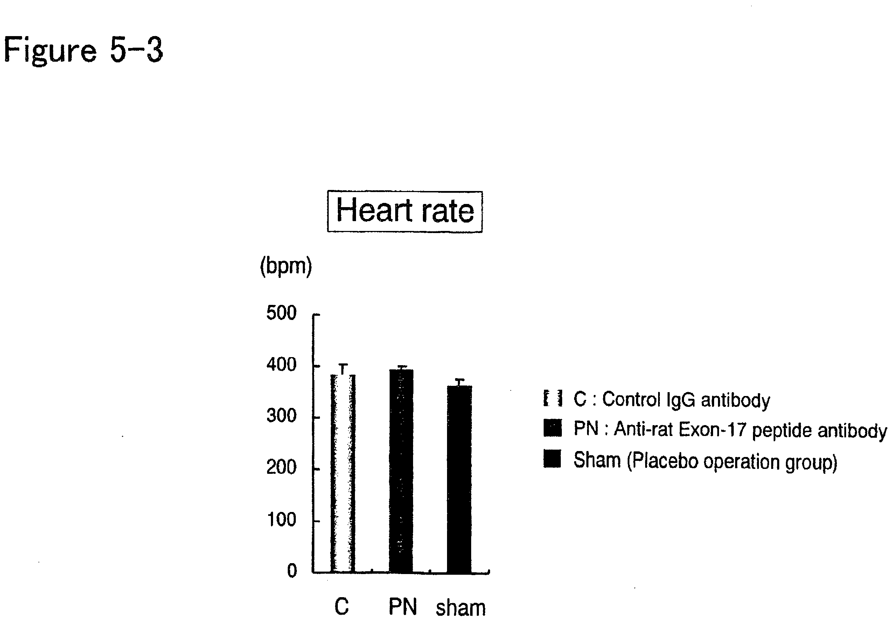

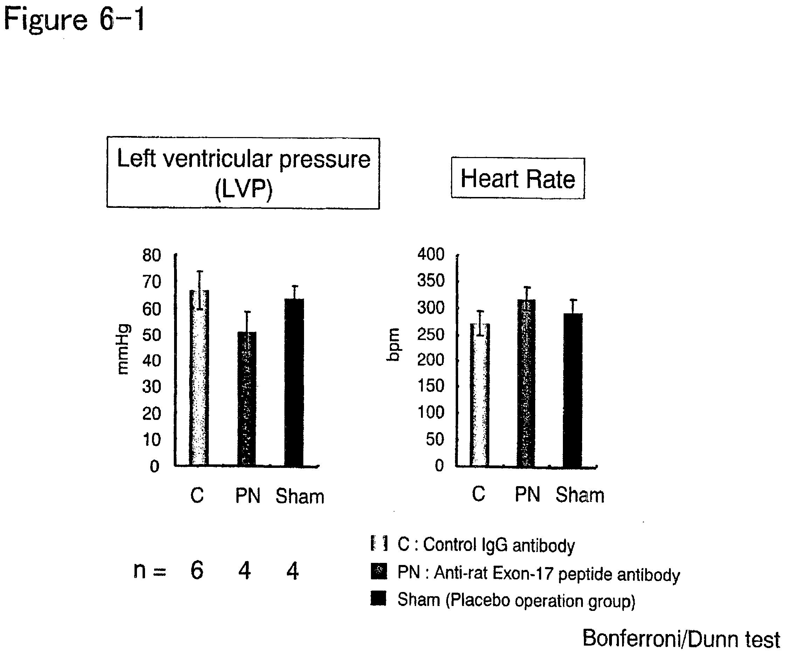

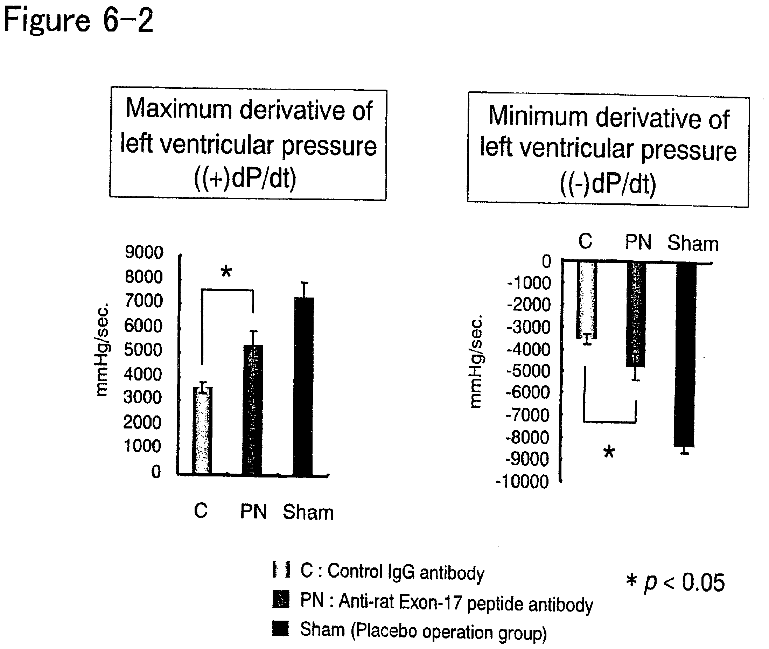

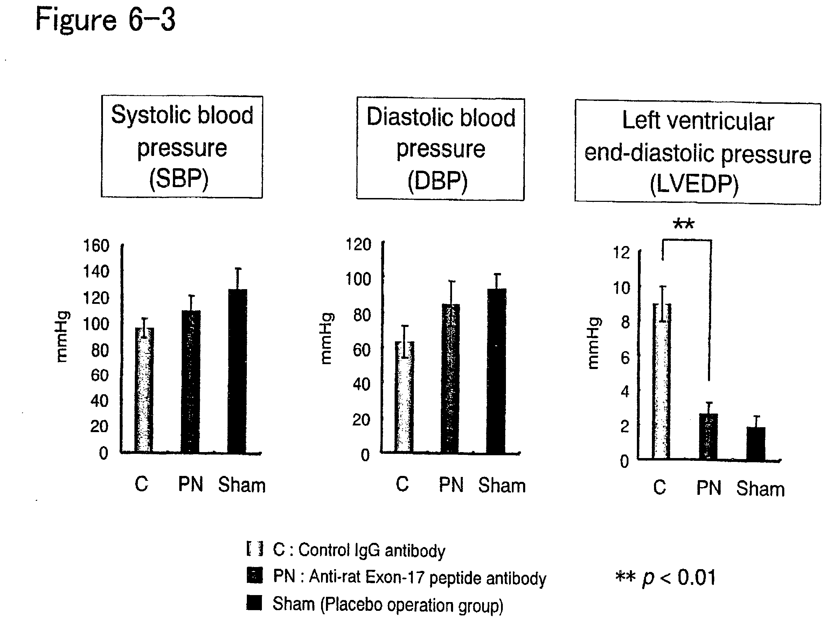

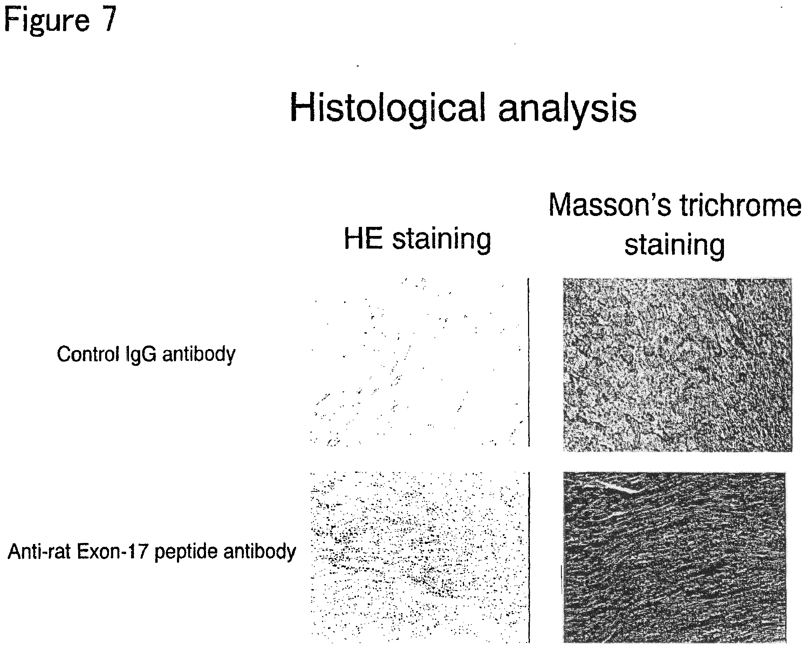

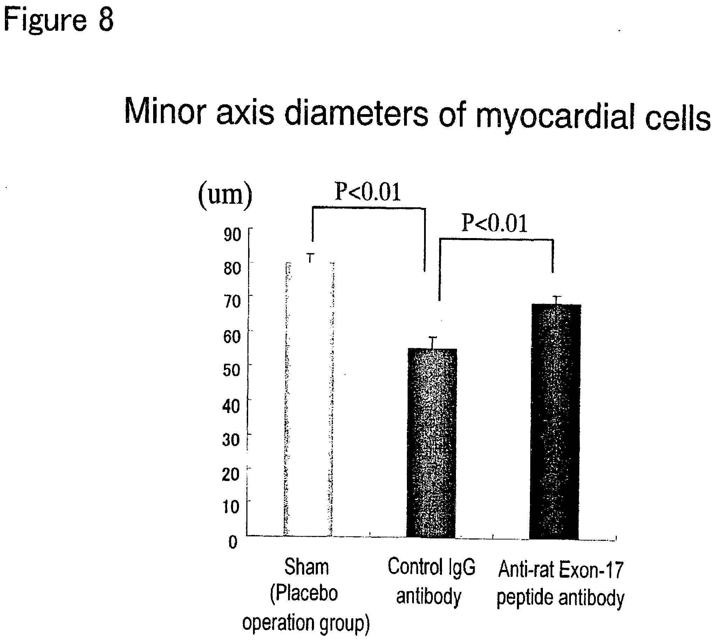

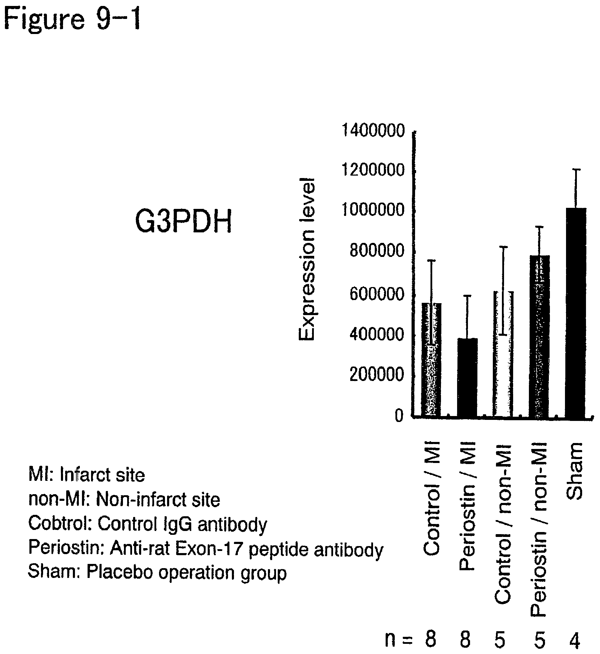

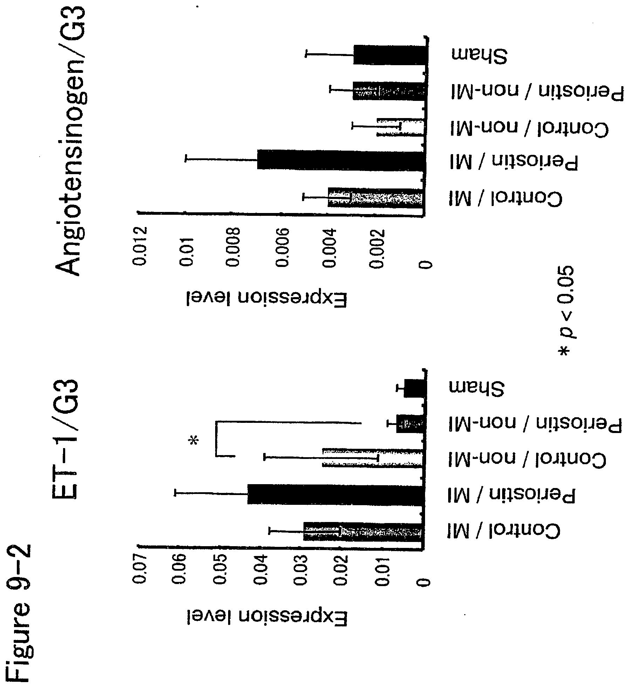

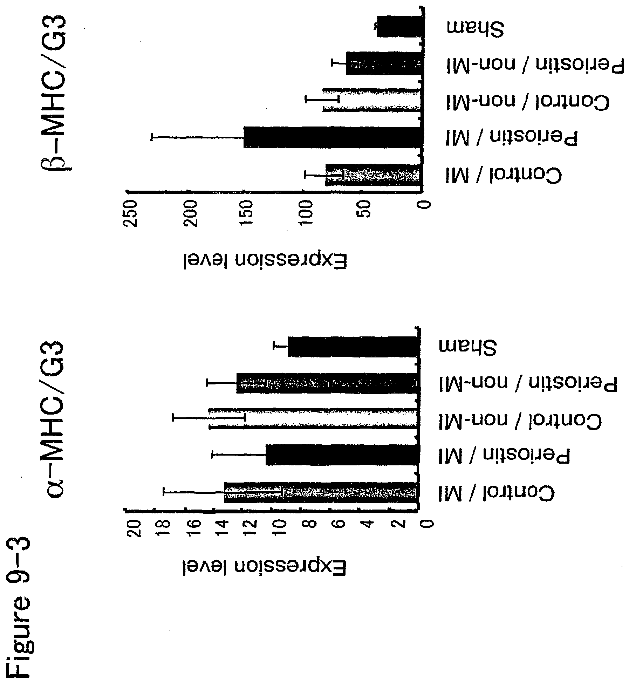

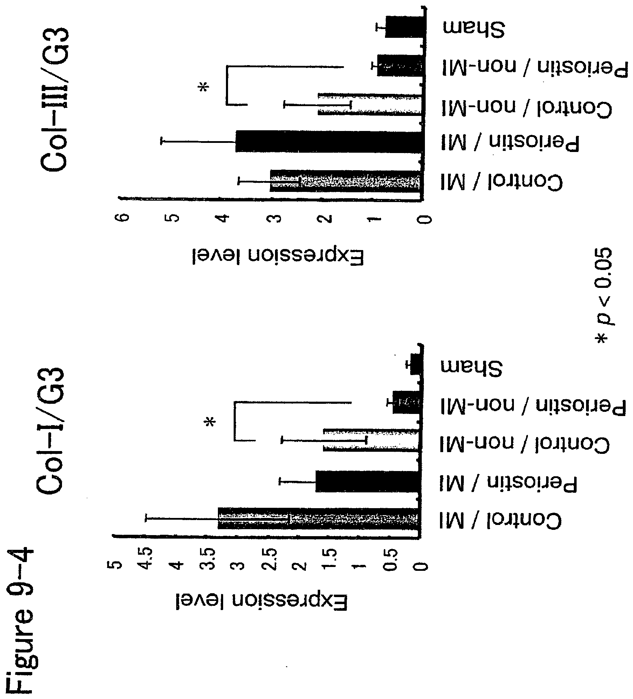

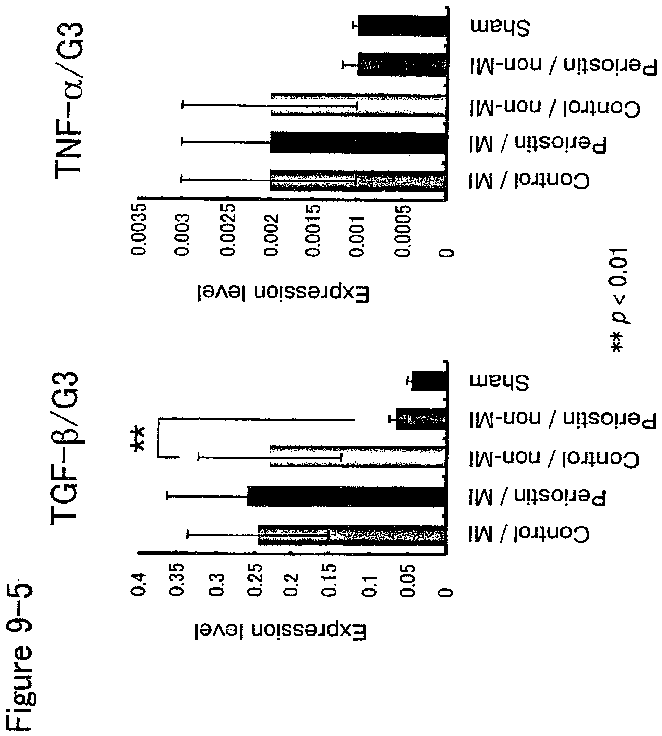

The results of echocardiography 4 weeks after the preparation of the models showed that the reduction of the anterior wall thickness and posterior wall thickness of the heart was inhibited, the increase of the end-diastolic inner diameter and end-systolic inner diameter was inhibited, and that the EF value indicative of the contractile function of the heart increased in the group treated with anti-rat Exon-17 peptide antibody significantly as compared with the control group treated with rabbit IgG. In brief, heart dilation was inhibited, showing that heart function was improved (Figure 4-1 to Figure 4-3). The results of echocardiography 8 weeks after the preparation of the models also showed inhibition of heart dilation and improvement of heart function in the same manner as the results after 4 weeks (Figure 5-1 to Figure 5-3). These results suggested that the effect of anti-rat Exon-17 peptide antibody inhibiting heart dilation and improving heart function is maintained even about 4 weeks after the administration of the antibody. Then, hemodynamics showed significant differences in maximum derivative of left ventricular pressure ((+)dP/dt), minimum derivative of left ventricular pressure ((-)dP/dt) and left ventricular end-diastolic pressure (LVEDP) in the group treated with anti-rat Exon-17 peptide antibody as compared with the control group treated with IgG, suggesting that heart function was improved (Figure 6-1 to Figure 6-3). In heart sections stained with Masson trichrome, blue sites decreased in the group treated with anti-rat Exon-17 peptide antibody as compared with the control group treated with IgG, showing that fibrosis was inhibited (Figure 7). Analysis of the minor axis diameters of myocardial cells showed that the reduction of the minor axis diameters of myocardial cells was significantly inhibited in the group treated with anti-rat Exon-17 peptide antibody as compared with the control group treated with IgG (Figure 8). This result correlates with the result of echocardiography. Moreover, the results of gene expression analysis in infarct sites and non-infarct sites showed that the expression levels of endothelin-1 (ET-1), collagen type I, III, and TGF-beta at non-infarct sites significantly decreased in the group treated with the neutralizing antibody as compared with the group treated with the control antibody to levels comparable to those of the sham group, indicating that the condition was improved (Figure 9-1 to Figure 9-5).

### [Example 5] Cloning of full-length human periostin-1 cDNA

cDNA prepared from 1µg of human heart-derived total RNA (Clontech, catalog No. 64100-1, lot No. 4120493) was used as a template to perform PCR with KOD plus DNA polymerase (Toyobo Co., Ltd.) using the following primers for full-length cloning: sense chain 5'-AAGCTAGCCACCATGATTCCCTTTTTACCCAT-3' (SEQ ID NO: 27) and antisense chain 5'-AACTCCACAATTTCCCTCAT-3' (SEQ ID NO: 28). The resulting PCR products were cloned using a Zero Blunt TOPO PCR Cloning kit (Invitrogen).

A sense chain 5'-TAACCAAAGTTGTGGAACCAA-3' (SEQ ID NO: 29) was prepared from a region corresponding to human periostin Exon-17, while an antisense chain 5'-TGTGTCTCCCTGAAGCAGTC-3' (SEQ ID NO: 30) was prepared from a region corresponding to Exon-21, followed by selection of clones detected with these primers from this clone group. Then, the nucleotide sequences of the selected clones were determined to select an unspliced clone, thereby completing the full-length cloning of human periostin-1 cDNA. The resulting clone was designated as pCR4/human periostin-1.

### [Example 6]

### Construction of human periostin-1 expression vector for in vitro translation

The plasmid pCR4/human periostin-1 obtained in Example 5 was digested with restriction enzymes Pme I and Not I to excise a DNA fragment human periostin-1, which was then blunted. A pTNT expression vector (Promega), into which CATCACCATCACCATCACTAA (6 x His + termination codon) (SEQ ID NO: 31) had been inserted, was enzymatically digested at the Mlu I site in its multicloning site and then blunted. The DNA fragment obtained above was ligated to this vector using a ligation kit (TaKaRa Bio Inc.).

Then, for the purpose of creating in-frame fusion with the His tag, synthetic linkers (sense chain 5'-CTAGAAGACGATTAAGGGAAGGTCGTTCTCAGCTGGAAGTTCTGTTCCAGGGGCCC-3' (SEQ ID NO: 32) and antisense chain 5'-GGGCCCCTGGAACAGAACTTCCAGCTGAGAACGACCTTCCCTTAATCGTCTT-3' (SEQ ID NO: 33)) were prepared and ligated to the vector fragment digested with restriction enzymes Xba I and Sma I using a ligation kit. The nucleotide sequence of the ligated part was confirmed, and the resulting expression vector was designated as pTNT/human periostin-1/His.

### [Example 7]

Protein synthesis by *in vitro* translation The expression vector obtained in Example 6 was provided for *in vitro* protein synthesis with TNT SP6 Quick Coupled Transcription/Translation Systems (Promega). More specifically, relative to 2µg of the pTNT/human periostin-1/His expression vector, 40µl of SP6 Quick Master Mix and 1µl of 1mM methionine were added and diluted with DEPC-treated water to give a total volume of 50µl, followed by reaction at 30°C for 90 minutes. The reaction product was stored at -80°C until purification.

### [Example 8]

### Purification of human periostin protein (PN-1)

The synthetic protein obtained in Example 7 was purified using a MagZ Protein Purification System (Promega). More specifically, to the synthetic protein obtained in Example 7, 2 volumes of MagZ Binding/Wash buffer were added and mixed well. The sample thus prepared was added to MagZ Binding Particles. After stirring of this mixture at 4°C for 1 hour, the supernatant was removed and the MagZ Binding Particles were washed four times with MagZ Binding/Wash buffer, followed by elution of the synthetic protein with MagZ Elution buffer. The protein thus purified was stored at -80°C until use.

### [Example 9]

### Preparation of monoclonal antibody against human periostin Exon-17 peptide chain

### (1) Antigen preparation

A peptide (antigen peptide; SEQ ID NO: 25) having a Cys residue added to the N-terminus of the amino acid sequence constituting human periostin Exon-17 (SEQ ID NO: 4) was chemically synthesized by the Fmoc method to obtain the antigen peptide in 10mg yield at a purity of 90% or more. As a carrier protein, KLH (5mg, CALBIOCHEM) was coupled to this antigen peptide to give an antigen solution. Namely, KLH was dissolved in PBS (0.01M) and adjusted to 3.3mg/mL, to which a 0.2524 mg/mL MBS solution (GE Healthcare Bio-Sciences KK) was then added dropwise and reacted with stirring at room temperature for 60 minutes. Dichloromethane was used to remove free MBS, to thereby obtain KLH-MB. This KLH-MB (5mg) was mixed with the antigen peptide (5mg) dissolved in 0.01M sodium phosphate buffer (pH7.2) and reacted with stirring at 4°C for 12 hours to obtain the antigen solution.

### (2) Immunization

Three female BALB/c mice at 6 weeks of age were each subcutaneously injected into both paws with the whole volume of a mixed emulsion of the antigen solution (50µl) containing 100µg KLH-coupled antigen peptide obtained in (1) and FCA (Freund's complete adjuvant, 50µl). The mice were then injected twice into both paws with an *in situ* prepared mixed emulsion of the above antigen solution and FIA (Freund's incomplete adjuvant) at an interval of 2 weeks. The mice were then sacrificed by cervical dislocation and lymph nodes in their paws were aseptically collected.

While supplying RPMI medium (Kohjinbio Co., Ltd.), the above lymph nodes were crushed and passed through a mesh of about 10µm pore size to obtain lymph node cells suspended in RPMI medium. This suspension was centrifuged at 1000rpm for 10 minutes to obtain lymph node cells as a pellet fraction. After this pellet fraction was hemolyzed to remove red blood cells in a solution (1ml) prepared by adding 20mM HEPES buffer (pH7.4) to a 0.84% ammonium chloride solution, centrifugation was repeated at 1,000rpm for 5 minutes. The resulting pellet fraction (cell fraction) was washed several times with RPMI medium and then used for cell fusion.

### (3) Preparation of myeloma cells

The mouse myeloma cell line P3X63Ag8U.1 (P3U1) that was resistant to 8-azaguanine and secreted no immunoglobulin was cultured in RPMI medium containing 20% fetal calf serum (FCS) in a 10% CO₂, 37°C incubator. Cells in the logarithmic growth phase were collected and centrifuged at 1,000rpm for 5 minutes to obtain the cells alone as a pellet fraction, which were then suspended in RPMI medium.

### (4) Cell fusion

The RPMI medium obtained in (2) containing 10⁸ to 3 x 10⁸ immunized lymph node cells and the RPMI medium obtained in (3) containing 10⁸ myeloma cells were mixed and then centrifuged at 1 ,000rpm for 10 minutes. The supernatant was gently removed to obtain the cells as a pellet fraction, followed by addition of 1ml of 25% (w/v) polyethylene glycol 1500 (PEG 1500, Boehringer). The cells were further diluted to a total volume of 10ml by slow addition of RPMI medium. To this suspension, 20% FCS-containing RPMI medium (10ml) was added and allowed to stand for a while, followed by centrifugation at 1,000rpm for 5 minutes. The resulting pellet fraction (cell fraction) was adjusted to a cell density of 10⁶cells/ml by addition of 20% FCS-containing RPMI, and this cell suspension was dispensed at 200µl/well in 96-well culture plates (Corning). After culturing in a 5% CO₂, 37°C incubator for 24 hours, HAT solution (Invitrogen) was added and culturing was continued for an additional 2 weeks.

### (5) Screening by ELISA

Screening was performed to determine positive wells showing a reaction between the culture supernatant and the antigen peptide.

For use as an antigen solution for assay, the antigen peptide (2mg) obtained in (1) was coupled to ovalbumin (OVA) as a carrier protein to prepare a conjugate.

Each well of a 96-well microtiter plate (Falcon 353912) was coated with the above conjugate (1µg/ml) by standing overnight at 4°C. After washing this plate, the culture supernatant from (4) (50µl, containing monoclonal antibodies) was added dropwise to each well and allowed to stand in a 37°C incubator for 2 hours, followed by washing with PBS(-) (phosphate buffered saline). After addition of alkaline phosphatase-conjugated sheep anti-mouse IgG antibody (Zymed), the plate was allowed to stand in a 37°C incubator for 1 hour, washed with PBS(-) and then color developed for 20 minutes by addition of a color development substrate (ALP). The absorbance (antibody titer) at OD 490nm was measured for each well with a plate reader (BIO-RAD, Model 680 MICRO PLATE READER) to confirm its reactivity with the antigen peptide, to thereby determine positive wells showing a reaction between the culture supernatant and the antigen peptide.

### (6) Cloning of antibody-producing cells

Cells in the positive wells whose reactivity with the antigen peptide was confirmed by ELISA in (5) were provided for cloning of antibody-producing cell lines by limiting dilution. Namely, cells in the positive wells were plated into each well of a 96-well culture plate and cultured in a 5% CO₂, 37°C incubator for 2 weeks. In the same manner as used in (5), reactivity with the antigen peptide was confirmed by ELISA for the culture supernatant in each well, and cloning by limiting dilution was repeated again for each positive well to obtain 30 cells having a high reactivity with the antigen peptide and showing good colony growth. These cells were transferred to 24-well culture plates and cultured in a 5% CO₂, 37°C incubator for 2 weeks. In the same manner as used in (5), reactivity with the antigen peptide (antibody titer) was confirmed again by ELISA for each culture supernatant. Cells in 10 wells showing a high absorbance at OD 490nm, i.e., 10 hybridoma cell lines were determined to be useful as antibody-producing cells and were selected.

**Hybridoma cell lines**

| No. | OD value |
|---|---|
| 1 | 0.41 |
| 2 | 0.37 |
| 3 | 0.68 |
| 4 | 0.24 |
| 5 | 0.33 |
| 6 | 0.32 |
| 7 | 0.33 |
| 8 | 0.32 |
| 9 | 0.12 |
| 10 | 0.3 |

Since the antibody-producing cells thus obtained always produce the antibodies of the present invention, i.e., anti-human Exon-17 monoclonal antibodies, the supernatant of the culture in which these antibody-producing cells were cultured can be directly used as the antibody solution of the present invention. It is to be noted that the above antibody-producing cell line (hybridoma) No. 1 (SBM337), which produces anti-human Exon-17 monoclonal antibody, was deposited under FERM BP-10718 on November 1, 2006 with the International Patent Organism Depositary, the National Institute of Advanced Industrial Science and Technology.

### (7) Confirmation of binding capacity to human periostin protein (PN-1)

Antibodies produced by the 10 antibody-producing cells obtained in (6) were confirmed for their binding capacity to human periostin protein (PN-1) by dot blotting. Namely, the synthetic protein obtained in Example 8 (30µg/ml) was spotted in 5µl volumes on a Hybond-ECL nitrocellulose membrane (GE Healthcare Bio-Sciences KK) and washed once with TBS solution (10mM Tris-HCl (pH8.0), 150mM NaCl). Blocking buffer (Block Ace, Snow Brand Milk Products Co., Ltd.) was added and shaken at room temperature for 1 hour. After a 1µg/ml solution of each monoclonal antibody (primary antibody) obtained in (6) was added to the membrane and shaken for 3 hours, the membrane was washed four times with TBS solution under shaking for 10 minutes. After a 0.4µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (ECL plus western blotting detection system, GE Healthcare Bio-Sciences KK) were added and reacted for 1 minute to detect chemiluminescence. As a result, it was confirmed that all of the 10 antibody-producing cells cloned in (6) bind to human periostin PN-1.

### (8) Mass production and purification of monoclonal antibody

BALB/c mice were intraperitoneally administered with pristane [2,6,10,14-tetramethyl pentadecane (0.5ml, Wako Pure Chemical Industries, Ltd.) and kept for 2 to 3 weeks. The monoclonal antibody-producing hybridomas No. 1 and No. 3 which had been maintained at the logarithmic growth phase were collected and centrifuged to remove the culture supernatant. To the cells in each pellet fraction, FCS-free RPMI medium was added to prepare a cell suspension at a cell density of 1 x 10⁷cells/ml. This cell suspension was intraperitoneally injected into the BALB/c mice pretreated with pristane and, after about three weeks, the exuded ascites fluid was collected from the abdominal region by a syringe. After each collected ascites fluid was filtered using a filter with a pore size of φ0.22µm, the filtrates were purified in a routine manner by affinity chromatography on a Protein G-sepharose column (Millipore, 11511324) to prepare two anti-human Exon-17 monoclonal antibodies.

### [Example 10]

### Recognition site analysis of anti-human Exon-17 monoclonal antibody in human periostin Exon-17 peptide chain

The resulting two monoclonal antibodies (No. 1 and No. 3) were analyzed for their recognition sites in the human periostin Exon-17 peptide chain (epitope identification). Namely, based on an amino acid sequence consisting of 45 amino acids in total between the -9th phenylalanine from the N-terminus and the 9th isoleucine from the C-terminus of the human periostin Exon-17 peptide chain (SEQ ID NO: 4; the 1st threonine up to the 27th glutamic acid), the following 36 peptides composed of 10 amino acids were synthesized on a cellulose membrane to prepare a membrane-bound peptide array (custom SPOTs service of Sigma-Aldrich Japan K.K.).

1 FKEIPVTVYT
2 KEIPVTVYTT
3 EIPVTVYTTK
4 IPVTVYTTKI
5 PVTVYTTKII I
6 VTVYTTKIIT
7 TVYTTKIITK
8 VYTTKIITKV
9 YTTKIITKVV
10 TTKIITKVVE
11 TKIITKVVEP
12 KIITKVVEPK
13 IITKVVEPKI
14 ITKVVEPKIK
15 TKVVEPKIKV
16 KVVEPKIKVI
17 VVEPKIKVIE
18 VEPKIKVIEG
19 EPKIKVIEGS
20 PKIKVIEGSL
21 KIKVIEGSLQ
22 IKVIEGSLQP
23 KVIEGSLQPI
24 VIEGSLQPII
25 IEGSLQPIIK
26 EGSLQPIIKT
27 GSLQPIIKTE
28 SLQPIIKTEG
29 LQPIIKTEGP
30 QPIIKTEGPT
31 PIIKTEGPTL
32 IIKTEGPTLT
33 IKTEGPTLTK
34 KTEGPTLTKV
35 TEGPTLTKVK
36 EGPTLTKVKI

This membrane was allowed to stand in a small volume of methanol for 5 minutes and was then washed three times with TBS solution. Blocking buffer (casein, included in SPOTs) was added and stirred at room temperature for 2 hours. After a 1µg/ml solution of each monoclonal antibody (primary antibody) obtained in Example 9(8) was added to the membrane and shaken for 3 hours, the membrane was washed three times in TBS solution under shaking for 10 minutes. After a 0.4 µg/ml solution of an HRP-labeled anti-mouse IgG antibody (Promega) (secondary antibody) was added to the membrane and incubated for 2 hours, the membrane was washed three times with TBS solution under shaking for 5 minutes. Detection reagents (SuperSignal West Pico, Pierce) were added and reacted for 1 minute to detect chemiluminescence. As a result, monoclonal antibodies No. 1 and No. 3 were found to react with and bind to only synthetic peptide No. 9 consisting of the amino acid sequence YTTKIITKVV (SEQ ID NO: 26), i.e., a peptide consisting of an amino acid sequence covering from the -1st tyrosine to the 9th valine from the N-terminus of the amino acid sequence of the human periostin Exon-17 peptide chain (SEQ ID NO: 4) or covering from the 669th tyrosine to the 679th valine from the N-terminus of the amino acid sequence of human periostin PN-1 (SEQ ID NO: 2).

### [Example 11]

### Recognition site analysis of anti-rat Exon-17 polyclonal antibody in human periostin Exon-17 peptide chain

In the same manner as shown in Example 10, the polyclonal antibody prepared in Example 1 was analyzed for its recognition site in the human periostin Exon-17 peptide chain (epitope identification). As a result, as in the case of the monoclonal antibodies in Example 10, the polyclonal antibody was found to react with only synthetic peptide No. 9, indicating that the polyclonal antibody specifically recognizes the same site as the monoclonal antibodies. This suggests that antibodies having the same specificity are obtainable in both cases where a rat periostin Exon-17 peptide is used as an antigen to prepare a polyclonal antibody and where a human periostin Exon-17 peptide is used as an antigen to prepare a monoclonal antibody.

### [Example 12]

### Confirmation of binding capacity to rat periostin protein (PN-1)

The resulting two monoclonal antibodies (No. 1 and No. 3) were confirmed for their binding capacity to rat periostin protein (PN-1) by dot blotting. Namely, the purified protein obtained in Preparation example 6 (30µg/ml) was spotted in 5µl volumes on a Hybond-ECL nitrocellulose membrane (GE Healthcare Bio-Sciences KK) and washed once with TBS solution (10mM Tris-HCl (pH8.0), 150mM NaCl). Blocking buffer (Block Ace, Snow Brand Milk Products Co., Ltd.) was added and shaken at room temperature for 1 hour. After a 1µg/ml solution of each monoclonal antibody (primary antibody) was added to the membrane and shaken for 3 hours, the membrane was washed four times with TBS solution under shaking for 10 minutes. After a 0.4µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (ECL plus western blotting detection system, GE Healthcare Bio-Sciences KK) were added and reacted for 1 minute to detect chemiluminescence. As a result, it was confirmed that the resulting two monoclonal antibodies also bind to rat periostin PN-1.

### [Example 13]

### Epitope analysis of anti-human Exon-17 monoclonal antibody

The results of Example 10 indicated that the epitope part of each anti-human Exon-17 monoclonal antibody recognizes an amino acid sequence (TTKIITKVV; SEQ ID NO: 22) covering from the N-terminal threonine to the 9th valine of the human periostin Exon-17 peptide chain (SEQ ID NO: 4). Likewise, the results of Example 12 confirmed that each anti-human Exon-17 monoclonal antibody also binds to rat periostin protein (PN-1). These results suggested that the epitope part of each anti-human Exon-17 monoclonal antibody recognizes a region, whose amino acids do not differ between humans and rats, in the amino acid sequence (TTKIITKVV; SEQ ID NO: 22) covering from the N-terminal threonine to the 9th valine of the human periostin Exon-17 peptide chain (SEQ ID NO: 4), i.e., the entire amino acid sequence, or a part thereof, which covers from the N-terminal threonine to the 7th lysine of the human periostin Exon-17 peptide chain (SEQ ID NO: 4) or the rat periostin Exon-17 peptide chain (SEQ ID NO: 3). Thus, for further analysis of the epitope part, alanine scanning was performed.

Based on an amino acid sequence (YTTKIITKVV; SEQ ID NO: 26) covering from the -1st tyrosine to the 9th valine from the N-terminus of the human periostin Exon-17 peptide chain (SEQ ID NO: 4), the following 10 peptides modified to replace some amino acids by alanines were synthesized at a purity of 80% or more.
#1 YTTKIITKW
#2 ATTKIITKAA
#3 AATKIITKAA
#4 AAAKIITKAA
#5 AAAAIITKAA
#6 AAAAAITKAA
#7 ATTKIITAAA
#8 ATTKIIAAAA
#9 ATTKIAAAAA
#10 ATTKAAAAAA

The synthetic peptides (1mg each) were solubilized with 50µl PBS(-), spotted in 1.5µl volumes on a Hybond-ECL nitrocellulose membrane (GE Healthcare Bio-Sciences KK) and washed once with TBS solution. Blocking buffer (Block Ace, Snow Brand Milk Products Co., Ltd.) was added and shaken at room temperature for 1 hour. After a 1µg/ml solution of each monoclonal antibody (primary antibody) obtained in Example 9(8) was added to the membrane and shaken for 3 hours, the membrane was washed four times with TBS solution under shaking for 10 minutes. After a 0.4µg/ml solution of an HRP-labeled anti-mouse IgG antibody (Promega) (secondary antibody) was added to the membrane and shaken at room temperature for 1 hour, the membrane was washed four times with TBS solution under shaking for 10 minutes. Detection reagents (SuperSignal West Pico, Pierce) were added and reacted for 1 minute to detect chemiluminescence.

As a result, the monoclonal antibodies were found to strongly react with synthetic peptide #7 shown above (a peptide comprising alanine substitutions at the 1st and the 8-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)), weakly react with synthetic peptides #1 (a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)) and #2 (a peptide comprising alanine substitutions at the 1st and the 9-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)), and more weakly react with synthetic peptides #3 (a peptide comprising alanine substitutions at the 1st, the 2nd and the 9-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO:4)) and #8 (a peptide comprising alanine substitutions at the 1st and the 7-10th amino acids from the N-terminus of a peptide consisting of an amino acid sequence (YTTKIITKVV; SEQ ID NO: 26) covering from the -1st tyrosine to the 9th valine from the N-terminus of the human periostin Exon-17 peptide chain (SEQ ID NO: 4)).

### [Example 14]

### Recognition site analysis of anti-rat Exon-17 polyclonal antibody in human periostin Exon-17 peptide chain

In the same manner as shown in Example 13, the polyclonal antibody prepared in Example 1 was analyzed for its recognition site in the human periostin Exon-17 peptide chain (epitope identification). As a result, as in the case of the monoclonal antibodies in Example 13, the polyclonal antibody was found to strongly react with synthetic peptide #7, weakly react with synthetic peptides #1 and #2, and still more weakly react with synthetic peptides #3 and #8, indicating that the polyclonal antibody specifically recognizes the same site as the monoclonal antibodies. This suggests that antibodies having the same specificity are obtainable in both cases where a rat periostin Exon-17 peptide is used as an antigen to prepare a polyclonal antibody and where a human periostin Exon-17 peptide is used as an antigen to prepare a monoclonal antibody.

### [Example 15]

### In vitro study of the presence or absence of anti-cell adhesive activity of human periostin-1

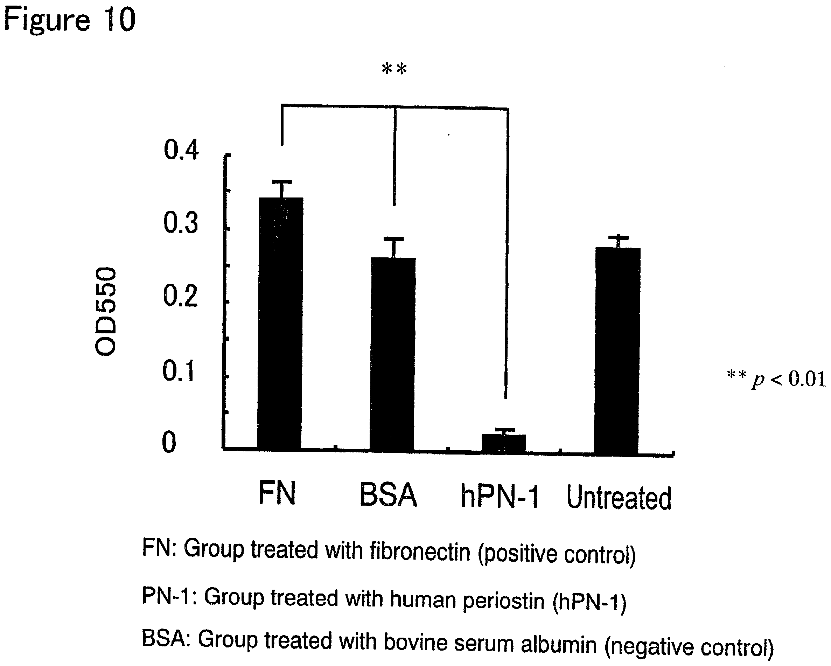

In the same manner as shown in Example 2, human heart fibroblasts (Dainippon Pharmaceutical Co., Ltd., catalog No. CS-ABI-5118) were plated on a 96-well plate at a density of 6.4 x 10⁴ cells/100µl and cultured overnight, and then the culture was incubated in fresh CSC medium (Cell System Corporation) with 10% FBS containing 10µg/ml cycloheximide at 37°C for 1 hour. Then, the cells were washed twice with CSC medium (serum free) prewarmed at 37°C, and human periostin-1 protein prepared according to the Examples was added to CSC medium (serum free) at a final concentration of 1µg/ml. Fibronectin having cell adhesion-promoting properties was used as a positive control and BSA (bovine serum albumin) having no cell adhesive properties was used as a negative control. After incubation at 37°C for 3.5 hours, microscopy showed that all the cells were separated in the group treated with human periostin protein, and the cells were washed twice with PBS (- ) and then fixed in 10% neutral buffered formalin for 30 minutes. Then, the cells were washed with PBS (-) three times and then stained with crystal violet for 30 minutes. Then, the degree of staining was measured using a plate reader at 550nm (BIO-RAD, Model 680 MICRO PLATE READER) (Figure 10). As a result, the control groups treated with fibronectin and BSA and the untreated group did not show anti-cell adhesive properties, in contrast to the group treated with human periostin-1 protein in which the cells were separated, showing that human periostin-1 protein has anti-cell adhesive properties.

### [Example 16]

### In vitro study of the neutralizing activity of anti-human Exon-17 monoclonal antibody

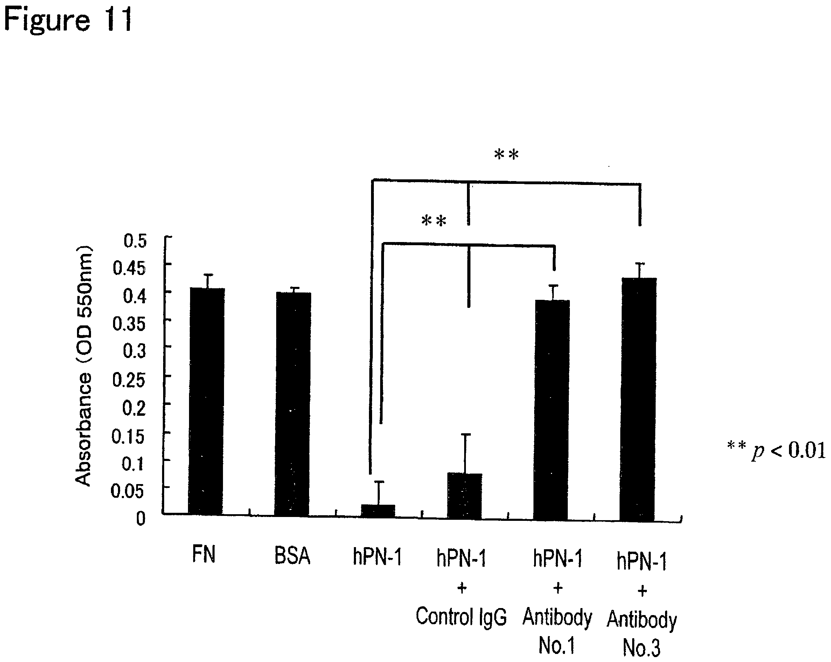

In the same manner as shown in Example 3, human heart fibroblasts were plated on a 96-well plate at a density of 6.4 x 10⁴cells/100µl and cultured overnight, and then the culture was incubated in fresh CSC medium with 10% FBS containing 10µg/ml cycloheximide at 37°C for 1 hour. Then, the cells were washed twice with CSC medium (serum free) prewarmed at 37°C, and human periostin-1 protein and anti-human Exon-17 monoclonal antibody (No. 1 or No. 3) were added to CSC medium (serum free) at final concentrations of 1µg/ml and 200µg/ml, respectively. Human periostin-1 protein alone was used as a positive control and BSA was used as a negative control. After incubation at 37°C for 3.5 hours, microscopy showed that all the cells were separated in the group treated with human periostin protein alone, and the cells were washed twice with PBS (-) and then fixed in 10% neutral buffered formalin for 30 minutes. Then, the cells were washed with PBS (-) three times and then stained with crystal violet for 30 minutes. Then, the degree of staining was measured using a plate reader at 550nm (BIO-RAD, Model 680 MICRO PLATE READER) (Figure 11). The results showed that anti-human Exon-17 monoclonal antibodies are antibodies having the activity of inhibiting the anti-cell adhesive properties of human periostin-1 protein, i.e., neutralizing the anti-cell adhesive properties of human periostin-1 protein.

As shown above, the anti-cell adhesive properties of human periostin-1 protein were inhibited by antibodies against Exon-17 of human periostin-1 protein which specifically recognize a sequence or a part thereof consisting of the N-terminal 1st to 6th amino acids of Exon-17, suggesting that Exon-17, at least a peptide segment or a part thereof consisting of the N-terminal 1st to 6th amino acids of Exon-17 constitutes a region related to the anti-cell adhesive properties of human periostin-1 protein.

### [Example 17]

### Effect of anti-human Exon-17 monoclonal antibody on acute myocardial infarction model rats

In the same manner as shown in Example 4, a male Lewis rat weighing 250-300g was fixed on a rat surgical table after the animal was thoroughly anesthetized by peritoneal administration of pentobarbital (0.1ml/1000g). A tube was orally inserted into the trachea and connected to a rat ventilator (tidal volume 3ml, 80breaths/min), and the skin was laterally incised from the left third intercostal space of the sternum and the underlying greater pectoral muscle was also laterally incised, and the intercostal space was opened using a rat rib spreader to expose the heart.

Then, the left coronary artery nearly beneath the left atrium was ligated with 1.0 silk using a curved needle having a diameter of 5 mm. After visual confirmation that the anterior and lateral walls along which the left coronary artery runs have been changed from red to white to show sufficient blockage of the coronary bloodstream and the disappearance of wall motion at these sites (in the sham operation group, the needle was passed through the coronary artery and then removed without ligation), the third and fourth ribs were fixed by ligation with 3.0 silk (after the lung was expanded to remove the air existing outside the lung in the rib cage so that the lung can be easily expanded). The incision site in the skin was sutured with 3.0 silk in the same manner and then observed for a while, and the tube was removed after confirmation of recovery of consciousness and resumption of spontaneous breathing.

Acute myocardial infarction models were sequentially prepared by the foregoing procedure.

On the following day, percutaneous echocardiography was performed under intranasal anesthesia with isoflurane, and small infarction models having an infarction size less than 20% of the entire periphery of the left ventricle were excluded. The remaining infarction models were ranked in order of increasing heart function, and alternately classified into a group treated with anti-human Exon-17 monoclonal antibody (No. 3) and a group treated with a control antibody (rabbit IgG) each 200µg via tail vein.

The antibodies were administered to each group on the day following the preparation of the models and at intervals of 6 days after the initial administration, a total of 4 times.

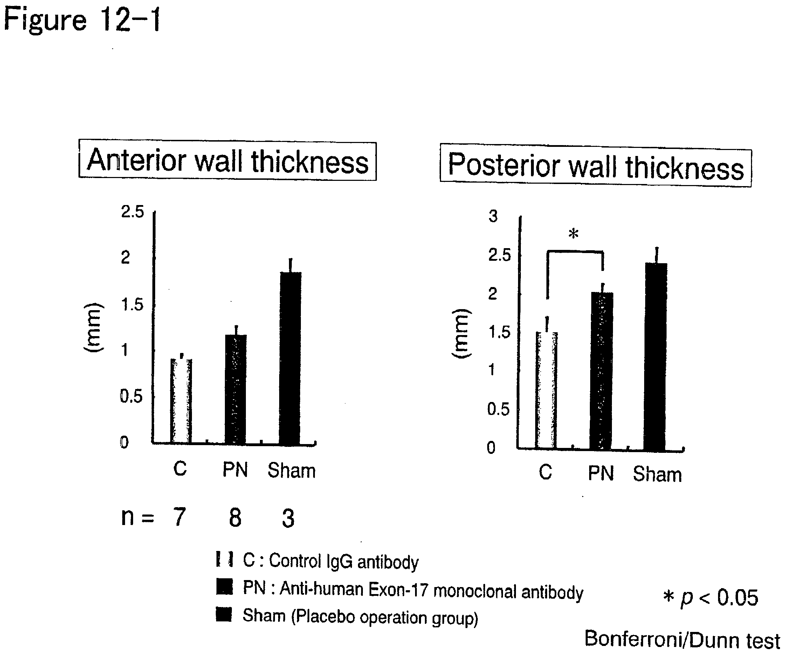

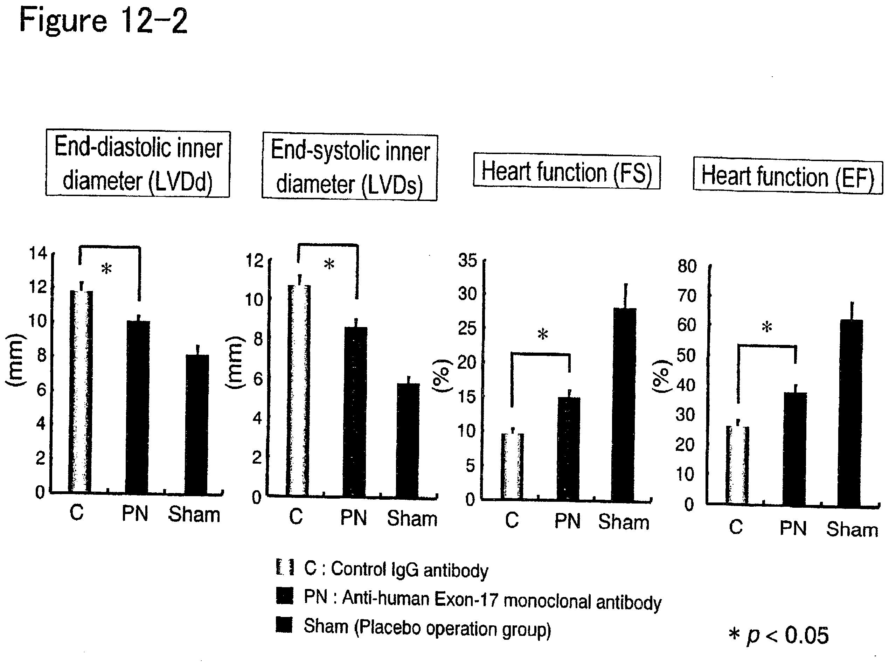

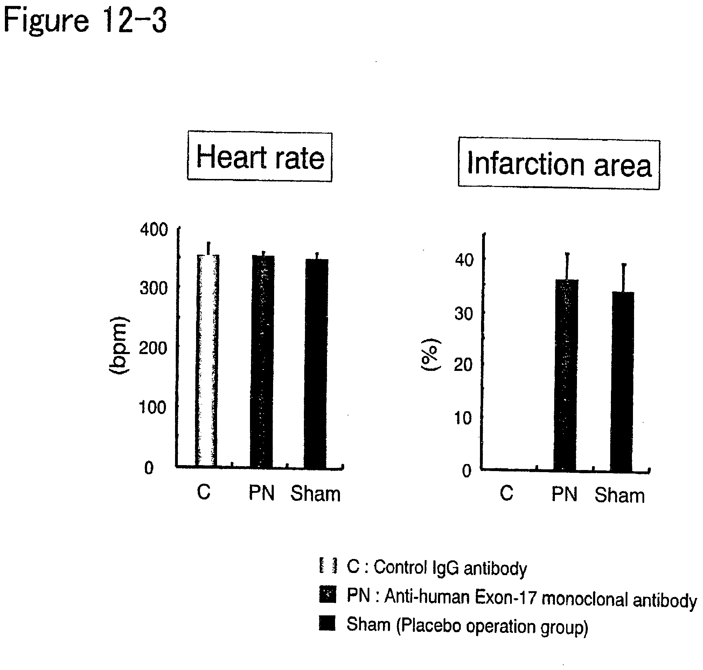

The heart was evaluated by echocardiography through the chest wall at intervals of one week until the end of 4 weeks. The results of echocardiography 4 weeks after the preparation of the models showed that the reduction of the anterior wall thickness and posterior wall thickness of the heart was inhibited, the increase of the end-diastolic inner diameter and end-systolic inner diameter was inhibited, and that the FS value or EF value indicative of the contractile function of the heart increased in the group treated with anti-human Exon-17 monoclonal antibody significantly as compared with the control group treated with rabbit IgG. In brief, heart dilation was inhibited, showing that heart function was improved (Figure 12-1 to Figure 12-3).

As shown above, in acute myocardial infarction model rats, effects of inhibiting heart dilation and improving heart function were caused by antibodies against Exon-17 of human periostin-1 protein which have an epitope composed of at least a sequence or a part thereof consisting of the N-terminal 1st to 6th amino acids of Exon-17, suggesting that Exon-17 of human periostin-1 protein, especially a region comprising at least a peptide segment or a part thereof consisting of the N-terminal 1st to 6th amino acids of Exon-17 is a region related to heart dilation and reduced heart function following myocardial infarction.

### INDUSTRIAL APPLICABILITY

Diseases in which periostin is involved can be prevented and treated by suppression of the function of a periostin isoform having anti-cell adhesive activity highly expressed in a disease such as heart failure, inhibition of the aggravation of condition and improvement of the function of tissue by use of an antibody against the periostin isoform having anti-cell adhesive activity. Moreover, the presence of the diseases and the degree of progress of symptoms can be known by measurement of the amount of the periostin isoform in a sample from a patient.

## Claims

1. An antibody against a periostin isoform having anti-cell adhesive activity, wherein the antibody specifically recognises a site responsible for the anti-cell adhesion of periostin, is against one of the amino acid sequences selected from the group of SEQ ID NOs: 3, 4, 21, 22, 23, 24, 26 and 34, and has ability to inhibit anti-cell adhesive activity of periostin.

2. The antibody of claim 1, wherein the amino acid sequence is the amino acid sequence of SEQ ID NOs: 3, 4, or 21.

3. The antibody of claim 1, wherein the antibody specifically recognizes an amino acid sequence shown in SEQ ID NO: 34.

4. The antibody of any one of claims 1 to 3, which is a monoclonal antibody.

5. An antibody of claim 4, produced by a hybridoma cell line FERM BP-10718.

6. A hybridoma producing the antibody of claim 1, obtainable by a process comprising the steps of:
immunising a non-human mammal with a peptide having one of the amino acid sequences selected from the group of SEQ ID No. 3, 4 and 21, or a peptide selected from the group of SEQ ID : No 3, 4 and 21 coupled to a carrier protein via cysteine residues introduced into the N-terminus of said peptide, and
fusing an antibody-producing cell of the mammal with a myeloma cell.

7. A hybridoma cell line FERM BP-10718.

8. A method for producing an antibody of claim 4, comprising the steps of:
immunising a non-human mammal with peptide having one of the amino acid sequences selected from the group of SEQ ID No. 3, 4 and 21, or a peptide selected from the group of SEQ ID : NO 3, 4 and 21 coupled to a carrier protein via cysteine residues introduced into the N-terminus of said peptide,
fusing an antibody-producing cell of the mammal with a myeloma cell; and
culturing the obtained hybridoma.

9. A method for producing an antibody, comprising a step of culturing the hybridoma cell line FERM BP-10718.

10. A pharmaceutical composition comprising the antibody of any one of claims 1 to 5.

11. A pharmaceutical composition for use in preventing or treating a disease, comprising the antibody of any one of claims 1 to 5 wherein the disease is heart failure, myocardial infarction, heart dilation, cardiac hypertrophy, cardiac fibrosis, cardiomyopathy, myocarditis, valvular disease or cancer.

12. The antibody as defined in any one of claims 1 to 5 for use in a method for preventing or treating a disease , wherein the disease is a heart failure, myocardial infarction, heart dilation, cardiac hypertrophy, cardiac fibrosis, cardiomyopathy, myocarditis, valvular disease or cancer.

13. The antibody as defined in any one of claims 1 to 5 for use in a method for diagnosing a disease in which a periostin isoform having anti-cell adhesive activity is involved, comprising measuring the amount of the periostin isoform in a biological sample, wherein the disease is heart failure, myocardial infarction, heart dilation, cardiac hypertrophy, cardiac fibrosis, cardiomyopathy, myocarditis, valvular disease or cancer.

14. The use of claim 13, wherein the antibody is a labeled antibody.

15. The antibody as defined in any one of claims 1 to 5 for use in a method for detecting or quantifying a periostin isoform having anti-cell adhesive activity in a sample.

16. A diagnostic reagent for use in diagnosing a disease, comprising the antibody of any one of claims 1 to 5 wherein the disease is heart failure, myocardial infarction, heart dilation, cardiac hypertrophy, cardiac fibrosis, cardiomyopathy, myocarditis, valvular disease or cancer.

## Patentansprüche

1. Antikörper gegen eine Periostin-Isoform mit Antizellhaftungsaktivität, wobei der Antikörper spezifisch eine Stelle erkennt, die für die Antizellhaftung des Periostins verantwortlich ist, wobei der Antikörper spezifisch gegen einen Vertreter der Aminosäuresequenzen, ausgewählt aus der Gruppe der SEQ ID NO: 3, 4, 21, 22, 23, 24, 26 und 34, ist und die Fähigkeit besitzt, Antizellhaftungsaktivität des Periostatins zu inhibieren.

2. Antikörper nach Anspruch 1, wobei die Aminosäuresequenz die Aminosäuresequenz SEQ ID NO: 3, 4, oder 21 ist.

3. Antikörper nach Anspruch 1, wobei der Antikörper spezifisch eine Aminosäuresequenz erkennt, die in SEQ ID NO: 34 dargestellt ist.

4. Antikörper nach einem der Ansprüche 1 bis 3, der ein monoklonaler Antikörper ist.

5. Antikörper nach Anspruch 4, produziert von einer Hybridom-Zellinie FERM BP-10718.

6. Hybridom, welches einen Antikörper nach Anspruch 1 herstellt, erhältlich durch ein Verfahren, das die Schritte umfasst:
Immunisieren eines nicht-menschlichen Säugetiers mit einem Peptid, das einen Vertreter der Aminosäuresequenzen, ausgewählt aus der Gruppe der SEQ ID NO: 3, 4, und 21, aufweist, oder einem Peptid, ausgewählt aus der Gruppe der SEQ ID NO: 3, 4, und 21, das an ein Trägerprotein über in den N-Terminus des genannten Peptids eingeführte Cystein-Reste gekoppelt ist, und
Fusionieren einer Antikörper-produzierenden Zelle des Säugetiers mit einer Myelomzelle.

7. Hybridom-Zellinie FERM BP-10718.

8. Verfahren zur Herstellung eines Antikörpers nach Anspruch 4, das die Schritte umfasst:
Immunisieren eines nicht-menschlichen Säugetiers mit einem Peptid, das einen Vertreter der Aminosäuresequenzen, ausgewählt aus der Gruppe der SEQ ID NO: 3, 4, und 21, aufweist, oder einem Peptid, ausgewählt aus der Gruppe der SEQ ID NO: 3, 4, und 21, das an ein Trägerprotein über in den N-Terminus des genannten Peptids eingeführte Cystein-Reste gekoppelt ist,
Fusionieren einer Antikörper-produzierenden Zelle des Säugetiers mit einer Myelomzelle; und
Kultivieren des erhaltenen Hybridoms.

9. Verfahren zur Herstellung eines Antikörpers, umfassend einen Kultivierungsschritt der Hybridom-Zellinie FERM BP-10718.

10. Pharmazeutische Zusammensetzung, umfassend den Antikörper nach einem der Ansprüche 1 bis 5.

11. Pharmazeutische Zusammensetzung zur Verwendung bei der Prävention oder Behandlung einer Erkrankung, umfassend den Antikörper nach einem der Ansprüche 1 bis 5, wobei die Erkrankung Herzinsuffizienz, Herzinfarkt, Herzerweiterung, Herzhypertrophie, Herzfibrose, Kardiomyopathie, Myokarditis, Herzklappenerkrankungen oder Krebs ist.

12. Antikörper nach einem der Ansprüche 1 bis 5 zur Verwendung bei einem Verfahren zur Prävention oder Behandlung einer Erkrankung, wobei die Erkrankung Herzinsuffizienz, Herzinfarkt, Herzerweiterung, Herzhypertrophie, Herzfibrose, Kardiomyopathie, Myokarditis, Herzklappenerkrankungen oder Krebs ist.

13. Antikörper nach einem der Ansprüche 1 bis 5 zur Verwendung bei einem Verfahren zur Diagnose einer Erkrankung, bei der eine Periostin-Isoform mit Antizellhaftungsaktivität beteiligt ist, umfassend Messen der Menge der Periostin-Isoform in einer biologischen Probe, wobei die Erkrankung Herzinsuffizienz, Herzinfarkt, Herzerweiterung, Herzhypertrophie, Herzfibrose, Kardiomyopathie, Myokarditis, Herzklappenerkrankungen oder Krebs ist.

14. Verwendung nach Anspruch 13, wobei der Antikörper ein markierter Antikörper ist.

15. Antikörper nach einem der Ansprüche 1 bis 5 zur Verwendung bei einem Verfahren zur Detektion oder Quantifizierung einer Periostin-Isoform mit Antizellhaftungsaktivität in einer Probe.

16. Diagnostisches Reagenz zur Verwendung bei der Diagnose einer Erkrankung, umfassend den Antikörper nach einem der Ansprüche 1 bis 5, wobei die Erkrankung Herzinsuffizienz, Herzinfarkt, Herzerweiterung, Herzhypertrophie, Herzfibrose, Kardiomyopathie, Myokarditis, Herzklappenerkrankungen oder Krebs ist.

## Revendications

1. Anticorps dirigé contre une isoforme de la périostine ayant une activité anti-adhésion cellulaire, où l'anticorps reconnaît spécifiquement un site responsable de l'anti-adhésion cellulaire de la périostine, est dirigé contre une des séquences d'acides aminés sélectionnée dans le groupe de SEQ ID NO: 3, 4, 21, 22, 23, 24, 26 et 34, et possède une capacité d'inhibition de l'activité anti-adhésion cellulaire de la périostine.

2. Anticorps selon la revendication 1, où la séquence d'acides aminés est la séquence d'acides aminés de SEQ ID NO: 3, 4, ou 21.

3. Anticorps selon la revendication 1, où l'anticorps reconnaît spécifiquement une séquence d'acides aminés montrée dans SEQ ID NO: 34.

4. Anticorps selon l'une quelconque des revendications 1 à 3, qui est un anticorps monoclonal.

5. Anticorps selon la revendication 4, produit par une lignée cellulaire d'hybridome FERM BP-10718.

6. Hybridome, qui produit un anticorps selon la revendication 1, pouvant être obtenu par un procédé comprenant les étapes qui consistent à :
immuniser un mammifère non humain avec un peptide possédant l'une des séquences d'acides aminés sélectionnée dans le groupe de SEQ ID NO: 3, 4 et 21, ou un peptide sélectionné dans le groupe de SEQ ID NO: 3, 4 et 21 couplé à une protéine porteuse via des résidus cystéine introduits dans l'extrémité N-terminale dudit peptide, et
fusionner une cellule productrice d'anticorps du mammifère avec une cellule de myélome.

7. Lignée cellulaire d'hybridome FERM BP-10718.

8. Procédé pour produire un anticorps selon la revendication 4, comprenant les étapes qui consistent à :
immuniser un mammifère non humain avec un peptide possédant l'une des séquences d'acides aminés sélectionnée dans le groupe de SEQ ID NO: 3, 4 et 21, ou un peptide sélectionné dans le groupe de SEQ ID NO: 3, 4 et 21 couplé à une protéine porteuse via des résidus cystéine introduits dans l'extrémité N-terminale dudit peptide,
fusionner une cellule productrice d'anticorps du mammifère avec une cellule de myélome ; et
mettre en culture l'hybridome obtenu.

9. Procédé pour produire un anticorps, comprenant une étape de mise en culture de la lignée cellulaire d'hybridome FERM BP-10718.

10. Composition pharmaceutique comprenant l'anticorps selon l'une quelconque des revendications 1 à 5.

11. Composition pharmaceutique destinée à être utilisée dans la prévention ou le traitement d'une maladie, comprenant l'anticorps selon l'une quelconque des revendications 1 à 5, où la maladie est l'insuffisance cardiaque, l'infarctus du myocarde, la dilatation cardiaque, une hypertrophie cardiaque, la fibrose cardiaque, une cardiomyopathie, la myocardite, une valvulopathie ou le cancer.

12. Anticorps tel que défini dans l'une quelconque des revendications 1 à 5 destiné à être utilisé dans un procédé de prévention ou de traitement d'une maladie, où la maladie est l'insuffisance cardiaque, l'infarctus du myocarde, la dilatation cardiaque, une hypertrophie cardiaque, la fibrose cardiaque, une cardiomyopathie, la myocardite, une valvulopathie ou le cancer.

13. Anticorps tel que défini dans l'une quelconque des revendications 1 à 5 destiné à être utilisé dans un procédé de diagnostic d'une maladie dans laquelle une isoforme de la périostine ayant une activité anti-adhésion cellulaire est impliquée, comprenant la mesure de la quantité de l'isoforme de la périostine dans un échantillon biologique, où la maladie est l'insuffisance cardiaque, l'infarctus du myocarde, la dilatation cardiaque, une hypertrophie cardiaque, la fibrose cardiaque, une cardiomyopathie, la myocardite, une valvulopathie ou le cancer.

14. Utilisation selon la revendication 13, dans laquelle l'anticorps est un anticorps marqué.

15. Anticorps tel que défini dans l'une quelconque des revendications 1 à 5 destiné à être utilisé dans un procédé de détection ou de quantification d'une isoforme de la périostine ayant une activité anti-adhésion cellulaire dans un échantillon.

16. Réactif diagnostique destiné à être utilisé dans le diagnostic d'une maladie, comprenant l'anticorps selon l'une quelconque des revendications 1 à 5, où la maladie est l'insuffisance cardiaque, l'infarctus du myocarde, la dilatation cardiaque, une hypertrophie cardiaque, la fibrose cardiaque, une cardiomyopathie, la myocardite, une valvulopathie ou le cancer.


### SEQUENCE LISTING

<110> Daiichi Sankyo company, Limited Osaka University
<120> An antibody against Periostin, and a pahrmaceutical composition comprising it for preventing or treating a disease in which Periostin is involved.
<130> D-11-35
<160> 34
<170> PatentIn version 3.1
<210> 1
   <211> 838
   <212> PRT
   <213> Rattus sp.
<400> 1
   Met Val Pro Leu Leu Pro Leu Ser Ala Leu Leu Leu Leu Phe Leu Cys
   1               5                   10                  15
   Asp Val Asp Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Asp Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                   165                 170                 175
   Leu Thr Lys Asp Leu Lys His Gly Met Val Ile Pro Ser Met Tyr Asn
               180                 185                 190
   Asn Leu Gly Leu Phe Ile Asn His Tyr Pro Asn Gly Val Val Thr Val
   195 200 205
   Asn Cys Ala Arg Val Ile His Gly Asn Gln Ile Ala Thr Asn Gly Val
   210 215 220
   Val His Val Ile Asp Arg Val Leu Thr Gln Ile Gly Thr Ser Ile Gln
   225 230 235 240
   Asp Phe Ile Glu Ala Glu Asp Glu Leu Ser Ser Phe Arg Ala Ala Ala
   245 250 255
   Ile Thr Ser Asp Leu Leu Glu Ser Leu Gly Arg Asp Gly His Phe Thr
   260 265 270
   Leu Phe Ala Pro Thr Asn Glu Ala Phe Glu Lys Leu Pro Arg Gly Val
   275 280 285
   Leu Glu Arg Ile Met Gly Asp Lys Val Ala Ser Glu Ala Leu Met Lys
   290 295 300
   Tyr His Ile Leu Asn Thr Leu Gln Cys Ser Glu Ala Ile Thr Gly Gly
   305 310 315 320
   Ala Val Phe Glu Thr Met Glu Gly Asn Thr Ile Glu Ile Gly Cys Glu
   325 330 335
   Gly Asp Ser Ile Ser Ile Asn Gly Ile Lys Met Val Asn Lys Lys Asp
   340 345 350
   Ile Val Thr Lys Asn Gly Val Ile His Leu Ile Asp Glu Val Leu Ile
   355 360 365
   Pro Asp Ser Ala Lys Gln Val Ile Glu Leu Ala Gly Lys Gln Gln Thr
   370 375 380
   Thr Phe Thr Asp Leu Val Ala Gln Leu Gly Leu Ala Ser Ser Leu Lys
   385 390 395 400
   Pro Asp Gly Glu Tyr Thr Leu Leu Ala Pro Val Asn Asn Ala Phe Ser
   405 410 415
   Asp Asp Thr Leu Ser Met Asp Gln Arg Leu Leu Lys Leu Ile Leu Gln
   420 425 430
   Asn His Ile Leu Lys Val Lys Val Gly Leu Ser Asp Leu Tyr Asn Gly
   435 440 445
   Gln Ile Leu Glu Thr Ile Gly Gly Lys Gln Leu Arg Val Phe Val Tyr
   450 455 460
   Arg Thr Ala Ile Cys Ile Glu Asn Ser Cys Met Val Arg Gly Ser Lys
   465 470 475 480
   Gln Gly Arg Asn Gly Ala Ile His Ile Phe Arg Glu Ile Ile Gln Pro
   485 490 495
   Ala Glu Lys Ser Leu His Glu Lys Leu Arg Gln Asp Lys Arg Phe Ser
   500 505 510
   Ile Phe Leu Ser Leu Leu Glu Ala Ala Asp Leu Lys Asp Leu Leu Thr
   515 520 525
   Gln Pro Gly Asp Trp Thr Leu Phe Ala Pro Thr Asn Asp Ala Phe Lys
   530 535 540
   Gly Met Thr Asn Glu Glu Arg Glu Ile Leu Ile Gly Asp Lys Asn Ala
   545 550 555 560
   Leu Gln Asn Ile Ile Leu Tyr His Leu Thr Pro Gly Val Tyr Ile Gly
   565 570 575
   Lys Gly Phe Glu Pro Gly Val Thr Asn Ile Leu Lys Thr Thr Gln Gly
   580 585 590
   Ser Lys Ile Tyr Val Lys Gly Val Asn Glu Thr Leu Leu Val Asn Glu
   595 600 605
   Leu Lys Ser Lys Glu Ser Asp Ile Met Thr Thr Asn Gly Val Ile His
   610 615 620
   Val Val Asp Lys Leu Leu Tyr Pro Ala Asp Ile Pro Val Gly Asn Asp
   625 630 635 640
   Gln Leu Leu Glu Leu Leu Asn Lys Leu Ile Lys Tyr Ile Gln Ile Lys
   645 650 655
   Phe Val Arg Gly Ser Thr Phe Lys Glu Ile Pro Met Thr Val Tyr Thr
   660 665 670
   Thr Lys Ile Ile Thr Lys Leu Val Glu Pro Lys Ile Lys Val Ile Gln
   675 680 685
   Gly Ser Leu Gln Pro Ile Ile Lys Thr Glu Gly Pro Ala Met Thr Lys
   690 695 700
   Ile His Ile Glu Gly Glu Pro Asp Phe Arg Leu Ile Lys Glu Gly Glu
   705 710 715 720
   Thr Val Thr Glu Val Ile His Gly Glu Pro Val Ile Lys Lys Tyr Thr
   725 730 735
   Lys Ile Ile Asp Gly Val Pro Val Glu Ile Thr Glu Lys Glu Thr Arg
   740 745 750
   Glu Glu Arg Ile Ile Thr Gly Pro Glu Ile Lys Tyr Thr Arg Ile Ser
   755 760 765
   Thr Gly Gly Gly Glu Thr Glu Glu Thr Leu Gln Lys Phe Leu Gln Lys
   770 775 780
   Glu Val Ser Lys Val Thr Lys Phe Ile Glu Gly Gly Asp Gly His Leu
   785 790 795 800
   Phe Glu Asp Glu Ala Ile Lys Arg Leu Leu Gln Gly Asp Thr Pro Ala
   805 810 815
   Lys Lys Ile Gln Ala Asn Lys Arg Val Gln Gly Ser Arg Arg Arg Ser
   820 825 830
   Arg Glu Gly Arg Ser Gln
   835
<210> 2
   <211> 836
   <212> PRT
   <213> Homo sapiens
<400> 2
   Met Ile Pro Phe Leu Pro Met Phe Ser Leu Leu Leu Leu Leu Ile Val
   1 5 10 15
   Asn Pro Ile Asn Ala Asn Asn His Tyr Asp Lys Ile Leu Ala His Ser
   20 25 30
   Arg Ile Arg Gly Arg Asp Gln Gly Pro Asn Val Cys Ala Leu Gln Gln
   35 40 45
   Ile Leu Gly Thr Lys Lys Lys Tyr Phe Ser Thr Cys Lys Asn Trp Tyr
   50 55 60
   Lys Lys Ser Ile Cys Gly Gln Lys Thr Thr Val Leu Tyr Glu Cys Cys
   65 70 75 80
   Pro Gly Tyr Met Arg Met Glu Gly Met Lys Gly Cys Pro Ala Val Leu
   85 90 95
   Pro Ile Asp His Val Tyr Gly Thr Leu Gly Ile Val Gly Ala Thr Thr
   100 105 110
   Thr Gln Arg Tyr Ser Asp Ala Ser Lys Leu Arg Glu Glu Ile Glu Gly
   115 120 125
   Lys Gly Ser Phe Thr Tyr Phe Ala Pro Ser Asn Glu Ala Trp Asp Asn
   130 135 140
   Leu Asp Ser Asp Ile Arg Arg Gly Leu Glu Ser Asn Val Asn Val Glu
   145 150 155 160
   Leu Leu Asn Ala Leu His Ser His Met Ile Asn Lys Arg Met Leu Thr
   165 170 175
   Lys Asp Leu Lys Asn Gly Met Ile Ile Pro Ser Met Tyr Asn Asn Leu
               180                 185                 190
   Gly Leu Phe Ile Asn His Tyr Pro Asn Gly Val Val Thr Val Asn Cys
           195                 200                 205
   Ala Arg Ile Ile His Gly Asn Gln Ile Ala Thr Asn Gly Val Val His
       210                 215                 220
   Val Ile Asp Arg Val Leu Thr Gln Ile Gly Thr Ser Ile Gln Asp Phe
   225                 230                 235                 240
   Ile Glu Ala Glu Asp Asp Leu Ser Ser Phe Arg Ala Ala Ala Ile Thr
                   245                 250                 255
   Ser Asp Ile Leu Glu Ala Leu Gly Arg Asp Gly His Phe Thr Leu Phe
               260                 265                 270
   Ala Pro Thr Asn Glu Ala Phe Glu Lys Leu Pro Arg Gly Val Leu Glu
           275                 280                 285
   Arg Phe Met Gly Asp Lys Val Ala Ser Glu Ala Leu Met Lys Tyr His
       290                 295                 300
   Ile Leu Asn Thr Leu Gln Cys Ser Glu Ser Ile Met Gly Gly Ala Val
   305                 310                 315                 320
   Phe Glu Thr Leu Glu Gly Asn Thr Ile Glu Ile Gly Cys Asp Gly Asp
                   325                 330                 335
   Ser Ile Thr Val Asn Gly Ile Lys Met Val Asn Lys Lys Asp Ile Val
               340                 345                 350
   Thr Asn Asn Gly Val Ile His Leu Ile Asp Gln Val Leu Ile Pro Asp
           355                 360                 365
   Ser Ala Lys Gln Val Ile Glu Leu Ala Gly Lys Gln Gln Thr Thr Phe
       370                 375                 380
   Thr Asp Leu Val Ala Gln Leu Gly Leu Ala Ser Ala Leu Arg Pro Asp
   385                 390                 395                 400
   Gly Glu Tyr Thr Leu Leu Ala Pro Val Asn Asn Ala Phe Ser Asp Asp
                   405                 410                 415
   Thr Leu Ser Met Val Gln Arg Leu Leu Lys Leu Ile Leu Gln Asn His
               420                 425                 430
   Ile Leu Lys Val Lys Val Gly Leu Asn Glu Leu Tyr Asn Gly Gln Ile
           435                 440                 445
   Leu Glu Thr Ile Gly Gly Lys Gln Leu Arg Val Phe Val Tyr Arg Thr
       450                 455                 460
   Ala Val Cys Ile Glu Asn Ser Cys Met Glu Lys Gly Ser Lys Gln Gly
   465                 470                 475                 480
   Arg Asn Gly Ala Ile His Ile Phe Arg Glu Ile Ile Lys Pro Ala Glu
                   485                 490                 495
   Lys Ser Leu His Glu Lys Leu Lys Gln Asp Lys Arg Phe Ser Thr Phe
               500                 505                 510
   Leu Ser Leu Leu Glu Ala Ala Asp Leu Lys Glu Leu Leu Thr Gln Pro
           515                 520                 525
   Gly Asp Trp Thr Leu Phe Val Pro Thr Asn Asp Ala Phe Lys Gly Met
       530                 535                 540
   Thr Ser Glu Glu Lys Glu Ile Leu Ile Arg Asp Lys Asn Ala Leu Gln
   545                 550                 555                 560
   Asn Ile Ile Leu Tyr His Leu Thr Pro Gly Val Phe Ile Gly Lys Gly
                   565                 570                 575
   Phe Glu Pro Gly Val Thr Asn Ile Leu Lys Thr Thr Gln Gly Ser Lys
               580                 585                 590
   Ile Phe Leu Lys Glu Val Asn Asp Thr Leu Leu Val Asn Glu Leu Lys
           595                 600                 605
   Ser Lys Glu Ser Asp Ile Met Thr Thr Asn Gly Val Ile His Val Val
       610                 615                 620
   Asp Lys Leu Leu Tyr Pro Ala Asp Thr Pro Val Gly Asn Asp Gln Leu
   625                 630                 635                 640
   Leu Glu Ile Leu Asn Lys Leu Ile Lys Tyr Ile Gln Ile Lys Phe Val
                   645                 650                 655
   Arg Gly Ser Thr Phe Lys Glu Ile Pro Val Thr Val Tyr Thr Thr Lys
               660                 665                 670
   Ile Ile Thr Lys Val Val Glu Pro Lys Ile Lys Val Ile Glu Gly Ser
           675                 680                 685
   Leu Gln Pro Ile Ile Lys Thr Glu Gly Pro Thr Leu Thr Lys Val Lys
       690                 695                 700
   Ile Glu Gly Glu Pro Glu Phe Arg Leu Ile Lys Glu Gly Glu Thr Ile
   705                 710                 715                 720
   Thr Glu Val Ile His Gly Glu Pro Ile Ile Lys Lys Tyr Thr Lys Ile
                   725                 730                 735
   Ile Asp Gly Val Pro Val Glu Ile Thr Glu Lys Glu Thr Arg Glu Glu
                       740                 745                 750
   Arg Ile Ile Thr Gly Pro Glu Ile Lys Tyr Thr Arg Ile Ser Thr Gly
           755                 760                 765
   Gly Gly Glu Thr Glu Glu Thr Leu Lys Lys Leu Leu Gln Glu Glu Val
       770                 775                 780
   Thr Lys Val Thr Lys Phe Ile Glu Gly Gly Asp Gly His Leu Phe Glu
   785                 790                 795                 800
   Asp Glu Glu Ile Lys Arg Leu Leu Gln Gly Asp Thr Pro Val Arg Lys
                   805                 810                 815
   Leu Gln Ala Asn Lys Lys Val Gln Gly Ser Arg Arg Arg Leu Arg Glu
               820                 825                 830
   Gly Arg Ser Gln
           835
<210> 3
   <211> 27
   <212> PRT
   <213> Rattus sp.
<400> 3
   Thr Thr Lys Ile Ile Thr Lys Leu Val Glu Pro Lys Ile Lys Val Ile
   1               5                   10                  15
   Gln Gly Ser Leu Gln Pro Ile Ile Lys Thr Glu
               20                  25
<210> 4
   <211> 27
   <212> PRT
   <213> Homo sapiens
<400> 4
   Thr Thr Lys Ile Ile Thr Lys Val Val Glu Pro Lys Ile Lys Val Ile
   1               5                   10                  15
   Glu Gly Ser Leu Gln Pro Ile Ile Lys Thr Glu
               20                  25
<210> 6
   <211> 2517
   <212> DNA
   <213> Rattus sp.
<400> 6
   atggttcctc tcctgccctt atctgctctg ctgctgctgt tcctgtgtga cgttgacccc 60
   gcaaatgcca acagttacta tgacaaggtc ctagctcaca gccgcatcag gggtcgggat 120
   cagggcccaa atgtctgtgc cctccagcag attctgggca ccaaaaagaa atacttcagc 180
   tcctgtaaga actggtatca aggtgctatc tgcgggaaga aaaccactgt gctatatgaa 240
   tgctgccccg gctatatgag aatggaaggg atgaaaggct gcccagcagt gatgcccatt 300
   gaccatgttt atggcacgct gggcatcgtg ggagccacga ccactcaaca ctattctgat 360
   gtctcgaagc tcagggaaga gattgaagga aaagggtcct acacatactt cgcgccgagt 420
   aacgaagctt gggacaacct ggattccgac atccgcagag gactagagaa caatgtcaat 480
   gttgagttac tgaacgcttt acacagccac atggttaata agagaatgct aaccaaggac 540
   ctgaaacacg gcatggttat tccttcaatg tacaacaatc tggggctttt tatcaatcat 600
   tatcccaatg gggttgtcac tgtgaactgt gctcgagtaa tccacgggaa ccagattgcc 660
   acaaatggtg ttgtccatgt catcgaccgt gtcctgacac aaattggcac ctccatccaa 720
   gacttcattg aagcagaaga tgagctttca tcattcagag cggctgccat cacttctgac 780
   cttttggagt cccttggaag agacggtcac ttcacactct ttgctcccac caatgaggct 840
   ttcgagaaac tcccacgagg agtcctagaa aggatcatgg gagacaaagt ggcttctgaa 900
   gctctcatga agtaccacat cctgaatacc ctccagtgct ctgaggctat cacaggagga 960
   gcggtgtttg agaccatgga aggaaacact attgaaatag ggtgtgaggg agacagcatc 1020
   tccattaacg gaatcaagat ggtgaacaag aaagacattg tgacgaagaa tggtgtcatc 1080
   cacctgattg atgaagtcct cattcctgat tctgctaaac aagttattga gctggctgga 1140
   aaacagcaaa ccactttcac ggacctggta gcccagttag ggttggcgtc ttctctgaag 1200
   ccggatggag agtacacgct gttagcgcct gtgaacaatg cgttctctga tgacactctg 1260
   agcatggacc agcgccttct taagctaatt ctgcaaaatc acatattgaa agtaaaagtc 1320
   ggccttagtg atctctacaa tggacagatt ctggagacca ttggaggcaa acaactccgt 1380
   gtcttcgtgt atcggacggc tatctgcata gaaaactcat gcatggtgag aggaagcaag 1440
   caggggagga acggtgccat tcacatattc cgagagatca tccaaccggc ggagaagtcc 1500
   ctgcacgaaa aactgcgcca agataagcgc ttcagcatct tcctcagcct cctcgaagct 1560
   gcagatctga aagatcttct gacacagccc ggagattgga ccttgtttgc accaaccaat 1620
   gatgccttca agggaatgac taatgaagaa agggagattc tgattgggga taaaaatgct 1680
   ctccaaaaca tcattcttta ccacctgacc ccaggggttt atattggaaa gggatttgaa 1740
   cccggagtca ccaacatcct gaagaccaca cagggaagca aaatctatgt gaaaggagtc 1800
   aatgagacgc ttttggtgaa tgagttgaag tccaaagaat ctgacatcat gacaacaaac 1860
   ggcgtcattc acgttgtgga caaactcctc tatccagcag acattccggt tggaaatgat 1920
   cagctcttgg aattactgaa caaactgata aaatacatcc aaattaagtt cgttcgtggc 1980
   agcaccttca aagaaatccc catgactgtc tatacaacta aaattataac caaactcgtg 2040
   gaaccaaaaa ttaaagtcat tcaaggcagt cttcagccta ttatcaaaac agaaggacct 2100
   gcaatgacga agatccacat tgaaggcgag cctgacttca ggctgattaa agaaggtgaa 2160
   acagtgacag aagtgatcca cggagaacca gtcattaaaa agtacaccaa aatcatagac 2220
   ggggttcctg ttgaaataac tgaaaaagag acccgggaag aacgcatcat cacaggtcct 2280
   gagataaaat acactaggat ttccacagga ggtggggaaa cagaagagac cctgcagaaa 2340
   ttcttgcaaa aagaggtctc caaggtcaca aagttcattg aaggtggcga tggtcactta 2400
   tttgaagatg aggcgattaa aagactgctt cagggagaca cacctgcaaa gaagatacaa 2460
   gccaacaaaa gggttcaagg gtctagaagg cgatcaagag aaggccgttc tcagtga 2517
<210> 7
   <211> 2436
   <212> DNA
   <213> Rattus sp.
<400> 7
   atggttcctc tcctgccctt atctgctctg ctgctgctgt tcctgtgtga cgttgacccc     60
   gcaaatgcca acagttacta tgacaaggtc ctagctcaca gccgcatcag gggtcgggat     120
   cagggcccaa atgtctgtgc cctccagcag attctgggca ccaaaaagaa atacttcagc    180
   tcctgtaaga actggtatca aggtgctatc tgcgggaaga aaaccactgt gctatatgaa    240
   tgctgccccg gctatatgag aatggaaggg atgaaaggct gcccagcagt gatgcccatt    300
   gaccatgttt atggcacgct gggcatcgtg ggagccacga ccactcaaca ctattctgat    360
   gtctcgaagc tcagggaaga gattgaagga aaagggtcct acacatactt cgcgccgagt    420
   aacgaagctt gggacaacct ggattccgac atccgcagag gactagagaa caatgtcaat    480
   gttgagttac tgaacgcttt acacagccac atggttaata agagaatgct aaccaaggac    540
   ctgaaacacg gcatggttat tccttcaatg tacaacaatc tggggctttt tatcaatcat    600
   tatcccaatg gggttgtcac tgtgaactgt gctcgagtaa tccacgggaa ccagattgcc    660
   acaaatggtg ttgtccatgt catcgaccgt gtcctgacac aaattggcac ctccatccaa    720
   gacttcattg aagcagaaga tgagctttca tcattcagag cggctgccat cacttctgac    780
   cttttggagt cccttggaag agacggtcac ttcacactct ttgctcccac caatgaggct    840
   ttcgagaaac tcccacgagg agtcctagaa aggatcatgg gagacaaagt ggcttctgaa    900
   gctctcatga agtaccacat cctgaatacc ctccagtgct ctgaggctat cacaggagga    960
   gcggtgtttg agaccatgga aggaaacact attgaaatag ggtgtgaggg agacagcatc   1020
   tccattaacg gaatcaagat ggtgaacaag aaagacattg tgacgaagaa tggtgtcatc   1080
   cacctgattg atgaagtcct cattcctgat tctgctaaac aagttattga gctggctgga   1140
   aaacagcaaa ccactttcac ggacctggta gcccagttag ggttggcgtc ttctctgaag   1200
   ccggatggag agtacacgct gttagcgcct gtgaacaatg cgttctctga tgacactctg   1260
   agcatggacc agcgccttct taagctaatt ctgcaaaatc acatattgaa agtaaaagtc   1320
   ggccttagtg atctctacaa tggacagatt ctggagacca ttggaggcaa acaactccgt   1380
   gtcttcgtgt atcggacggc tatctgcata gaaaactcat gcatggtgag aggaagcaag   1440
   caggggagga acggtgccat tcacatattc cgagagatca tccaaccggc ggagaagtcc   1500
   ctgcacgaaa aactgcgcca agataagcgc ttcagcatct tcctcagcct cctcgaagct   1560
   gcagatctga aagatcttct gacacagccc ggagattgga ccttgtttgc accaaccaat   1620
   gatgccttca agggaatgac taatgaagaa agggagattc tgattgggga taaaaatgct   1680
   ctccaaaaca tcattcttta ccacctgacc ccaggggttt atattggaaa gggatttgaa   1740
   cccggagtca ccaacatcct gaagaccaca cagggaagca aaatctatgt gaaaggagtc   1800
   aatgagacgc ttttggtgaa tgagttgaag tccaaagaat ctgacatcat gacaacaaac   1860
   ggcgtcattc acgttgtgga caaactcctc tatccagcag acattccggt tggaaatgat 1920
   cagctcttgg aattactgaa caaactgata aaatacatcc aaattaagtt cgttcgtggc 1980
   agcaccttca aagaaatccc catgactgtc tatagacctg caatgacgaa gatccacatt 2040
   gaaggcgagc ctgacttcag gctgattaaa gaaggtgaaa cagtgacaga agtgatccac 2100
   ggagaaccag tcattaaaaa gtacaccaaa atcatagacg gggttcctgt tgaaataact 2160
   gaaaaagaga cccgggaaga acgcatcatc acaggtcctg agataaaata cactaggatt 2220
   tccacaggag gtggggaaac agaagagacc ctgcagaaat tcttgcaaaa agaggtctcc 2280
   aaggtcacaa agttcattga aggtggcgat ggtcacttat ttgaagatga ggcgattaaa 2340
   agactgcttc agggagacac acctgcaaag aagatacaag ccaacaaaag ggttcaaggg 2400
   tctagaaggc gatcaagaga aggccgttct cagtga 2436
<210> 8
   <211> 838
   <212> PRT
   <213> Mus musculus
<400> 8
   Met Val Pro Leu Leu Pro Leu Tyr Ala Leu Leu Leu Leu Phe Leu Cys
   1               5                   10                  15
   Asp Ile Asn Pro Ala Asn Ala Asn Ser Tyr Tyr Asp Lys Val Leu Ala
               20                  25                  30
   His Ser Arg Ile Arg Gly Arg Asp Gln Gly Pro Asn Val Cys Ala Leu
           35                  40                  45
   Gln Gln Ile Leu Gly Thr Lys Lys Lys Tyr Phe Ser Ser Cys Lys Asn
       50                  55                  60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Glu Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                   165                 170                 175
   Leu Thr Lys Asp Leu Lys His Gly Met Val Ile Pro Ser Met Tyr Asn
   180 185 190
   Asn Leu Gly Leu Phe Ile Asn His Tyr Pro Asn Gly Val Val Thr Val
   195 200 205
   Asn Cys Ala Arg Val Ile His Gly Asn Gln Ile Ala Thr Asn Gly Val
   210 215 220
   Val His Val Ile Asp Arg Val Leu Thr Gln Ile Gly Thr Ser Ile Gln
   225 230 235 240
   Asp Phe Leu Glu Ala Glu Asp Asp Leu Ser Ser Phe Arg Ala Ala Ala
   245 250 255
   Ile Thr Ser Asp Leu Leu Glu Ser Leu Gly Arg Asp Gly His Phe Thr
   260 265 270
   Leu Phe Ala Pro Thr Asn Glu Ala Phe Glu Lys Leu Pro Arg Gly Val
   275 280 285
   Leu Glu Arg Ile Met Gly Asp Lys Val Ala Ser Glu Ala Leu Met Lys
   290 295 300
   Tyr His Ile Leu Asn Thr Leu Gln Cys Ser Glu Ala Ile Thr Gly Gly
   305 310 315 320
   Ala Val Phe Glu Thr Met Glu Gly Asn Thr Ile Glu Ile Gly Cys Glu
   325 330 335
   Gly Asp Ser Ile Ser Ile Asn Gly Ile Lys Met Val Asn Lys Lys Asp
   340 345 350
   Ile Val Thr Lys Asn Gly Val Ile His Leu Ile Asp Glu Val Leu Ile
   355 360 365
   Pro Asp Ser Ala Lys Gln Val Ile Glu Leu Ala Gly Lys Gln Gln Thr
   370 375 380
   Thr Phe Thr Asp Leu Val Ala Gln Leu Gly Leu Ala Ser Ser Leu Lys
   385 390 395 400
   Pro Asp Gly Glu Tyr Thr Leu Leu Ala Pro Val Asn Asn Ala Phe Ser
   405 410 415
   Asp Asp Thr Leu Ser Met Asp Gln Arg Leu Leu Lys Leu Ile Leu Gln
   420 425 430
   Asn His Ile Leu Lys Val Lys Val Gly Leu Ser Asp Leu Tyr Asn Gly
   435 440 445
   Gln Ile Leu Glu Thr Ile Gly Gly Lys Gln Leu Arg Val Phe Val Tyr
   450 455 460
   Arg Thr Ala Ile Cys Ile Glu Asn Ser Cys Met Val Arg Gly Ser Lys
   465 470 475 480
   Gln Gly Arg Asn Gly Ala Ile His Ile Phe Arg Glu Ile Ile Gln Pro
   485 490 495
   Ala Glu Lys Ser Leu His Asp Lys Leu Arg Gln Asp Lys Arg Phe Ser
   500 505 510
   Ile Phe Leu Ser Leu Leu Glu Ala Ala Asp Leu Lys Asp Leu Leu Thr
   515 520 525
   Gln Pro Gly Asp Trp Thr Leu Phe Ala Pro Thr Asn Asp Ala Phe Lys
   530 535 540
   Gly Met Thr Ser Glu Glu Arg Glu Leu Leu Ile Gly Asp Lys Asn Ala
   545 550 555 560
   Leu Gln Asn Ile Ile Leu Tyr His Leu Thr Pro Gly Val Tyr Ile Gly
   565 570 575
   Lys Gly Phe Glu Pro Gly Val Thr Asn Ile Leu Lys Thr Thr Gln Gly
   580 585 590
   Ser Lys Ile Tyr Leu Lys Gly Val Asn Glu Thr Leu Leu Val Asn Glu
   595 600 605
   Leu Lys Ser Lys Glu Ser Asp Ile Met Thr Thr Asn Gly Val Ile His
   610 615 620
   Val Val Asp Lys Leu Leu Tyr Pro Ala Asp Ile Pro Val Gly Asn Asp
   625 630 635 640
   Gln Leu Leu Glu Leu Leu Asn Lys Leu Ile Lys Tyr Ile Gln Ile Lys
   645 650 655
   Phe Val Arg Gly Ser Thr Phe Lys Glu Ile Pro Met Thr Val Tyr Thr
   660 665 670
   Thr Lys Ile Ile Thr Lys Val Val Glu Pro Lys Ile Lys Val Ile Gln
   675 680 685
   Gly Ser Leu Gln Pro Ile Ile Lys Thr Glu Gly Pro Ala Met Thr Lys
   690 695 700
   Ile Gln Ile Glu Gly Asp Pro Asp Phe Arg Leu Ile Lys Glu Gly Glu
   705 710 715 720
   Thr Val Thr Glu Val Ile His Gly Glu Pro Val Ile Lys Lys Tyr Thr
   725 730 735
   Lys Ile Ile Asp Gly Val Pro Val Glu Ile Thr Glu Lys Gln Thr Arg
                   740                 745                 750
   Glu Glu Arg Ile Ile Thr Gly Pro Glu Ile Lys Tyr Thr Arg Ile Ser
               755                 760                 765
   Thr Gly Gly Gly Glu Thr Gly Glu Thr Leu Gln Lys Phe Leu Gln Lys
       770                 775                 780
   Glu Val Ser Lys Val Thr Lys Phe Ile Glu Gly Gly Asp Gly His Leu
   785                 790                 795                 800
   Phe Glu Asp Glu Glu Ile Lys Arg Leu Leu Gln Gly Asp Thr Pro Ala
                   805                 810                 815
   Lys Lys Ile Pro Ala Asn Lys Arg Val Gln Gly Pro Arg Arg Arg Ser
                   820                 825                 830
   Arg Glu Gly Arg Ser Gln
               835
<210> 9
   <211> 2517
   <212> DNA
   <213> Mus musculus
<400> 9
   atggttcctc tcctgccctt atatgctctg ctgctgctgt tcctgtgtga tattaaccct      60
   gcaaatgcca acagttacta tgacaaggtc ctggctcaca gccgcatcag gggtcgggat      120
   cagggcccaa acgtctgtgc cctccagcaa attctgggca ccaaaaagaa atacttcagc     180
   tcctgtaaga actggtatca aggtgctatc tgcgggaaga aaaccactgt gctatatgaa     240
   tgctgccctg gctatatgag aatggaaggg atgaaaggct gccccgcagt gatgcctatt     300
   gaccatgttt atggcacgct gggcattgtg ggagccacta ccactcagca ctactccgat     360
   gtctcgaagc tgagagaaga gattgaagga aaagggtcat acacgtactt cgcgccgagt     420
   aacgaggctt gggagaacct ggattctgac attcgcagag gactggagaa caatgtcaat     480
   gttgagctac tgaatgcctt acacagccac atggttaata agagaatgtt aaccaaggac     540
   ctgaaacacg gcatggttat tccttcaatg tacaacaatc tggggctttt tattaaccat     600
   tatcccaatg gggttgtcac tgtgaactgt gctcgagtca tccatgggaa ccagattgcc     660
   acaaatggtg tcgtccatgt cattgaccgt gtcctgacac aaattggtac ctccatccaa     720
   gacttccttg aagcagaaga cgacctttca tcatttagag cagccgccat cacctctgac     780
   ctcttggagt cccttggaag agatggtcac ttcacgctct ttgctcccac caatgaagct     840
   ttcgagaaac tgccacgagg tgtcctagaa aggatcatgg gagacaaagt ggcttctgaa     900
   gctctcatga agtaccacat cctaaatacc ctccagtgct ctgaggccat cactggagga     960
   gccgtgtttg agaccatgga aggaaacact attgagatag ggtgcgaagg ggacagtatc    1020
   tccattaacg gaatcaagat ggtgaacaag aaagacattg tgactaagaa tggtgtcatc    1080
   cacctgattg atgaagtcct cattcctgat tctgccaaac aagttattga gctggctgga    1140
   aaacagcaaa ccactttcac cgacctggta gcccaattag gcttggcatc ctctctgaag 1200
   ccagatggag agtacacctt attagcacct gtgaacaatg cgttctctga tgacactctg 1260
   agcatggacc aacgccttct taagctaatt ctgcaaaatc acatattgaa agtaaaagtt 1320
   ggccttagcg acctctacaa tggacagata ctggaaacca ttggaggcaa acaactccga 1380
   gtctttgtgt atcggacggc tatctgcata gaaaactcat gcatggtgag aggaagcaag 1440
   cagggaagga atggtgccat tcacatattc cgagaaatca tccaaccagc agagaaatcc 1500
   ctgcacgaca agctgcggca agacaagcgc tttagcatct tcctcagcct ccttgaagct 1560
   gcagatttga aagatctcct gacacagccc ggagattgga ccttgtttgc accaaccaat 1620
   gatgccttca agggaatgac tagcgaagaa agggagcttc tgattgggga taaaaatgct 1680
   ctccaaaaca tcattcttta tcacctgacc ccaggggttt atattggaaa gggattcgaa 1740
   cccggagtca ctaatatcct gaagaccaca cagggaagca aaatctatct gaaaggagta 1800
   aacgaaacgc ttctagtgaa tgagttgaag tccaaagaat ctgacatcat gacgacaaat 1860
   ggtgtcatcc acgtcgtgga caaactcctc tatccagcag atattccagt tggaaatgat 1920
   cagctcttgg aattactgaa caaactgata aaatacatcc aaatcaagtt tgttcgtggc 1980
   agcaccttca aagaaatccc catgactgtc tatacaacta aaattataac caaagtcgtg 2040
   gaaccaaaaa ttaaagtcat tcaaggcagt cttcagccta ttatcaaaac ggaaggacct 2100
   gcaatgacga agatccaaat tgaaggtgat cccgacttca ggctgattaa agaaggcgaa 2160
   acggtgacag aagtgatcca cggagagcca gtcattaaaa agtacaccaa aatcatagat 2220
   ggagttcctg ttgaaataac tgaaaaacag actcgggaag aacgaatcat tacaggtcct 2280
   gagataaaat ataccaggat ttccacagga ggtggagaaa caggagagac cttgcagaaa 2340
   ttcttgcaaa aagaggtctc caaggtcaca aagttcattg aaggtggcga tggtcactta 2400
   tttgaagatg aggagattaa aagactgctt cagggagaca cacctgcaaa gaagatacca 2460
   gccaacaaaa gggttcaagg gcctagaaga cgatcaagag aaggccgttc tcagtga 2517
<210> 10
   <211> 811
   <212> PRT
   <213> Mus musculus
<400> 10
   Met Val Pro Leu Leu Pro Leu Tyr Ala Leu Leu Leu Leu Phe Leu Cys
   1 5 10 15
   Asp Ile Asn Pro Ala Asn Ala Asn Ser Tyr Tyr Asp Lys Val Leu Ala
   20 25 30
   His Ser Arg Ile Arg Gly Arg Asp Gln Gly Pro Asn Val Cys Ala Leu
   35 40 45
   Gln Gln Ile Leu Gly Thr Lys Lys Lys Tyr Phe Ser Ser Cys Lys Asn
   50 55 60
   Trp Tyr Gln Gly Ala Ile Cys Gly Lys Lys Thr Thr Val Leu Tyr Glu
   65                  70                  75                  80
   Cys Cys Pro Gly Tyr Met Arg Met Glu Gly Met Lys Gly Cys Pro Ala
                   85                  90                  95
   Val Met Pro Ile Asp His Val Tyr Gly Thr Leu Gly Ile Val Gly Ala
               100                 105                 110
   Thr Thr Thr Gln His Tyr Ser Asp Val Ser Lys Leu Arg Glu Glu Ile
           115                 120                 125
   Glu Gly Lys Gly Ser Tyr Thr Tyr Phe Ala Pro Ser Asn Glu Ala Trp
       130                 135                 140
   Glu Asn Leu Asp Ser Asp Ile Arg Arg Gly Leu Glu Asn Asn Val Asn
   145                 150                 155                 160
   Val Glu Leu Leu Asn Ala Leu His Ser His Met Val Asn Lys Arg Met
                   165                 170                 175
   Leu Thr Lys Asp Leu Lys His Gly Met Val Ile Pro Ser Met Tyr Asn
               180                 185                 190
   Asn Leu Gly Leu Phe Ile Asn His Tyr Pro Asn Gly Val Val Thr Val
           195                 200                 205
   Asn Cys Ala Arg Val Ile His Gly Asn Gln Ile Ala Thr Asn Gly Val
       210                 215                 220
   Val His Val Ile Asp Arg Val Leu Thr Gln Ile Gly Thr Ser Ile Gln
   225                 230                 235                 240
   Asp Phe Leu Glu Ala Glu Asp Asp Leu Ser Ser Phe Arg Ala Ala Ala
                   245                 250                 255
   Ile Thr Ser Asp Leu Leu Glu Ser Leu Gly Arg Asp Gly His Phe Thr
               260                 265                 270
   Leu Phe Ala Pro Thr Asn Glu Ala Phe Glu Lys Leu Pro Arg Gly Val
           275                 280                 285
   Leu Glu Arg Ile Met Gly Asp Lys Val Ala Ser Glu Ala Leu Met Lys
       290                 295                 300
   Tyr His Ile Leu Asn Thr Leu Gln Cys Ser Glu Ala Ile Thr Gly Gly
   305                 310                 315                 320
   Ala Val Phe Glu Thr Met Glu Gly Asn Thr Ile Glu Ile Gly Cys Glu
                   325                 330                 335
   Gly Asp Ser Ile Ser Ile Asn Gly Ile Lys Met Val Asn Lys Lys Asp
               340                 345                 350
   Ile Val Thr Lys Asn Gly Val Ile His Leu Ile Asp Glu Val Leu Ile
           355                 360                 365
   Pro Asp Ser Ala Lys Gln Val Ile Glu Leu Ala Gly Lys Gln Gln Thr
       370                 375                 380
   Thr Phe Thr Asp Leu Val Ala Gln Leu Gly Leu Ala Ser Ser Leu Lys
   385                 390                 395                 400
   Pro Asp Gly Glu Tyr Thr Leu Leu Ala Pro Val Asn Asn Ala Phe Ser
                   405                 410                 415
   Asp Asp Thr Leu Ser Met Asp Gln Arg Leu Leu Lys Leu Ile Leu Gln
               420                 425                 430
   Asn His Ile Leu Lys Val Lys Val Gly Leu Ser Asp Leu Tyr Asn Gly
           435                 440                 445
   Gln Ile Leu Glu Thr Ile Gly Gly Lys Gln Leu Arg Val Phe Val Tyr
       450                 455                 460
   Arg Thr Ala Ile Cys Ile Glu Asn Ser Cys Met Val Arg Gly Ser Lys
   465                 470                 475                 480
   Gln Gly Arg Asn Gly Ala Ile His Ile Phe Arg Glu Ile Ile Gln Pro
                   485                 490                 495
   Ala Glu Lys Ser Leu His Asp Lys Leu Arg Gln Asp Lys Arg Phe Ser
               500                 505                 510
   Ile Phe Leu Ser Leu Leu Glu Ala Ala Asp Leu Lys Asp Leu Leu Thr
           515                 520                 525
   Gln Pro Gly Asp Trp Thr Leu Phe Ala Pro Thr Asn Asp Ala Phe Lys
       530                 535                 540
   Gly Met Thr Ser Glu Glu Arg Glu Leu Leu Ile Gly Asp Lys Asn Ala
   545                 550                 555                 560
   Leu Gln Asn Ile Ile Leu Tyr His Leu Thr Pro Gly Val Tyr Ile Gly
                   565                 570                 575
   Lys Gly Phe Glu Pro Gly Val Thr Asn Ile Leu Lys Thr Thr Gln Gly
               580                 585                 590
   Ser Lys Ile Tyr Leu Lys Gly Val Asn Glu Thr Leu Leu Val Asn Glu
           595                 600                 605
   Leu Lys Ser Lys Glu Ser Asp Ile Met Thr Thr Asn Gly Val Ile His
       610                 615                 620
   Val Val Asp Lys Leu Leu Tyr Pro Ala Asp Ile Pro Val Gly Asn Asp
   625                 630                 635                 640
   Gln Leu Leu Glu Leu Leu Asn Lys Leu Ile Lys Tyr Ile Gln Ile Lys
   645 650 655
   Phe Val Arg Gly Ser Thr Phe Lys Glu Ile Pro Met Thr Val Tyr Arg
   660 665 670
   Pro Ala Met Thr Lys Ile Gln Ile Glu Gly Asp Pro Asp Phe Arg Leu
   675 680 685
   Ile Lys Glu Gly Glu Thr Val Thr Glu Val Ile His Gly Glu Pro Val
   690 695 700
   Ile Lys Lys Tyr Thr Lys Ile Ile Asp Gly Val Pro Val Glu Ile Thr
   705 710 715 720
   Glu Lys Gln Thr Arg Glu Glu Arg Ile Ile Thr Gly Pro Glu Ile Lys
   725 730 735
   Tyr Thr Arg Ile Ser Thr Gly Gly Gly Glu Thr Gly Glu Thr Leu Gln
   740 745 750
   Lys Phe Leu Gln Lys Glu Val Ser Lys Val Thr Lys Phe Ile Glu Gly
   755 760 765
   Gly Asp Gly His Leu Phe Glu Asp Glu Glu Ile Lys Arg Leu Leu Gln
   770 775 780
   Gly Asp Thr Pro Ala Lys Lys Ile Pro Ala Asn Lys Arg Val Gln Gly
   785 790 795 800
   Pro Arg Arg Arg Ser Arg Glu Gly Arg Ser Gln
   805 810
<210> 11
<211> 2436
<212> DNA
   <213> Mus musculus
<400> 11
   atggttcctc tcctgccctt atatgctctg ctgctgctgt tcctgtgtga tattaaccct     60
   gcaaatgcca acagttacta tgacaaggtc ctggctcaca gccgcatcag gggtcgggat    120
   cagggcccaa acgtctgtgc cctccagcaa attctgggca ccaaaaagaa atacttcagc    180
   tcctgtaaga actggtatca aggtgctatc tgcgggaaga aaaccactgt gctatatgaa    240
   tgctgccctg gctatatgag aatggaaggg atgaaaggct gccccgcagt gatgcctatt    300
   gaccatgttt atggcacgct gggcattgtg ggagccacta ccactcagca ctactccgat    360
   gtctcgaagc tgagagaaga gattgaagga aaagggtcat acacgtactt cgcgccgagt    420
   aacgaggctt gggagaacct ggattctgac attcgcagag gactggagaa caatgtcaat    480
   gttgagctac tgaatgcctt acacagccac atggttaata agagaatgtt aaccaaggac    540
   ctgaaacacg gcatggttat tccttcaatg tacaacaatc tggggctttt tattaaccat    600
   tatcccaatg gggttgtcac tgtgaactgt gctcgagtca tccatgggaa ccagattgcc 660
   acaaatggtg tcgtccatgt cattgaccgt gtcctgacac aaattggtac ctccatccaa 720
   gacttccttg aagcagaaga cgacctttca tcatttagag cagccgccat cacctctgac 780
   ctcttggagt cccttggaag agatggtcac ttcacgctct ttgctcccac caatgaagct 840
   ttcgagaaac tgccacgagg tgtcctagaa aggatcatgg gagacaaagt ggcttctgaa 900
   gctctcatga agtaccacat cctaaatacc ctccagtgct ctgaggccat cactggagga 960
   gccgtgtttg agaccatgga aggaaacact attgagatag ggtgcgaagg ggacagtatc 1020
   tccattaacg gaatcaagat ggtgaacaag aaagacattg tgactaagaa tggtgtcatc 1080
   cacctgattg atgaagtcct cattcctgat tctgccaaac aagttattga gctggctgga 1140
   aaacagcaaa ccactttcac cgacctggta gcccaattag gcttggcatc ctctctgaag 1200
   ccagatggag agtacacctt attagcacct gtgaacaatg cgttctctga tgacactctg 1260
   agcatggacc aacgccttct taagctaatt ctgcaaaatc acatattgaa agtaaaagtt 1320
   ggccttagcg acctctacaa tggacagata ctggaaacca ttggaggcaa acaactccga 1380
   gtctttgtgt atcggacggc tatctgcata gaaaactcat gcatggtgag aggaagcaag 1440
   cagggaagga atggtgccat tcacatattc cgagaaatca tccaaccagc agagaaatcc 1500
   ctgcacgaca agctgcggca agacaagcgc tttagcatct tcctcagcct ccttgaagct 1560
   gcagatttga aagatctcct gacacagccc ggagattgga ccttgtttgc accaaccaat 1620
   gatgccttca agggaatgac tagcgaagaa agggagcttc tgattgggga taaaaatgct 1680
   ctccaaaaca tcattcttta tcacctgacc ccaggggttt atattggaaa gggattcgaa 1740
   cccggagtca ctaatatcct gaagaccaca cagggaagca aaatctatct gaaaggagta 1800
   aacgaaacgc ttctagtgaa tgagttgaag tccaaagaat ctgacatcat gacgacaaat 1860
   ggtgtcatcc acgtcgtgga caaactcctc tatccagcag atattccagt tggaaatgat 1920
   cagctcttgg aattactgaa caaactgata aaatacatcc aaatcaagtt tgttcgtggc 1980
   agcaccttca aagaaatccc catgactgtc tatagacctg caatgacgaa gatccaaatt 2040
   gaaggtgatc ccgacttcag gctgattaaa gaaggcgaaa cggtgacaga agtgatccac 2100
   ggagagccag tcattaaaaa gtacaccaaa atcatagatg gagttcctgt tgaaataact 2160
   gaaaaacaga ctcgggaaga acgaatcatt acaggtcctg agataaaata taccaggatt 2220
   tccacaggag gtggagaaac aggagagacc ttgcagaaat tcttgcaaaa agaggtctcc 2280
   aaggtcacaa agttcattga aggtggcgat ggtcacttat ttgaagatga ggagattaaa 2340
   agactgcttc agggagacac acctgcaaag aagataccag ccaacaaaag ggttcaaggg 2400
   cctagaagac gatcaagaga aggccgttct cagtga 2436
<210> 12
   <211> 2511
   <212> DNA
   <213> Homo sapiens
<400> 12
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgttttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctataca actaaaatta taaccaaagt tgtggaacca 2040
   aaaattaaag tgattgaagg cagtcttcag cctattatca aaactgaagg acccacacta 2100
   acaaaagtca aaattgaagg tgaacctgaa ttcagactga ttaaagaagg tgaaacaata 2160
   actgaagtga tccatggaga gccaattatt aaaaaataca ccaaaatcat tgatggagtg 2220
   cctgtggaaa taactgaaaa agagacacga gaagaacgaa tcattacagg tcctgaaata 2280
   aaatacacta ggatttctac tggaggtgga gaaacagaag aaactctgaa gaaattgtta 2340
   caagaagagg tcaccaaggt caccaaattc attgaaggtg gtgatggtca tttatttgaa 2400
   gatgaagaaa ttaaaagact gcttcaggga gacacacccg tgaggaagtt gcaagccaac 2460
   aaaaaagttc aaggttctag aagacgatta agggaaggtc gttctcagtg a 2511
<210> 13
   <211> 809
   <212> PRT
   <213> Homo sapiens
<400> 13
   Met Ile Pro Phe Leu Pro Met Phe Ser Leu Leu Leu Leu Leu Ile Val
   1 5 10 15
   Asn Pro Ile Asn Ala Asn Asn His Tyr Asp Lys Ile Leu Ala His Ser
   20 25 30
   Arg Ile Arg Gly Arg Asp Gln Gly Pro Asn Val Cys Ala Leu Gln Gln
   35 40 45
   Ile Leu Gly Thr Lys Lys Lys Tyr Phe Ser Thr Cys Lys Asn Trp Tyr
   50 55 60
   Lys Lys Ser Ile Cys Gly Gln Lys Thr Thr Val Leu Tyr Glu Cys Cys
   65 70 75 80
   Pro Gly Tyr Met Arg Met Glu Gly Met Lys Gly Cys Pro Ala Val Leu
   85 90 95
   Pro Ile Asp His Val Tyr Gly Thr Leu Gly Ile Val Gly Ala Thr Thr
   100 105 110
   Thr Gln Arg Tyr Ser Asp Ala Ser Lys Leu Arg Glu Glu Ile Glu Gly
   115 120 125
   Lys Gly Ser Phe Thr Tyr Phe Ala Pro Ser Asn Glu Ala Trp Asp Asn
   130 135 140
   Leu Asp Ser Asp Ile Arg Arg Gly Leu Glu Ser Asn Val Asn Val Glu
   145 150 155 160
   Leu Leu Asn Ala Leu His Ser His Met Ile Asn Lys Arg Met Leu Thr
   165 170 175
   Lys Asp Leu Lys Asn Gly Met Ile Ile Pro Ser Met Tyr Asn Asn Leu
   180 185 190
   Gly Leu Phe Ile Asn His Tyr Pro Asn Gly Val Val Thr Val Asn Cys
   195 200 205
   Ala Arg Ile Ile His Gly Asn Gln Ile Ala Thr Asn Gly Val Val His
   210 215 220
   Val Ile Asp Arg Val Leu Thr Gln Ile Gly Thr Ser Ile Gln Asp Phe
   225 230 235 240
   Ile Glu Ala Glu Asp Asp Leu Ser Ser Phe Arg Ala Ala Ala Ile Thr
   245 250 255
   Ser Asp Ile Leu Glu Ala Leu Gly Arg Asp Gly His Phe Thr Leu Phe
   260 265 270
   Ala Pro Thr Asn Glu Ala Phe Glu Lys Leu Pro Arg Gly Val Leu Glu
   275 280 285
   Arg Phe Met Gly Asp Lys Val Ala Ser Glu Ala Leu Met Lys Tyr His
   290 295 300
   Ile Leu Asn Thr Leu Gln Cys Ser Glu Ser Ile Met Gly Gly Ala Val
   305 310 315 320
   Phe Glu Thr Leu Glu Gly Asn Thr Ile Glu Ile Gly Cys Asp Gly Asp
   325 330 335
   Ser Ile Thr Val Asn Gly Ile Lys Met Val Asn Lys Lys Asp Ile Val
   340 345 350
   Thr Asn Asn Gly Val Ile His Leu Ile Asp Gln Val Leu Ile Pro Asp
   355 360 365
   Ser Ala Lys Gln Val Ile Glu Leu Ala Gly Lys Gln Gln Thr Thr Phe
   370 375 380
   Thr Asp Leu Val Ala Gln Leu Gly Leu Ala Ser Ala Leu Arg Pro Asp
   385 390 395 400
   Gly Glu Tyr Thr Leu Leu Ala Pro Val Asn Asn Ala Phe Ser Asp Asp
   405 410 415
   Thr Leu Ser Met Val Gln Arg Leu Leu Lys Leu Ile Leu Gln Asn His
   420 425 430
   Ile Leu Lys Val Lys Val Gly Leu Asn Glu Leu Tyr Asn Gly Gln Ile
   435 440 445
   Leu Glu Thr Ile Gly Gly Lys Gln Leu Arg Val Phe Val Tyr Arg Thr
   450 455 460
   Ala Val Cys Ile Glu Asn Ser Cys Met Glu Lys Gly Ser Lys Gln Gly
   465 470 475 480
   Arg Asn Gly Ala Ile His Ile Phe Arg Glu Ile Ile Lys Pro Ala Glu
   485 490 495
   Lys Ser Leu His Glu Lys Leu Lys Gln Asp Lys Arg Phe Ser Thr Phe
   500 505 510
   Leu Ser Leu Leu Glu Ala Ala Asp Leu Lys Glu Leu Leu Thr Gln Pro
   515 520 525
   Gly Asp Trp Thr Leu Phe Val Pro Thr Asn Asp Ala Phe Lys Gly Met
   530 535 540
   Thr Ser Glu Glu Lys Glu Ile Leu Ile Arg Asp Lys Asn Ala Leu Gln
   545 550 555 560
   Asn Ile Ile Leu Tyr His Leu Thr Pro Gly Val Phe Ile Gly Lys Gly
   565 570 575
   Phe Glu Pro Gly Val Thr Asn Ile Leu Lys Thr Thr Gln Gly Ser Lys
   580 585 590
   Ile Phe Leu Lys Glu Val Asn Asp Thr Leu Leu Val Asn Glu Leu Lys
   595 600 605
   Ser Lys Glu Ser Asp Ile Met Thr Thr Asn Gly Val Ile His Val Val
   610 615 620
   Asp Lys Leu Leu Tyr Pro Ala Asp Thr Pro Val Gly Asn Asp Gln Leu
   625 630 635 640
   Leu Glu Ile Leu Asn Lys Leu Ile Lys Tyr Ile Gln Ile Lys Phe Val
   645 650 655
   Arg Gly Ser Thr Phe Lys Glu Ile Pro Val Thr Val Tyr Arg Pro Thr
   660 665 670
   Leu Thr Lys Val Lys Ile Glu Gly Glu Pro Glu Phe Arg Leu Ile Lys
   675 680 685
   Glu Gly Glu Thr Ile Thr Glu Val Ile His Gly Glu Pro Ile Ile Lys
   690 695 700
   Lys Tyr Thr Lys Ile Ile Asp Gly Val Pro Val Glu Ile Thr Glu Lys
   705 710 715 720
   Glu Thr Arg Glu Glu Arg Ile Ile Thr Gly Pro Glu Ile Lys Tyr Thr
   725 730 735
   Arg Ile Ser Thr Gly Gly Gly Glu Thr Glu Glu Thr Leu Lys Lys Leu
   740 745 750
   Leu Gln Glu Glu Val Thr Lys Val Thr Lys Phe Ile Glu Gly Gly Asp
   755 760 765
   Gly His Leu Phe Glu Asp Glu Glu Ile Lys Arg Leu Leu Gln Gly Asp
   770 775 780
   Thr Pro Val Arg Lys Leu Gln Ala Asn Lys Lys Val Gln Gly Ser Arg
   785 790 795 800
   Arg Arg Leu Arg Glu Gly Arg Ser Gln
   805
<210> 14
   <211> 2430
   <212> DNA
   <213> Homo sapiens
<400> 14
   atgattccct ttttacccat gttttctcta ctattgctgc ttattgttaa ccctataaac 60
   gccaacaatc attatgacaa gatcttggct catagtcgta tcaggggtcg ggaccaaggc 120
   ccaaatgtct gtgcccttca acagattttg ggcaccaaaa agaaatactt cagcacttgt 180
   aagaactggt ataaaaagtc catctgtgga cagaaaacga ctgttttata tgaatgttgc 240
   cctggttata tgagaatgga aggaatgaaa ggctgcccag cagttttgcc cattgaccat 300
   gtttatggca ctctgggcat cgtgggagcc accacaacgc agcgctattc tgacgcctca 360
   aaactgaggg aggagatcga gggaaaggga tccttcactt actttgcacc gagtaatgag 420
   gcttgggaca acttggattc tgatatccgt agaggtttgg agagcaacgt gaatgttgaa 480
   ttactgaatg ctttacatag tcacatgatt aataagagaa tgttgaccaa ggacttaaaa 540
   aatggcatga ttattccttc aatgtataac aatttggggc ttttcattaa ccattatcct 600
   aatggggttg tcactgttaa ttgtgctcga atcatccatg ggaaccagat tgcaacaaat 660
   ggtgttgtcc atgtcattga ccgtgtgctt acacaaattg gtacctcaat tcaagacttc 720
   attgaagcag aagatgacct ttcatctttt agagcagctg ccatcacatc ggacatattg 780
   gaggcccttg gaagagacgg tcacttcaca ctctttgctc ccaccaatga ggcttttgag 840
   aaacttccac gaggtgtcct agaaaggttc atgggagaca aagtggcttc cgaagctctt 900
   atgaagtacc acatcttaaa tactctccag tgttctgagt ctattatggg aggagcagtc 960
   tttgagacgc tggaaggaaa tacaattgag ataggatgtg acggtgacag tataacagta 1020
   aatggaatca aaatggtgaa caaaaaggat attgtgacaa ataatggtgt gatccatttg 1080
   attgatcagg tcctaattcc tgattctgcc aaacaagtta ttgagctggc tggaaaacag 1140
   caaaccacct tcacggatct tgtggcccaa ttaggcttgg catctgctct gaggccagat 1200
   ggagaataca ctttgctggc acctgtgaat aatgcatttt ctgatgatac tctcagcatg 1260
   gttcagcgcc tccttaaatt aattctgcag aatcacatat tgaaagtaaa agttggcctt 1320
   aatgagcttt acaacgggca aatactggaa accatcggag gcaaacagct cagagtcttc 1380
   gtatatcgta cagctgtctg cattgaaaat tcatgcatgg agaaagggag taagcaaggg 1440
   agaaacggtg cgattcacat attccgcgag atcatcaagc cagcagagaa atccctccat 1500
   gaaaagttaa aacaagataa gcgctttagc accttcctca gcctacttga agctgcagac 1560
   ttgaaagagc tcctgacaca acctggagac tggacattat ttgtgccaac caatgatgct 1620
   tttaagggaa tgactagtga agaaaaagaa attctgatac gggacaaaaa tgctcttcaa 1680
   aacatcattc tttatcacct gacaccagga gttttcattg gaaaaggatt tgaacctggt 1740
   gttactaaca ttttaaagac cacacaagga agcaaaatct ttctgaaaga agtaaatgat 1800
   acacttctgg tgaatgaatt gaaatcaaaa gaatctgaca tcatgacaac aaatggtgta 1860
   attcatgttg tagataaact cctctatcca gcagacacac ctgttggaaa tgatcaactg 1920
   ctggaaatac ttaataaatt aatcaaatac atccaaatta agtttgttcg tggtagcacc 1980
   ttcaaagaaa tccccgtgac tgtctataga cccacactaa caaaagtcaa aattgaaggt 2040
   gaacctgaat tcagactgat taaagaaggt gaaacaataa ctgaagtgat ccatggagag 2100
   ccaattatta aaaaatacac caaaatcatt gatggagtgc ctgtggaaat aactgaaaaa 2160
   gagacacgag aagaacgaat cattacaggt cctgaaataa aatacactag gatttctact 2220
   ggaggtggag aaacagaaga aactctgaag aaattgttac aagaagaggt caccaaggtc 2280
   accaaattca ttgaaggtgg tgatggtcat ttatttgaag atgaagaaat taaaagactg 2340
   cttcagggag acacacccgt gaggaagttg caagccaaca aaaaagttca aggttctaga 2400
   agacgattaa gggaaggtcg ttctcagtga 2430
<210> 15
   <211> 23
   <212> DNA
   <213> Artificial
<220>
   <223> Primer No.1
<400> 15
   gttcattgaa ggtggcgatg gtc 23
<210> 16
   <211> 24
   <212> DNA
   <213> Artificial
<220>
   <223> Primer No.2
<400> 16
   gagataaaat ccctgcatgg tcct 24
<210> 17
   <211> 25
   <212> DNA
   <213> Artificial
<220>
   <223> Primer No. 3
<400> 17
   cacggtcgat gacatggaca acacc 25
<210> 18
   <211> 22
   <212> DNA
   <213> Artificial
<220>
   <223> Primer No. 4
<400> 18
   acggagctca gggctgaaga tg 22
<210> 19
   <211> 22
   <212> DNA
   <213> Artificial
<220>
   <223> Primer No. 5
<400> 19
   gacccgggaa gaacgcatca tc 22
<210> 20
   <211> 32
   <212> DNA
   <213> Artificial
<220>
   <223> Primer No. 6
<400> 20
   tgggtgaccc tgagaacggc cttctcttga tc 32
<210> 21
   <211> 27
   <212> PRT
   <213> Mus musculus
<400> 21
   Thr Thr Lys Ile Ile Thr Lys Val Val Glu Pro Lys Ile Lys Val Ile
   1               5                   10                  15
   Glu Gly Ser Leu Gln Pro Ile Ile Lys Thr Glu
               20                  25
<210> 22
   <211> 9
   <212> PRT
   <213> Homo sapiens
<400> 22
   Thr Thr Lys Ile Ile Thr Lys Val Val
   1               5
<210> 23
   <211> 9
   <212> PRT
   <213> Rattus sp.
<400> 23
   Thr Thr Lys Ile Ile Thr Lys Leu Val
   1 5
<210> 24
   <211> 9
   <212> PRT
   <213> Mus musculus
<400> 24
   Thr Thr Lys Ile Ile Thr Lys Val Val
   1 5
<210> 25
   <211> 28
   <212> PRT
   <213> Homo sapiens
<400> 25
   Cys Thr Thr Lys Ile Ile Thr Lys Val Val Glu Pro Lys Ile Lys Val Ile
   1 5 10 15
   Glu Gly Ser Leu Gln Pro Ile Ile Lys Thr Glu
   20 25
<210> 26
   <211> 10
   <212> PRT
   <213> Homo sapiens
<400> 26
   Tyr Thr Thr Lys Ile Ile Thr Lys Val Val
   1 5 10
<210> 27
   <211> 32
   <212> DNA
   <213> Artificial
<220>
   <223> Primer No.7
<400> 27
   aagctagcca ccatgattcc ctttttaccc at 32
<210> 28
   <211> 20
   <212> DNA
   <213> Artificial
<220>
   <223> Primer No.8
<400> 28
   aactccacaa tttccctcat 20
<210> 29
   <211> 21
   <212> DNA
   <213> Artificial
<220>
   <223> Primer No. 9
<400> 29
   taaccaaagt tgtggaacca a 21
<210> 30
   <211> 20
   <212> DNA
   <213> Artificial
<220>
   <223> Primer No. 10
<400> 30
   tgtgtctccc tgaagcagtc 20
<210> 31
   <211> 21
   <212> DNA
   <213> Artificial
<220>
   <223> Primer No. 11
<400> 31
   catcaccatc accatcacta a 21
<210> 32
   <211> 56
   <212> DNA
   <213> Artificial
<220>
   <223> Primer No. 12
<400> 32
   ctagaagacg attaagggaa ggtcgttctc agctggaagt tctgttccag gggccc 56
<210> 33
   <211> 52
   <212> DNA
   <213> Artificial
<220>
   <223> Primer No. 13
<400> 33
   gggcccctgg aacagaactt ccagctgaga acgaccttcc cttaatcgtc tt 52
<210> 34
   <211> 6
   <212> PRT
   <213> Homo sapiens
<400> 34
   Thr Thr Lys Ile Ile Thr
   1               5